# FASCICULE DE BREVET EUROPEEN

(11) **EP 0 514 442 B1**
(45) Date de publication et mention de la délivrance du brevet: **27.04.1994**
(21) Numéro de dépôt: 91903956.0
(22) Date de dépôt: 06.02.1991
(51) Int. Cl.: C07K 5/06, A61K 37/02

(54) **N-PHENYL N-ACETAMIDO GLYCINAMIDES, LEUR PREPARATION ET LES MEDICAMENTS LES CONTENANT**
N-PHENYL-N-ACETAMIDOGLYCINAMIDE, IHRE HERSTELLUNG UND MEDIKAMENTE, DIE SIE ENTHALTEN
N-PHENYL N-ACETAMIDO GLYCINAMIDES, PREPARATION METHOD AND DRUGS CONTAINING THEM

(30) Priorité: 09.02.1990 FR 9001553; 27.09.1990 FR 9011916; 12.10.1990 FR 9012594
(43) Date de publication de la demande: 25.11.1992
(73) Titulaire: RHONE-POULENC RORER S.A., 92165 Antony Cédex (FR)
(72) Inventeur: BOURZAT, Jean-Dominique, F-94300 Vincennes (FR); CAPET, Marc, F-94320 Thiais (FR); COTREL, Claude, F-75012 Paris (FR); GUYON, Claude, F-94100 S.-Maur-des-Fosses (FR); MANFRE, Franco, F-94400 Vitry-sur-Seine (FR); ROUSSEL, Gérard, F-91450 Soisy-sur-Seine (FR)
(74) Mandataire: Pilard, Jacques (FR)
(86) Numéro de dépôt international: FR9100087
(87) Numéro de publication internationale: WO9112264

(56) Documents cités:
- EP-A- 397 556
- EP-A- 0 166 355
- EP-A- 0 175 498
- Chemical Abstracts, vol. 113, No.25, 17 December 1990, (Columbus, Ohio,US) ,T. Lu et al.: "Synthesis of disulfides of N-(N-(2-mercaptobenzoyl) glycyl)-N-alkyl/aryl-glycine", see page 784, abstract 231990r.
- Chemical Abstracts, col. 93, No. 3, 21 July 1980, (Columbus, Ohio, US), G.P. Zecchini et al.: "Synthesis of peptides containing 1,2, 3,4-tetrahydroquinoline-2-carboxylic acid. Part 3. Cyclizaiton of dipeptides and amides with acetic anhydride", see pages 740-741, abstract 26769c

## Description

La présente invention concerne des composés de formule: 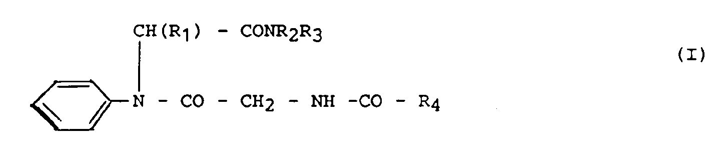
leur préparation et les médicaments les contenant.

Des phényluréidoacétamides antagonistes de la cholécystokinine sont décrits dans le brevet EP 397 556.

Dans la formule (I),
- R₁ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un atome d'hydrogène ou un radical alkyle (éventuellement substitué par un radical alcoxycarbonyle),
- R₃ représente un radical alkyle, phénylalkyle, indanyle, cycloalkylalkyle, phényle (éventuellement substitué par un ou plusieurs substituants choisis parmi les atomes d'halogène et les radicaux alkyle, alcoxy, alkylthio et dialkylamino) ou quinolyle, ou bien
- R₂ et R₃ forment ensemble avec l'atome d'azote auquel ils sont rattachés un hétérocycle mono ou polycyclique saturé ou insaturé contenant 4 à 9 atomes de carbone et un ou plusieurs hétéroatomes (O,S,N) et éventuellement substitué par un ou plusieurs radicaux alkyle, alcoxycarbonyle, dialkylcarbamoyle, phényle, alcoxy ou en combinaison avec un atome de carbone de l'hétérocycle un cycle spiro monocyclique à 4 ou 5 chaînons et contenant éventuellement un ou plusieurs hétéroatomes (O,S,N),
- R₄ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, hydroxy, mono ou polyhydroxyalkyle, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, alkylsulfinyle, hydroxyaminocarbonyle, tétrazolyl-5, tétrazolyl-5 alkyle, sulfo, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX et -S-alk-COOX,
- alk représente un radical alkyle ou alkylène,
- alk' représente un radical hydroxyalkylène ou hydroxyalkyle,
- X représente un atome d'hydrogène ou un radical alkyle, étant entendu que lorsque R₁ représente un atome d'hydrogène, R₄ représente un radical naphtyle, indolyle ou phénylamino dont le noyau phényle est éventuellement substitué par un radical alkyle, alcoxy, alkylthio, nitro, hydroxy ou par un ou deux atomes d'halogène, R₂ et R₃ ne peuvent pas former avec l'atome d'azote auquel ils sont rattachés un radical pyrrolidinyl-1 éventuellement substitué par un radical alkyle ou R₂ et R₃ ne peuvent pas représenter des radicaux alkyle.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les portions cycloalkyle contiennent 3 à 6 atomes de carbone et les radicaux acyle comportent 2 à 4 atomes de carbone.

Dans la formule (I), les atomes d'halogène sont de préférence les atomes de chlore, de brome ou de fluor.

Lorsque R₂ et R₃ forment avec l'atome d'azote auquel ils sont rattachés un hétérocycle, celui-ci est de préférence un cycle pipéridino (éventuellement substitué par au moins un radical alkyle, phényle, alcoxycarbonyle ou dialkylcarbamoyle), perhydroazépinyl-1, tétrahydro-1,2,3,6 pyridyl-1, tétrahydro-1,2,3,4 quinolyl-1, pyrrolidinyl-1, dihydro-3,4 2H-benzoxazine-1, 4 yl-4, dihydro-3,4 2H-benzothiazine-1,4 yl-4, N-alkyl tétrahydro-1,2,3,4 quinoxalinyl-1, perhydroquinolyl-l, tétrahydro-1,2,3,4 isoquinolyl-2, aza-8 spiro[4,5] décanyl-8, aza-8 dioxa-1,4 spiro[4,5] décanyl-8, phényl-2 ou -3 pyrrolidinyl-1, thiomorpholino (éventuellement substitué par au moins un radical alkyle) ou indolinyl-1.

Les composés de formule (I) contenant un ou plusieurs centres asymétriques présentent des formes isomères. Les racémiques et les énantiomères de ces composés font également partie de l'invention.

Les composés de formule (I) pour lesquels R₄ représente un radical phénylamino dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk peuvent être préparés par action d'un dérivé aminé de formule : 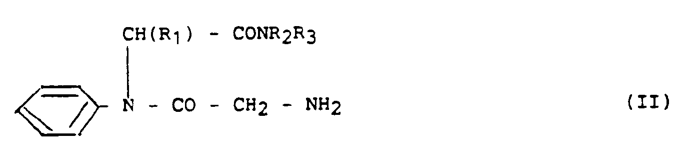
dans laquelle R₁, R₂ et R₃ ont les mêmes significations que dans la formule (I) sur un isocyanate de formule :

OCN - R₅ (III)

dans laquelle R₅ représente un radical phényle éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk.

Cette réaction s'effectue généralement au sein d'un solvant inerte tel que le tétrahydrofuranne, le diméthylformamide, un solvant chloré (chloroforme, chlorure de méthylène par exemple), un solvant aromatique (benzène, toluène par exemple), à une température comprise entre 10°C et la température d'ébullition du solvant.

Les isocyanates de formule (III) peuvent être obtenus par application ou adaptation de la méthode décrite par R. RICHTER et coll., the Chemistry of cyanate and their thio derivatives, S. PATAI, part 2, Wiley New York (1977).

Les dérivés aminés de formule (II) peuvent être obtenus par application ou adaptation de la méthode décrite par T. WIELAND et coll., Justus Liebigs Ann. Chem., 84 (1958) ou par adaptation de la méthode de GABRIEL (GIBSON et coll., Angew. Chem. Int. Ed., 7, 919 (1968)) qui consiste à faire réagir l'hydrate d'hydrazine ou la N-méthylhydrazine sur un dérivé de formule : 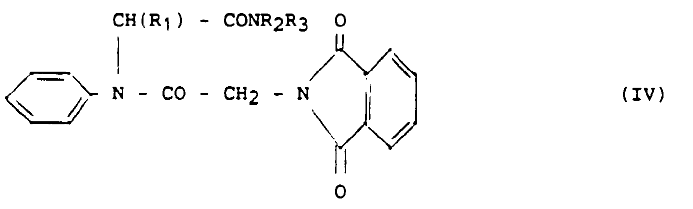
dans laquelle R₁, R₂ et R₃ ont les mêmes significations que dans la formule (I).

Cette réaction s'effectue de préférence au sein d'un solvant inerte tel qu'un alcool (méthanol, éthanol, propanol par exemple), à une température comprise entre 0°C et la température d'ébullition du solvant.

Les dérivés de formule (IV), à l'exception de ceux pour lesquels R₁ représente un radical alcoxycarbonyle peuvent être obtenus par action d'une amine de formule :

HNR₂R₃ (V)

dans laquelle R₂ et R₃ ont les mêmes significations que dans la formule (I), sur un acide de formule : 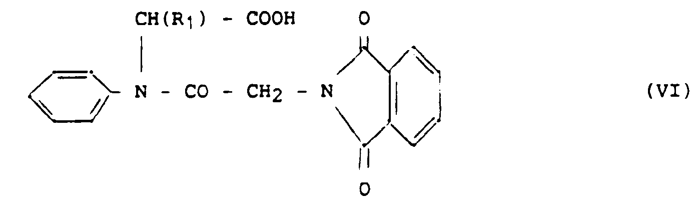
dans laquelle R₁ a les mêmes significations que précédemment ou un dérivé réactif de cet acide.

Lorsque l'on met en oeuvre l'acide on opère en présence d'un agent de condensation peptidique tel qu'un carbodiimide (par exemple le dicychlohexylcarbodiimide) ou le N,N'-diimidazole carbonyle, dans un solvant inerte tel qu'un éther (par exemple THF, dioxanne), un amide (par exemple DMF) ou un solvant chloré (par exemple chlorure de méthylène, dichloroéthane, chloroforme) à une température comprise entre 0°C et la température de reflux du mélange réactionnel.

Lorsque l'on met en oeuvre un dérivé réactif de l'acide il est possible de faire réagir l'anhydride, un anhydride mixte, un halogénure d'acide ou un ester (qui peut être choisi parmi les esters activés ou non de l'acide).

On opère alors soit en milieu organique, éventuellement en présence d'un accepteur d'acide tel qu'une base organique azotée (par exemple une trialkylamine, une pyridine, le diaza-1,8 bicyclo[5.4.0] undécène-7 ou le diaza-1,5 bicyclo[4.3.0] nonène-5), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les acides de formule (VI) peuvent être obtenus par hydrolyse d'un ester correspondant de formule : 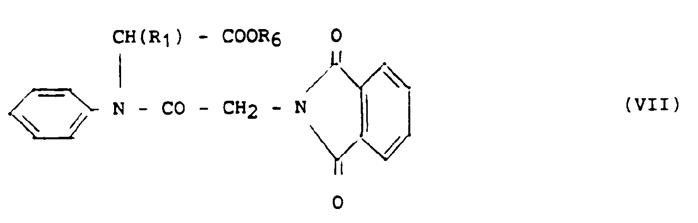
dans laquelle R₆ représente un radical alkyle et R₁ a les mêmes significations que précédemment.

Cette réaction s'effectue généralement au moyen d'acide trifluoroacétique, au sein d'un solvant inerte tel qu'un solvant chloré (chloroforme, dichlorométhane par exemple), à la température d'ébullition du solvant.

Les esters de formule (VII) peuvent être obtenus par action d'un dérivé de formule : 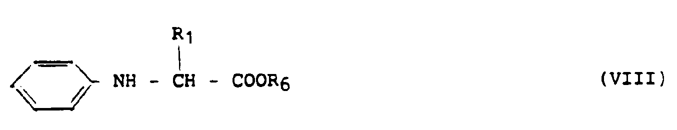
dans laquelle R₁ et R₆ ont les mêmes significations que précédemment sur un dérivé de formule :

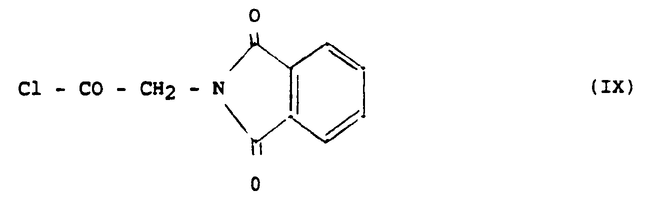

Cette réaction s'effectue généralement au sein d'un solvant inerte tel qu'un solvant chloré (chloroforme, dichloro-1,2 éthane par exemple) en présence d'un agent alcalin tel qu'un bicarbonate de métal alcalin, à une température comprise entre 20°C et la température d'ébullition du solvant.

Le dérivé de formule (IX) peut être obtenu par application de la méthode décrite par W. GRASSMANN et coll., Chem. Ber., 83, 244 (1950).

Les dérivés de formule (VIII) peuvent être obtenus par action d'aniline sur un dérivé de formule : 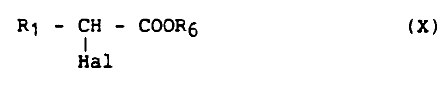
dans laquelle Hal représente un atome d'halogène, R₁ et R₆ ont les mêmes significations que précédemment.

Cette réaction s'effectue généralement au sein d'un solvant inerte tel qu'un solvant chloré (chloroforme, dichlorométhane par exemple) à la température d'ébullition du solvant.

Les dérivés de formule (IV) peuvent également être obtenus par action d'un dérivé de formule (IX) sur une amine de formule : 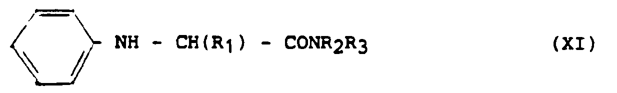
dans laquelle R₁, R₂ et R₃ ont les mêmes significations que dans la formule (I).

Cette réaction s'effectue généralement dans les mêmes conditions que celles décrites précédemment pour la réaction d'un dérivé de formule (IX) avec un composé de formule (VIII). Les amines de formule (XI) peuvent être obtenues par action d'aniline sur un dérivé de formule :

Br - CH(R₁) - CONR₂R₃ (XII)

dans laquelle R₁, R₂ et R₃ ont les mêmes significations que dans la formule (I).

Cette réaction s'effectue généralement dans les mêmes conditions que celles décrites précédemment pour la réaction de l'aniline sur un dérivé de formule (X).

Les dérivés de formule (XII) pour lesquels R₁ représente un radical alcoxycarbonyle peuvent être obtenus par bromuration d'un dérivé de formule :

R₁ - CH₂ - CONR₂R₃ (XIII)

dans laquelle R₁ représente un radical alcoxycarbonyle, R₂ et R₃ ont les mêmes significations que dans la formule (I).

On opère généralement au moyen de brome, éventuellement en présence d'acétamide.

Les dérivés de formule (XIII) peuvent être obtenus par action de malonate acide d'alkyle ou d'un dérivé réactif de cet acide sur une amine de formule (V) dans laquelle R₂ et R₃ ont les mêmes significations que dans la formule (I).

Cette réaction s'effectue généralement dans les conditions décrites précédemment pour la réaction de l'amine de formule (V) sur un acide de formule (VI).

Les dérivés de formule (XII) pour lesquels R₁ représente un atome d'hydrogène, un radical alkyle ou phényle éventuellement substitué peuvent être obtenus par action d'un chlorure d'acide de formule :

Br - CH(R₁) - COCl (XIV)

dans laquelle R₁ a les mêmes significations que précédemment sur une amine de formule (V).

Cette réaction s'effectue généralement au sein d'un solvant inerte tel que l'acétonitrile, le diméthylformamide, le tétrahydrofuranne, en présence d'une amine tertiaire telle qu'une trialkylamine, à une température voisine de 25°C.

Les composés de formule (I) pour lesquels R₄ représente un radical phénylamino dont le noyau phényle est éventuellement substitué par ou plusieurs substituants choisis parmi les atomes d'halogène et les radicaux alkyle, alkylthio, tétrazolyl-5 alkyle, tétrazolyl-5, trifluorométhylsulfonamido, alcoxy, nitro, acyle, cyano, sulfamoyle, benzoyle, alcoxycarbonyle et -alk-O-alk peuvent également être préparés par action d'une amine de formule (XI) sur un acide de formule :

HOOC - CH₂ - NH - CO - R₄ (XV)

dans laquelle R₄ a les mêmes significations que précédemment.

Cette réaction s'effectue généralement au sein d'un solvant inerte tel qu'un solvant chloré (chloroforme, dichloro-1,2 éthane par exemple), au moyen de chlorure de thionyle, à la température d'ébullition du solvant.

Les acides de formule (XV) peuvent être obtenus par action d'un isocyanate de formule (III) sur la glycine.

Cette réaction s'effectue généralement en milieu aqueux, en présence d'un agent alcalin tel qu'un bicarbonate de métal alcalin, à une température comprise entre 15 et 30°C.

Les composés de formule (I) pour lesquels R₄ représente un radical phénylamino éventuellement substitué peuvent également être préparés par action d'un dérivé de formule : 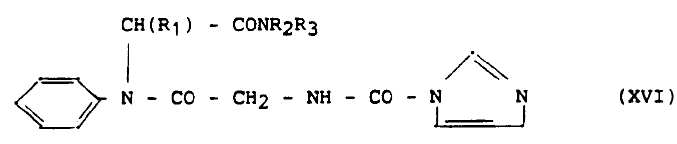
dans laquelle R₁, R₂ et R₃ ont les mêmes significations que dans la formule (I), sur une amine de formule:

H₂N - R₇ (XVII)

dans laquelle R₇ représente un radical phényle éventuellement substitué par un ou plusieurs substituants choisis parmi les atomes d'halogène et les radicaux alkyle, alcoxy, alkylthio, hydroxy, mono ou polyhydroxyalkyle, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, alkylsufinyle, hydroxyaminocarbonyle, tétrazolyl-5 alkyle, tétrazolyl-5, sulfo, -alk-O-CO-alk, -alk-O-alk, -alk-COOX, -O-alk-COOX, -alk'-COOX, -CH=CH-COOX, -CO-COOX, -alk-SO₃H, -CH-CH-alk', -C(=NOH)-COOX et -S-alk-COOX.

Cette réaction s'effectue généralement au sein d'un solvant inerte tel que le tétrahydrofuranne, le diméthylformamide, un solvant chloré, un solvant aromatique (benzène, toluène par exemple) à une température comprise entre 20°C et la température d'ébullition du solvant.

Pour les amines de formule (XVII) comportant au moins un radical sulfo, il est préférable de protéger la fonction sulfo par toute méthode connue de l'homme de l'art par exemple sous forme d'un sel de tétra-n-butyl ammonium et de régénérer ensuite cette fonction.

Les dérivés de formule (XVI) peuvent être obtenus par action d'un dérivé aminé de formule (II) sur le N,N'-carbonyldiimidazole.

Cette réaction s'effectue au sein d'un solvant inerte tel que le tétrahydrofuranne, le diméthylformamide, un solvant chloré ou aromatique, à une température comprise entre 10°C et 50°C. Il est possible de ne pas isoler le composé de formule (XVI) et de le faire réagir in situ avec l'amine de formule (XVII).

Les amines de formule (XVII) sont commercialisées ou peuvent être obtenues par application ou adaptation des méthodes décrites par G. J. ESSELEN et coll., J. Am. Chem. Soc., 36, 322 (1914) ; G. ADRIANT et coll., Bull. Soc. Chim. Fr., 1511 (1970) ; W. A. JACOBS et coll., J. Am. Chem. Soc., 39, 2428, (1917) ; J. Am. Chem. Soc, 39, 1438 (1917) et dans les exemples.

Les composés de formule (I) pour lesquels R₄ représente un radical phénylamino dont le noyau phényle est substitué par au moins un radical carboxy, -alk-COOH, -O-alk-COOH, -alk'-COOH, -CH = CH-COOH, -CO-COOH, -C(=NOH)-COOH ou -S-alk-COOH peuvent également être préparés par hydrolyse des esters correspondants.

Cette hydrolyse s'effectue généralement au moyen d'une base telle que la soude ou la potasse, au sein d'un solvant inerte tel que le tétrahydrofuranne, le dioxanne, l'eau ou un mélange de ces solvants, à une température comprise entre 20 et 40°C ou au moyen d'acide trifluoroacétique, au sein d'un solvant inerte tel qu' un solvant chloré (dichlorométhane, chloroforme, dichloro-1,2 éthane par exemple), à une température comprise entre 20°C et la température d'ébullition du solvant.

Les composés de formule (I) pour lesquels R₄ représente un radical phénylamino dont le noyau phényle est substitué par au moins un radical amino à l'exception de ceux pour lesquels R₁ représente un radical phényle substitué par au moins un radical nitro, peuvent également être obtenus par réduction des dérivés nitrés correspondants.

Cette réduction s'effectue par toute méthode connue de l'homme de l'art permettant de passer d'un groupe nitro à un groupe amino. De préférence, on réduit au moyen d'hydrogène, en présence d'un catalyseur tel que le palladium sur noir, l'oxyde de platine, le nickel de Raney, au sein d'un solvant inerte tel qu'un alcool (méthanol, éthanol par exemple), à une température comprise entre 20 et 100°C.

Les composés de formule (I) pour lesquels R₄ représente un radical phénylamino dont le noyau phényle est substitué par au moins un radical phénylhydroxyméthyle peuvent également être obtenus par réduction des composés benzoylés correspondants.

Cette réduction s'effectue généralement au moyen d'un agent réducteur tel que le borohydrure de sodium, au sein d'un solvant inerte tel que le tétrahydrofuranne, un alcool (méthanol, éthanol par exemple) ou un mélange de ces solvants, à une température voisine de 25°C.

Les composés de formule (I) pour lesquels R₄ représente un radical phénylamino dont le noyau phényle est substitué par au moins un radical acyle peuvent également être préparés par oxydation des composés hydroxyalkylés correspondants.

Cette oxydation s'effectue de préférence au moyen d'un agent oxydant tel que le dioxyde de manganèse, au sein d'un solvant inerte tel qu'un solvant chloré (chloroforme, chlorure de méthylène par exemple), à une température voisine de 25°C.

Les composés de formule (I) pour lesquels R₄ représente un radical phénylamino dont le noyau phényle est substitué par au moins un radical hydroxyiminoalkyle, alcoxyiminoalkyle, hydroxyaminocarbonyle ou -C(=NOH)-COOX dans lequel X représente un radical alkyle peuvent également être obtenus par action des dérivés correspondants pour lesquels R₄ représente un radical phénylamino dont le noyau phényle est substitué par au moins un radical acyle, formyle, alcoxycarbonyle ou -CO-COOX dans lequel x représente un radical alkyle sur un dérivé de formule :

H₂N-OR₈ (XVIII)

dans laquelle R₈ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les dérivés intermédiaires formylés peuvent être obtenus par oxydation des dérivés hydroxyméthylés par adaptation de la méthode décrite précédemment pour la préparation des composés acylés et de la méthode décrite dans les exemples.

Les composés de formule (I) pour lesquels R₄ représente un radical phénylamino dont le noyau phényle est substitué par au moins un radical alkylsulfinyle peuvent également être préparés par oxydation des dérivés alkylthio correspondants.

Cette oxydation s'effectue de préférence au moyen d'acide m-chloro perbenzoïque, au sein d'un solvant inerte tel qu'un solvant chloré (chloroforme, chlorure de méthylène par exemple), à une température voisine de 25°C.

Les composés de formule (I) pour lesquels R₄ représente un radical phénylamino dont le noyau phényle est substitué par au moins un radical -alk-O-CO-alk peuvent également être préparés par action d'un chlorure d'acyle sur les composés hydroxyalkylés correspondants.

Cette réaction s'effectue généralement au sein d'un solvant anhydre tel qu'un solvant chloré (chloroforme, chlorure de méthylène par exemple), en présence d'une base telle qu'une trialkylamine, à une température comprise entre 0 et 50°C.

Les composés de formule (I) pour lesquels R₄ représente un radical phénylamino dont le noyau phényle est substitué par au moins un radical trifluorométhylsulfonamido peuvent également être préparés par action d'anhydride trifluorométhanesulfonique sur un composé correspondant pour lequel R₄ représente un radical phénylamino dont le noyau phényle est substitué par au moins un radical amino.

Cette réaction s'effectue généralement au sein d'un solvant inerte tel qu'un solvant chloré (chlorure de méthylène, chloroforme) en présence d'une base azotée telle que la triéthylamine, à une température voisine de 25°C.

Les composés de formule (I) pour lesquels R₄ représente un radical phényle éventuellement substitué, naphtyle, indolyle ou quinolyle peuvent être préparés par action d'un dérivé aminé de formule (II) sur un dérivé de formule :

HOOC - R₄ (XIX)

dans laquelle R₄ a les mêmes significations que précédemment ou un dérivé réactif de cet acide.

Cette réaction s'effectue dans les mêmes conditions que celles décrites précédemment pour la réaction d'une amine de formule (V) sur un acide de formule (VI).

Il est entendu pour l'homme de métier que, pour la mise en oeuvre des procédés selon l'invention décrits précédemment, il peut être nécessaire d'introduire des groupes protecteurs des fonctions amino afin d'éviter des réactions secondaires. Ces fonctions peuvent par exemple être bloquées sous forme de trifluorométhylacétamide puis régénérées par action de méthanol ammoniacal après avoir mis en oeuvre le procédé selon l'invention.

Les énantiomères des composés de formule (I) contenant au moins un site asymétrique peuvent être obtenus par dédoublement des racémiques par exemple par chromatographie sur colonne chirale selon W.H. PIRCKLE et coll., asymetric synthesis, vol.1, Academic Press (1983) ou par synthèse à partir des précurseurs chiraux.

Les composés de formule (I) peuvent être purifiés par les méthodes connues habituelles, par exemple par cristallisation, chromatographie ou extractions.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un groupe carboxy, sulfo ou alk-SO₃H peuvent également être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac ou d'une amine sur un composé de formule (I), dans un solvant tel qu'un alcool, un éther ou l'eau ou par réaction d'échange avec un sel d'un acide organique. Le sel formé est séparé par les méthodes habituelles.

Ces sels font également partie de l'invention.

Comme exemples de sels pharmaceutiquement acceptables, peuvent être citées les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères et des désordres de la motilité intestinale, de certaines tumeurs de l'oesophage inférieur, du colon et de l'intestin et comme régulateur de l'appétit.

Ces composés ont également un effet de potentialisation sur l'activité analgésique des médicaments narcotiques et non narcotiques.

L'affinité des composés de formule (I) pour les récepteurs CCK a été déterminée selon une technique inspirée de celle de A. SAITO et coll. (J. Neuro. Chem., 37, 483-490 (1981)) au niveau du cortex cérébral et au niveau du pancréas.

Dans ces tests, la CI50 des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989) ; REYFELD et coll., Am. J. Physiol., 240, G255-266 (1981) ; BEINFELD et coll., Neuropetides, 3, 411- 427 (1983)).

Les composés de formule (I) présentent une toxicité faible. Leur DL50 est généralement supérieure à 40 mg/kg par voie sous cutanée chez la souris.

D'un intérêt particulier sont les composés de formule (I) pour lesquels R₁ représente un atome d'hydrogène, R₂ représente un radical alkyle, R₃ représente un radical phényle ou bien R₂ et R₃ forment avec l'atome d'azote auquel ils sont rattachés un radical pipéridino substitué par au moins un radical alkyle, ou thiomorpholino substitué par au moins un radical alkyle et R₄ représente un radical phénylamino dont le noyau phényle est substitué par un ou plusieurs substituants choisis parmi les radicaux hydroxy, carboxy, hydroxyiminoalkyle, -alk-COOH, alkyle, alkylthio, monohydroxyalkyle et -C(=NOH)-COOH.

Les composés préférés sont les suivants :
- {[(hydroxyméthyl-3 phényl)-3 uréido]-2 N-phényl-acétamido}-2 N-méthyl N-phényl-acétamide
- {{[(hydroxyimino-1 éthyl)-3 phényl]-3 uréido}-2 N-phényl-acétamido}-2 N-méthyl N-phényl-acétamide-(E)
- acide {[N-(N-méthyl N-phényl-carbamoylméthyl) N-phényl-carbamoylméthyl]-3 uréido}-3 benzoïque
- {{[(hydroxy-2 éthyl)-3 phényl]-3 uréido}-2 N-phényl-acétamido}-2 N-méthyl N-phényl-acétamide
- acide {[N-(N-méthyl N-phényl-carbamoylméthyl) N-phényl-carbamoylméthyl]-3 uréido]-5 salicylique
- acide {[N-(N-méthyl N-phényl-carbamoylméthyl) N-phényl-carbamoylméthyl]-3 uréido}-3 phénylacétique
- N-méthyl {[(méthylthio-3 phényl)-3 uréido]-2 N-phényl-acétamido}-2 N-phényl-acétamide
- N-[(diméthyl-3,3 pipéridino)-2 oxo-2 éthyl] {[(hydroxy-1 éthyl)-3 phényl]-3 uréido}-2 N-phényl-acétamide-(RS)
- N-[(diméthyl-3,3 pipéridino)-2 oxo-2 éthyl] [(méthylthio-3 phényl)-3 uréido]-2 N-phényl-acétamide
- {[(hydroxyiminométhyl-3 phényl)-3 uréido}-2 N-phényl-acétamido}-2 N-méthyl N-phényl-acétamide-(E)
- acide {{[N-(N-méthyl N-phényl-carbamoylméthyl) N-phényl-carbamoylméthyl]-3 uréido}-3 phényl}-3 propionique
- acide hydroxyimino-2 {{[N-(N-méthyl N-phényl-carbamoylméthyl) N-phényl-carbamoylméthyl]-3 uréido}-3 phényl}-2 acétique (forme A)
- N-[(diméthyl-2,2 thiomorpholino)-2 oxo-2 éthyl] [(méthyl-3 phényl)-3 uréido]-2 N-phényl-acétamide.

Les exemples suivants, donnés à titre non limitatif, montrent comment l'invention peut être mise en pratique.

### EXEMPLE 1

A une solution de 0,3 g d'(amino-2 N-phényl-acétamido)-2 N-phényl-acétamide dans 10 cm3 de tétrahydrofuranne anhydre, on ajoute à une température voisine de 25°C, 0,15 g d'isocyanate de méthyl-3 phényle. La suspension obtenue est agitée pendant 12 heures à une température voisine de 25°C et le produit insoluble est séparé par filtration. On obtient, après recristallisation dans l'acétonitrile, 0,3 g de {[(méthyl-3 phényl)-3 uréido]-2 acétamido}-2 N-phényl-acétamide fondant à 210°C.

L'(amino-2 N-phényl-acétamido)-2 N-phényl-acétamide peut être préparé de la manière suivante : à une solution de 1,2 g de (N-phényl phtalimido-2 acétamido)-2 N-phényl-acétamide dans 15 cm3 de méthanol, on ajoute 0,29 g d'hydrate d'hydrazine. Le mélange réactionnel est agité à reflux pendant 2 heures. Après refroidissement et addition de 5 cm3 d'une solution aqueuse 4N d'acide chlorhydrique, le produit insoluble est séparé par filtration. Le filtrat est concentré à sec sous pression réduite (2,7 kPa) à 30°C. Le résidu obtenu est dissous dans 10 cm3 d'eau distillée, puis la solution obtenue est lavée par 10 cm3 d'éther diéthylique, alcalinisée avec 0,5 g de soude en pastilles et extraite par 2 fois 20 cm3 d'acétate d'éthyle. Les phases organiques réunies sont séchées sur sulfate de magnésium puis concentrées à sec sous pression réduite (2,7 kPa) à 30°C. On obtient ainsi 0,3 g d'(amino-2 N-phényl-acétamido)-2 N-phényl-acétamide sous forme d'une huile utilisée telle quelle dans les synthèses ultérieures.

Le (N-phényl phtalimido-2 acétamido)-2 N-phényl-acétamide peut être préparé de la manière suivante : à une suspension de 1,7 g d'acide (N-phényl phtalimido-2 acétamido)-2 acétique dans 25 cm3 de dichloro-1,2 éthane on ajoute 0,7 g de dichlorure d'oxalyle puis une goutte de diméthylformamide. Le mélange est agité 2 heures à une température voisine de 25°C, puis on ajoute 1,12 g d'aniline en solution dans 10 cm3 de dichloro-1,2 éthane. La solution obtenue est agitée 2 heures à une température voisine de 25°C, puis lavée par 2 fois 30 cm3 d'eau distillée, séchée sur sulfate de magnésium et concentrée à sec sous pression réduite (2,7 kPa) à 40°C. On obtient, après recristallisation dans l'acétonitrile, 1,2 g de (N-phényl phtalimido-2 acétamido)-2 N-phényl-acétamide fondant à 140°C.

L'acide (N-phényl phtalimido-2 acétamido)-2 acétique peut être préparé de la manière suivante : à une solution de 8 g de (N-phényl phtalimido-2 acétamido)-2 acétate de tert-butyle dans 30 cm3 de dichlorométhane, on ajoute 17,9 g d'acide trifluoroacétique. La solution obtenue est agitée à reflux pendant 1 heure, puis concentrée à sec sous pression réduite (2,7 kPa) à 40°C. On obtient, après recristallisation dans l'éther diisopropylique, 5,9 g d'acide (N-phényl phtalimido-2 acétamido)-2 acétique fondant à 224°C.

Le (N-phényl phtalimido-2 acétamido)-2 acétate de tert-butyle peut être préparé de la manière suivante : à une solution de 207 g de N-phényl glycinate de tert-butyle dans 500 cm3 de dichloro-1,2 éthane, on ajoute 92,4 g d'hydrogénocarbonate de sodium. La suspension est agitée à une température voisine de 5°C et on ajoute une solution de 223 g de chlorure de phtalimido-2 acétyle dans 1100 cm3 de dichloro-1,2 éthane. Le mélange réactionnel est agité à reflux pendant 4 heures. Après séparation du produit insoluble par filtration, le filtrat est lavé par 300 cm3 d'eau distillée, séché sur sulfate de magnésium et concentré à sec sous pression réduite (2,7 kPa) à 40°C. On obtient, après recristallisation dans l'acétonitrile 236 g de (N-phényl phtalimido-2 acétamido)-2 acétate de tert-butyle fondant à 128°C.

Le N-phényl glycinate de tert-butyle peut être préparé de la manière suivante : à une solution de 56 g d'aniline dans 600 cm3 de dichloro-1,2 éthane, on ajoute 58 g de bromoacétate de tert-butyle et la solution obtenue est agitée à reflux pendant 48 heures. Après refroidissement, le produit insoluble est séparé par filtration et le filtrat est lavé par 200 cm3 d'une solution aqueuse d'acide chlorhydrique 0,1N et par 3 fois 200 cm3 d'eau distillée. La phase organique est séchée sur sulfate de magnésium puis concentrée à sec sous pression réduite (2,7 kPa) à 40°C. On obtient ainsi 54 g de N-phényl glycinate de tert-butyle sous forme d'une huile utilisée telle quelle dans les synthèses ultérieures.

Le chlorure de phtalimido-2 acétyle peut être préparé selon la méthode décrite par W. GRASSMANN et E. SCHULTE-UEBLING, Chem. Ber., 83, 244, (1950).

### EXEMPLE 2

On opère d'une manière analogue à celle décrite à l'exemple 1, mais à partir de 0,9 g d'(amino-2 N-phényl-acétamido)-2 N-méthyl N-phényl-acétamide et de 0,4 g d'isocyanate de méthyl-3 phényle. On obtient après recristallisation dans un mélange d'oxyde de diéthyle et d'acétate d'éthyle (60-40 en volumes), 0,4 g de N-méthyl N-phényl {[(méthyl-3 phényl)-3 uréido]-2 N-phényl-acétamido}-2 acétamide fondant à 168°C.

L'(amino-2 N-phényl-acétamido)-2 N-méthyl N-phényl-acétamide peut être préparé d'une manière analogue à celle décrite à l'exemple 1, pour la préparation de l'(amino-2 N-phényl-acétamido)-2 N-phényl-acétamide, mais à partir de 1,4 g de N-méthyl N-phényl (N-phényl phtalimido-2 acétamido)-2 acétamide et de 0,25 g d'hydrate d'hydrazine. On obtient ainsi, 0,9 g d'(amino-2 N-phényl-acétamido)-2 N-méthyl N-phényl-acétamide sous forme d'une huile utilisée telle quelle dans les synthèses ultérieures.

Le N-méthyl N-phényl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 10,1 g d'acide (N-phényl phtalimido-2 acétamido)-2 acétique, de 3,9 g de dichlorure d'oxalyle et de 7,7 g de N-méthyl aniline. On obtient, après recristallisation dans l'oxyde de diisopropyle, 9,6 g de N-méthyl N-phényl (N-phényl phtalimido-2 acétamido) acétamide fondant à 216°C.

### EXEMPLE 3

On opère d'une manière analogue à celle décrite à l'exemple 1, mais à partir de 2,2 g d'(amino-2 N-phényl-acétamido)-2 N-(chloro-4 phényl) N-méthyl-acétamide et de 0,87 g d'isocyanate de méthyl-3 phényle. Le produit obtenu est purifié par chromatographie sur 60 g de silice (0,065-0,200 mm) contenus dans une colonne de 2,5 cm de diamètre [éluant : chlorure de méthylène-méthanol (99-1 en volumes)] en recueillant des fractions de 20 cm3. Les fractions 30 à 43 sont réunies et concentrées à sec sous pression réduite (2,7 kPa) à 40 °C. On obtient, après recristallisation dans l'acétonitrile 1,0 g de N-(chloro-4 phényl) N-méthyl {[(méthyl-3 phényl)-3 uréido]-2 N-phényl-acétamido}-2 acétamide fondant à 160°C.

L'(amino-2 N-phényl-acétamido)-2 N-(chloro-4 phényl) N-méthyl-acétamide peut être préparé d'une manière analoque à celle décrite à l'exemple 1, pour la préparation de l'(amino-2 N-phényl-acétamido)-2 N-phényl-acétamide, mais à partir de 11 g de N-(chloro-4 phényl) N-méthyl (N-phényl phtalimido-2 acétamido)-2 acétamide et de 2,4 g d'hydrate d'hydrazine. On obtient ainsi 6,6 g d' (amino-2 N-phényl-acétamido)-2 N-(chloro-4 phényl) N-méthyl-acétamide sous forme d'une huile utilisée telle quelle dans les synthèses ultérieures.

Le N-(chloro-4 phényl) N-méthyl (N-phényl phtalimido-2 acétamido)-2 acétamide peut être préparé d'une manière analoque à celle décrite à l'exemple 1, pour la préparation du (N-phényl phtalimido-2 acétamido)-2 N-phényl-acétamide, mais à partir de 10,1 g d'acide (N-phényl phtalimido-2 acétamido)-2 acétique, de 3,9 g de dichlorure d'oxalyle et de 10,1 g de chloro-4 N-méthyl aniline. On obtient, après recristallisation dans l'oxyde de diisopropyle, 11,0 g de N-(chloro-4 phényl) N-méthyl (N-phényl phtalimido-2 acétamido)-2 acétamide fondant à 180°C.

### EXEMPLE 4

On opère d'une manière analogue à celle décrite à l'exemple 1, mais à partir de 1,2 g d'(amino-2 N-phényl-acétamido)-2 N-(difluoro-2,4 phényl) N-méthyl-acétamide et de 0,46 g d'isocyanate de méthyl-3 phényle. Le produit obtenu est purifié par chromatographie sur 70 g de silice (0,065-0,200 mm) contenus dans une colonne de 2,5 cm de diamètre [éluant : dichlorométhane - méthanol (99-1 en volumes)] en recueillant des fractions de 15 cm3. Les fractions 24 à 36 sont réunies et concentrées à sec sous pression réduite (2,7 kPa) à 40 °C. On obtient, après recristallisation dans l'oxyde de diisopropyle, 0,6 g de N-(difluoro-2,4 phényl) N-méthyl {[(méthyl-3 phényl)-3 uréido]-2 N-phényl-acétamido}-2 acétamide fondant à 100°C.

L'(amino-2 N-phényl-acétamido)-2 N-(difluoro-2,4 phényl) N-méthyl-acétamide peut être préparé d'une manière analogue à celle décrite à l'exemple 1, pour la préparation de l'(amino-2 N-phénylacétamido)-2 N-phényl-acétamide, mais à partir de 2,2 g de N-(difluoro-2,4 phényl) N-méthyl (N-phényl phtalimido-2 acétamido)-2 acétamide et de 0,47 g d'hydrate d'hydrazine. On obtient ainsi, 1,2 g d'(amino-2 N-phényl-acétamido)-2 N-(difluoro-2,4 phényl) N-méthyl-acétamide sous forme d'une huile utilisée telle quelle dans les synthèses ultérieures.

Le N-(difluoro-2,4 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 4,6 g d'acide (N-phényl phtalimido-2 acétamido)-2 acétique, de 1,7 g de dichlorure d'oxalyle et de 3,9 g de difluoro-2,4 N-méthyl aniline. Le produit obtenu est purifié par chromatographie sur 100 g de silice (0,065-0,200 mm) contenus dans une colonne de 3,5 cm de diamètre [éluant : dichlorométhane - méthanol (99-1 en volumes)] en recueillant des fractions de 20 cm3. Les fractions 14 à 23 sont réunies et concentrées à sec sous pression réduite (2,7 kPa) à 40°C. On obtient 2,2 g de N-(difluoro-2,4 phényl) N-méthyl (N-phényl phtalimido-2 acétamido)-2 acétamide sous forme d'une meringue utilisée telle quelle dans les synthèses ultérieures.

### EXEMPLE 5

On opère d'une manière analogue à celle décrite à l'exemple 1, mais à partir de 1,9 g d'(amino-2 N-phényl-acétamido)-2 N-(méthoxy-4 phényl) N-méthyl-acétamide et de 0,77 g d'isocyanate de méthyl-3 phényle. Le produit obtenu est purifié par chromatographie sur 70 g de silice (0,065-0,200 mm) contenus dans une colonne de 2,5 cm de diamètre [éluant : acétate d'éthyle - cyclohexane (80-20 en volumes)] en recueillant des fractions de 20 cm3. Les fractions 14 à 26 sont réunies et concentrées à sec sous pression réduite (2,7 kPa) à 40 °C. On obtient, après recristallisation dans l'acétonitrile, 1,0 g de N-(méthoxy-4 phényl) N-méthyl {[(méthyl-3 phényl)-3 uréido]-2 N-phényl-acétamido}-2 acétamide fondant à 202°C.

L'(amino-2 N-phényl-acétamido)-2 N-(méthoxy-4 phényl) N-méthyl-acétamide peut être préparé d'une manière analogue à celle décrite à l'exemple 1, pour la préparation de l'(amino-2 N-phénylacétamido)-2 N-phényl-acétamide, mais à partir de 3,8 g de N-(méthoxy-4 phényl) N-méthyl (N-phényl phtalimido-2 acétamido)-2 acétamide et de 0,83 g d'hydrate d'hydrazine. On obtient ainsi, 1,9 g d'(amino-2 N-phényl-acétamido)-2 N-(méthoxy-4 phényl) N-méthylacétamide sous forme d'une huile utilisée telle quelle dans les synthèses ultérieures.

Le N-(méthoxy-4 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 5,0 g d'acide (N-phényl phtalimido-2 acétamido)-2 acétique, de 1,86 g de dichlorure d'oxalyle et de 2,4 g de méthoxy-4 N-méthyl aniline. On obtient ainsi, 3,8 g de N-(méthoxy-4 phényl) N-méthyl (N-phényl phtalimido-2 acétamido)-2 acétamide fondant à 162°C.

### EXEMPLE 6

On opère d'une manière analogue à celle décrite à l'exemple 1, mais à partir de 3,3 g d'amino-2 N-[oxo-2 (diméthyl-3,3 pipéridino)-2 éthyl] N-phényl-acétamide et de 1,6 g d'isocyanate de méthyl-3 phényle. On obtient après recristallisation dans l'acétonitrile, 3,2 g de N-[oxo-2 (diméthyl-3,3 pipéridino)-2 éthyl] [(méthyl-3 phényl)-3 uréido]-2 N-phényl-acétamide fondant à 170°C.

L'amino-2 N-[oxo-2 (diméthyl-3,3 pipéridino)-2 éthyl] N-phényl-acétamide peut être préparé d'une manière analogue à celle décrite à l'exemple 1, pour la préparation de l'(amino-2 N-phénylacétamido)-2 N-phényl-acétamide, mais à partir de 5,0 g de N-[oxo-2 (diméthyl-3,3 pipéridino)-2 éthyl] N-phényl phtalimido-2 acétamide et de 1,2 g d'hydrate d'hydrazine. On obtient ainsi, 3,3 g d'amino-2 N-[oxo-2 (diméthyl-3,3 pipéridino)-2 éthyl] N-phényl-acétamide sous forme d'une huile utilisée telle quelle dans les synthèses ultérieures.

Le N-[oxo-2 (diméthyl-3,3 pipéridino)-2 éthyl] N-phényl phtalimido-2 acétamide peut être préparé d'une manière analoque à celle décrite à l'exemple 1, pour la préparation du (N-phényl phtalimido-2 acétamido)-2 N-phényl-acétamide, mais à partir de 6,8 g d'acide (N-phényl phtalimido-2 acétamido)-2 acétique, de 2,8 g de dichlorure d'oxalyle et de 5,4 g de diméthyl-3,3 pipéridine. On obtient ainsi, après recristallisation dans un mélange acétate d'éthyle - éther diisopropylique (50-50 en volumes), 5,0 g de N-[oxo-2 (diméthyl-3,3 pipéridino)-2 éthyl] N-phényl phtalimido-2 acétamide fondant à 156°C.

### EXEMPLE 7

On opère d'une manière analogue à celle décrite à l'exemple 1, mais à partir de 2,8 g d'amino-2 N-[(méthyl-4 pipéridino)-2 oxo-2 éthyl] N-phényl-acétamide et de 1,3 g d'isocyanate de méthyl-3 phényle. Le produit obtenu est purifié par chromatographie sur 80 g de silice (0,065-0,200 mm) contenus dans une colonne de 2,5 cm de diamètre [éluant : chlorure de méthylène-méthanol (95-5 en volumes)] en recueillant des fractions de 20 cm3. Les fractions 20 à 36 sont réunies et concentrées à sec sous pression réduite (2,7 kPa) à 40 °C. On obtient, après recristallisation dans l'acétonitrile 1,4 g de N-[(méthyl-4 pipéridino)-2 oxo-2 éthyl] [(méthyl-3 phényl)-3 uréido]-2 N-phényl-acétamide fondant à 180°C.

L'amino-2 N-[(méthyl-4 pipéridino)-2 oxo-2 éthyl] N-phényl-acétamide peut être préparé d'une manière analogue à celle décrite à l'exemple 1 pour la préparation de l'(amino-2 N-phénylacétamido)-2 N-phényl-acétamide, mais à partir de 5,7 g de N-[(méthyl-4 pipéridino)-2 oxo-2 éthyl] N-phényl phtalimido-2 acétamide et de 1,45 g d'hydrate d'hydrazine. On obtient ainsi, 2,8 g d'amino-2 N-[(méthyl-4 pipéridino)-2 oxo-2 éthyl] N-phényl-acétamide sous forme d'une huile utilisée telle quelle dans les synthèses ultérieures.

Le N-[(méthyl-4 pipéridino)-2 oxo-2 éthyl] N-phényl phtalimido-2 acétamide peut être préparé d'une manière analoque à celle décrite à l'exemple 1, pour la préparation du (N-phényl phtalimido-2 acétamido)-2 N-phényl-acétamide, mais à partir de 10,1 g d'acide (N-phényl phtalimido-2 acétamido)-2 acétique, de 3,9 g de dichlorure d'oxalyle et de 7,1 g de méthyl-4 pipéridine. On obtient, après recristallisation dans l'éther diisopropylique, 6,0 g de N-[(méthyl-4 pipéridino)-2 oxo-2 éthyl] N-phényl phtalimido-2 acétamide fondant à 138°C.

### EXEMPLE 8

On opère d'une manière analogue à celle décrite à l'exemple 1, mais à partir de 1,45 g d'amino-2 N-[(méthyl-2 pipéridino)-2 oxo-2 éthyl] N-phényl-acétamide-(RS) et de 0,67 g d'isocyanate de méthyl-3 phényle. On obtient après recristallisation dans un mélange d'éthanol et d'éther diisopropylique (50-50 en volumes), 0,80 g de N-[(méthyl-2 pipéridino)-2 oxo-2 éthyl] [(méthyl-3 phényl)-3 uréido]-2 N-phényl-acétamide-(RS) fondant à 188°C.

L'amino-2 N-[(méthyl-2 pipéridino)-2 oxo-2 éthyl] N-phényl-acétamide-(RS) peut être préparé d'une manière analogue à celle décrite à l'exemple 1, pour la préparation de l'(amino-2 N-phényl-acétamido)-2 N-phényl-acétamide, mais à partir de 4,5 g de N-[(méthyl-2 pipéridino)-2 oxo-2 éthyl] N-phényl phtalimido-2 acétamide-(RS) et de 1,1 g d'hydrate d'hydrazine. On obtient ainsi, 2,9 g d'amino-2 N-[(méthyl-2 pipéridino)-2 oxo-2 éthyl] N-phényl-acétamide-(RS) sous forme d'une huile utilisée telle quelle dans les synthèses ultérieures.

Le N-[(méthyl-2 pipéridino)-2 oxo-2 éthyl] N-phényl phtalimido-2 acétamide-(RS) peut être préparé d'une manière analogue à celle décrite à l'exemple 1, pour la préparation du (N-phényl phtalimido-2 acétamido)-2 N-phényl-acétamide, mais à partir de 5,1 g d'acide (N-phényl phtalimido-2 acétamido)-2 acétique, de 2,1 g de dichlorure d'oxalyle et de 3,6 g de méthyl-2 pipéridine-(RS). On obtient, après recristallisation dans l'éther diisopropylique, 4,4 g de N-[(méthyl-2 pipéridino)-2 oxo-2 éthyl] N-phényl phtalimido-2 acétamide-(RS) fondant à 110°C.

### EXEMPLE 9

On opère d'une manière analogue à celle décrite à l'exemple 1, mais à partir de 1,0 g d'amino-2 N-[(méthyl-3 pipéridino)-2 oxo-2 éthyl] N-phényl-acétamide-(RS) et de 0,47 g d'isocyanate de méthyl-3 phényle. On obtient après recristallisation dans un mélange d'éthanol et d'éther diisopropylique (50-50 en volumes), 0,66 g de N-[(méthyl-3 pipéridino)-2 oxo-2 éthyl] N-[(méthyl-3 phényl)-3 uréido]-2 N-phényl-acétamide-(RS) fondant à 180°C.

L'amino-2 N-[(méthyl-3 pipéridino)-2 oxo-2 éthyl] N-phényl-acétamide-(RS) peut être préparé d'une manière analogue à celle décrite à l'exemple 1 pour la préparation de l'(amino-2 N-phényl-acétamido)-2 N-phényl-acétamide, mais à partir de 3,3 g de N-[(méthyl-3 pipéridino)-2 oxo-2 éthyl] N-phényl phtalimido-2 acétamide-(RS) et de 0,8 g d'hydrate d'hydrazine. On obtient ainsi, 2 g d'amino-2 N-[(méthyl-3 pipéridino)-2 oxo-2 éthyl] N-phényl-acétamide-(RS) sous forme d'une huile utilisée telle quelle dans les synthèses ultérieures.

Le N-[(méthyl-3 pipéridino)-2 oxo-2 éthyl] N-phényl phtalimido-2 acétamide-(RS) peut être préparé d'une manière analogue à celle décrite à l'exemple 1, pour la préparation du (N-phényl phtalimido-2 acétamido)-2 N-phényl-acétamide, mais à partir de 5,1 g d'acide (N-phényl phtalimido-2 acétamido)-2 acétique, de 2,1 g de dichlorure d'oxalyle et de 3,6 g de méthyl-3 pipéridine-(RS). On obtient, après recristallisation dans l'éther diisopropylique, 3,3 g de N-[(méthyl-3 pipéridino)-2 oxo-2 éthyl] N-phényl phtalimido-2 acétamide-(RS) fondant à 134 °C.

### EXEMPLE 10

On opère d'une manière analogue à celle décrite à l'exemple 1, mais à partir de 1,2g d'amino-2 N-[oxo-2 (perhydroazépinyl-1)-2 éthyl] N-phényl-acétamide et de 0,55 g d'isocyanate de méthyl-3 phényle. On obtient après recristallisation dans un mélange de diméthylformamide et d'acétate d'éthyle (65-35 en volumes), 0,9g de N-[oxo-2 (perhydroazépinyl-1)-2 éthyl] [(méthyl-3 phényl)-3 uréido]-2 N-phényl-acétamide fondant à 245°C.

L'amino-2 N-[oxo-2 (perhydroazépinyl-1)-2 éthyl] N-phényl-acétamide peut être préparé d'une manière analogue à celle décrite à l'exemple 1, pour la préparation de l'(amino-2 N-phényl-acétamido)-2 N-phényl-acétamide, mais à partir de 2,1g de N-[oxo-2 (perhydroazépinyl-1)-2 éthyl] N-phényl phtalimido-2 acétamide et de 0,5 g d'hydrate d'hydrazine. On obtient ainsi, 1,2 g d'amino-2 N-[oxo-2 (perhydroazépinyl-1)-2 éthyl] N-phényl-acétamide sous forme d'une huile utilisée telle quelle dans les synthèses ultérieures.

Le N-[oxo-2 (perhydroazépinyl-1)-2 éthyl] N-phényl phtalimido-2 acétamide peut être préparé d'une manière analogue à celle décrite à l'exemple 1 pour la préparation du (N-phényl phtalimido-2 acétamido)-2 N-phényl-acétamide, mais à partir de 10,1g d'acide (N-phényl phtalimido-2 acétamido)-2 acétique, de 4,2g de dichlorure d'oxalyle et de 6g de perhydroazépine. On obtient ainsi, après recristallisation dans l'acétate d'éthyle, 3,8g de N-[oxo-2 (perhydroazépinyl-1)-2 éthyl] N-phényl phtalimido-2 acétamide fondant à 140°C.

### EXEMPLE 11

On opère d'une manière analogue à celle décrite à l'exemple 1, mais à partir de 1,8g d'amino-2 N-[(indolinyl-1)-2 oxo-2 éthyl] N-phényl-acétamide et de 0,8g d'isocyanate de méthyl-3 phényle. On obtient après recristallisation dans un mélange d'acétate d'éthyle et de diméthylformamide (80-20 en volumes), 0,7g de N-[(indolinyl-1)-2 oxo-2 éthyl] [(méthyl-3 phényl)-3 uréido]-2 N-phényl-acétamide fondant à 190°C.

L'amino-2 N-[(indolinyl-1)-2 oxo-2 éthyl] N-phényl-acétamide peut être préparé d'une manière analogue à celle décrite à l'exemple 1, pour la préparation de l'(amino-2 N-phényl-acétamido)-2 N-phényl-acétamide,, mais à partir de 3,7g de N-[(indolinyl-1)-2 oxo-2 éthyl] N-phényl phtalimido-2 acétamide et de 1,7g d'hydrate d'hydrazine. On obtient ainsi, 1,8g d'amino-2 N-[(indolinyl-1)-2 oxo-2 éthyl] N-phényl-acétamide sous forme d'une huile utilisée telle quelle dans les synthèses ultérieures.

Le N-[(indolinyl-1)-2 oxo-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 4 g d'acide (N-phényl phtalimido-2 acétamido)-2 acétique, de 1,7g de dichlorure d'oxalyle et de 2,8g d'indoline. On obtient ainsi, 3,7g de N-[(indolinyl-1)-2 oxo-2 éthyl] N-phényl phtalimido-2 acétamide fondant à 230 °C.

### EXEMPLE 12

On opère d'une manière analogue à celle décrite à l'exemple 1, mais à partir de 2,0 g d'amino-2 N-[oxo-2 (tétrahydro-1,2,3,6 pyridyl-1)-2 éthyl] N-phényl-acétamide et de 0,97 g d'isocyanate de méthyl-3 phényle. On obtient après recristallisation dans l'acétonitrile, 1,4 g de N-[oxo-2 (tétrahydro-1,2,3,6 pyridyl-1)-2 éthyl] [(méthyl-3 phényl)-3 uréido]-2 N-phényl-acétamide fondant à 171°C.

L'amino-2 N-[oxo-2 (tétrahydro-1,2,3,6 pyridyl-1)-2 éthyl] N-phényl-acétamide peut être préparé d'une manière analogue à celle décrite à l'exemple 1, pour la préparation de l'(amino-2 N-phénylacétamido)-2 N-phényl-acétamide, mais à partir de 3,6 g de N-[oxo-2 (tétrahydro-1,2,3,6 pyridyl-1)-2 éthyl] N-phényl phtalimido-2 acétamide et de 0,92 g d'hydrate d'hydrazine. On obtient ainsi, 2,1 g d'amino-2 N-[oxo-2 (tétrahydro-1,2,3,6 pyridyl-1)-2 éthyl] N-phényl-acétamide sous forme d'une huile utilisée telle quelle dans les synthèses ultérieures.

Le N-[oxo-2 (tétrahydro-1,2,3,6 pyridyl-1)-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 5,1 g d'acide (N-phényl phtalimido-2 acétamido)-2 acétique, de 2,1 g de dichlorure d'oxalyle et de 3,7 g de tétrahydro-1,2,3,6 pyridine. On obtient ainsi, après recristallisation dans l'éthanol 3,7 g de N-[oxo-2 (tétrahydro-1,2,3,6 pyridyl-1)-2 éthyl] N-phényl phtalimido-2 acétamide fondant à 180°C.

### EXEMPLE 13

On opère d'une manière analogue à celle décrite à l'exemple 1, mais à partir de 0,9 g d'(amino-2 N-phényl-acétamido)-2 N-cyclopropylméthyl-acétamide et de 0,45 g d'isocyanate de méthyl-3 phényle. On obtient après recristallisation dans l'acétonitrile 1g de N-cyclopropylméthyl {[(méthyl-3 phényl)-3 uréido]-2 N-phényl-acétamido}-2 acétamide fondant à 168°C.

L'(amino-2 N-phényl-acétamido)-2 N-cyclopropylméthylacétamide peut être préparé d'une manière analogue à celle décrite à l'exemple 1, pour la préparation de l'(amino-2 N-phényl-acétamido)-2 N-phényl-acétamide, mais à partir de 1,8 g de N-cyclopropylméthyl (N-phényl phtalimido-2 acétamido)-2 acétamide et de 0,46 g d'hydrate d'hydrazine. On obtient ainsi, 0,9 g d'(amino-2 N-phényl-acétamido)-2 N-cyclopropylméthyl-acétamide sous forme d'une huile utilisée telle quelle dans les synthèses ultérieures.

Le N-cyclopropylméthyl (N-phényl phtalimido-2 acétamido)-2 acétamide peut être préparé de la manière suivante : à une suspension de 1,7 g d'acide (N-phényl phtalimido-2 acétamido)-2 acétique dans 20 cm3 de tétrahydrofuranne anhydre on ajoute 0,8 g de N,N'-diimidazole carbonyle. Le mélange est agité 1 heure à une température voisine de 25°C puis on ajoute en une seule fois un mélange de 0,65 g de chlorhydrate de cyclopropylamine et de 0,62 g de triéthylamine dans 15 cm3 tétrahydrofuranne anhydre. Le mélange réactionnel est agité à reflux pendant 8 heures puis concentré à sec sous pression réduite (2,7 kPa) à 40°C. Le résidu est dissous dans 25 cm3 d'acétate d'éthyle et la solution obtenue est lavée par 2 fois 15 cm3 d'eau distillée, séchée sur sulfate de magnésium puis concentrée à sec sous pression réduite (2,7 kPa) à 40°C. On obtient, après recristallisation dans l'éther diéthylique, 1,5 g de N-cyclopropylméthyl (N-phényl phtalimido-2 acétamido)-2 acétamide fondant à 144°C.

### EXEMPLE 14

On opère d'une manière analogue à celle décrite à l'exemple 1, mais à partir de 1,0 g d'(amino-2 N-phényl-acétamido)-2 N-tert-butyl-acétamide et de 0,55 g d'isocyanate de méthyl-3 phényle. On obtient après recristallisation dans l'acétonitrile, 1,0 g de N-tert-butyl {[(méthyl-3 phényl)-3 uréido]-2 N-phényl-acétamido}-2 acétamide fondant à 214°C.

L'(amino-2 N-phényl-acétamido)-2 N-tert-butyl-acétamide peut être préparé d'une manière analogue à celle décrite à l'exemple 1, pour la préparation de l'(amino-2 N-phényl-acétamido)-2 N-phényl-acétamide, mais à partir de 1,6 g de N-tert-butyl (N-phényl phtalimido-2 acétamido)-2 acétamide et de 0,23 g d'hydrate d'hydrazine. On obtient ainsi, 1,1 g d'(amino-2 N-phényl-acétamido)-2 N-tert-butyl-acétamide sous forme d'une meringue utilisée telle quelle dans les synthèses ultérieures.

Le N-tert-butyl (N-phényl phtalimido-2 acétamido)-2 acétamide, peut être préparé d'une manière analogue à celle décrite à l'exemple 1, pour la préparation du (N-phényl phtalimido-2 acétamido)-2 N-phényl-acétamide, mais à partir de 1,7 g d'acide (N-phényl phtalimido-2 acétamido)-2 acétique, de 0,64 g de dichlorure d'oxalyle et de 0,8 g de tert-butylamine. On obtient ainsi 1,65 g de N-tert-butyl (N-phényl phtalimido-2 acétamido)-2 acétamide fondant à 166°C.

### EXEMPLE 15

On opère d'une manière analogue à celle décrite à l'exemple 1, mais à partir de 0,25 g d'(amino-2 N-phényl-acétamido)-2 N-benzyl-acétamide et de 0,11 g d'isocyanate de méthyl-3 phényle. On obtient après recristallisation dans l'acétonitrile, 0,17 g de N-benzyl {[(méthyl-3 phényl)-3 uréido]-2 N-phényl-acétamido}-2 acétamide fondant à 204°C.

L'(amino-2 N-phényl-acétamido)-2 N-benzyl-acétamide peut être préparé d'une manière analogue à celle décrite à l'exemple 1, pour la préparation de l'(amino-2 N-phényl-acétamido)-2 N-phényl acétamide, mais à partir de 1,0 g de N-benzyl (N-phényl phtalimido-2 acétamido)-2 acétamide et de 0,23 g d'hydrate d'hydrazine. On obtient ainsi, 0,25 g d'(amino-2 N-phényl-acétamido)-2 N-benzyl-acétamide sous forme d'une huile utilisée telle quelle dans les synthèses ultérieures.

Le N-benzyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 1,0 g d'acide (N-phényl phtalimido-2 acétamido)-2 acétique, de 0,38 g de dichlorure d'oxalyle et de 0,59 g de benzylamine. On obtient ainsi 1,0 g de N-benzyl (N-phényl phtalimido-2 acétamido)-2 acétamide sous forme d'une huile utilisée telle quelle dans les synthèses ultérieures.

### EXEMPLE 16

On opère d'une manière analogue à celle décrite à l'exemple 1, mais à partir de 3,0 g d'(amino-2 N-phényl-acétamido)-2 N-(chloro-4 phényl) acétamide et de 1,2 g d'isocyanate de méthyl-3 phényle. On obtient après recristallisation dans le tétrahydrofuranne, 2,3 g de N-(chloro-4 phényl) {[(méthyl-3 phényl)-3 uréido]-2 N-phényl-acétamido}-2 acétamide fondant à 220°C.

L'(amino-2 N-phényl-acétamido)-2 N-(chloro-4 phényl) acétamide peut être préparé d'une manière analogue à celle décrite à l'exemple 1, pour la préparation de l'(amino-2 N-phényl-acétamido)-2 N-phényl-acétamide, mais à partir de 3,0 g de N-(chloro-4 phényl) (N-phényl phtalimido-2 acétamido)-2 acétamide et de 0,9 g d'hydrate d'hydrazine. On obtient ainsi, 3,0 g d'(amino-2 N-phényl-acétamido)-2 N-(chloro-4 phényl) acétamide sous forme d'une huile utilisée telle quelle dans les synthèses ultérieures.

Le N-(chloro-4 phényl) (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 4,0 g d'acide (N-phényl phtalimido-2 acétamido)-2 acétique, de 1,7 g de dichlorure d'oxalyle et de 3,0 g de chloro-4 aniline. On obtient, après recristallisation dans un mélange acétate d'éthyle - éther diisopropylique (50-50 en volumes), 4,0 g de N-(chloro-4 phényl) (N-phényl phtalimido-2 acétamido)-2 acétamide fondant à 200°C.

### EXEMPLE 17

On opère d'une manière analogue à celle décrite à l'exemple 1, mais à partir de 1,9 g d'(amino-2 N-phényl-acétamido)-2 N-(méthyl-4 phényl) acétamide et de 0,85 g d'isocyanate de méthyl-3 phényle. On obtient après recristallisation dans l'acétonitrile, 1,3 g de N-(méthyl-4 phényl) {[(méthyl-3 phényl)-3 uréido]-2 N-phényl-acétamido}-2 acétamide fondant à 170°C.

L'(amino-2 N-phényl acétamido)-2 N-(méthyl-4 phényl) acétamide peut être préparé d'une manière analogue à celle décrite à l'exemple 1, pour la préparation de l'(amino-2 N-phényl-acétamido)-2 N-phényl-acétamide, mais à partir de 3,1 g de N-(méthyl-4 phényl) (N-phényl phtalimido-2 acétamido)-2 acétamide et de 0,73 g d'hydrate d'hydrazine. On obtient ainsi, 1,9 g d'(amino-2 N-phényl-acétamido)-2 N-(méthyl-4 phényl) acétamide sous forme d'une huile utilisée telle quelle dans les synthèses ultérieures.

Le N-(méthyl-4 phényl) (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 4,0 g d'acide (N-phényl phtalimido-2 acétamido)-2 acétique, de 1,7 g de dichlorure d'oxalyle et de 2,5 g de méthyl-4 aniline. On obtient, après recristallisation dans un mélange acétate d'éthyle - éther diisopropylique (50-50 en volumes), 3,1 g de N-(méthyl-4 phényl) (N-phényl phtalimido-2 acétamido)-2 acétamide fondant à 185°C.

### EXEMPLE 18

On opère d'une manière analogue à celle décrite à l'exemple 1, mais à partir de 3,0 g d'(amino-2 N-phényl-acétamido)-2 N-(indanyl-5) acétamide et de 1,2 g d'isocyanate de méthyl-3 phényle. On obtient après recristallisation dans l'acétonitrile, 0,8 g de N-(indanyl-5) {[(méthyl-3 phényl)-3 uréido]-2 N-phényl-acétamido}-2 acétamide fondant à 210°C.

L'(amino-2 N-phényl-acétamido)-2 N-(indanyl-5) acétamide peut être préparé d'une manière analogue à celle décrite à l'exemple 1, pour la préparation de l'(amino-2 N-phényl-acétamido)-2 N-phényl-acétamide, mais à partir de 4,0 g de N-(indanyl-5) (N-phényl phtalimido-2 acétamido)-2 acétamide et de 0,9 g d'hydrate d'hydrazine. On obtient ainsi, 3,0 g d'(amino-2 N-phényl-acétamido)-2 N-(indanyl-5) acétamide sous forme d'une huile utilisée telle quelle dans les synthèses ultérieures.

Le N-(indanyl-5) (N-phényl phtalimido-2 acétamido)-2 acétamide, peut être préparé d'une manière analogue à celle décrite à l'exemple 1, pour la préparation du (N-phényl phtalimido-2 acétamido)-2 N-phényl-acétamide, mais à partir de 4,0 g d'acide (N-phényl phtalimido-2 acétamido)-2 acétique, de 1,7 g de dichlorure d'oxalyle et de 3,1 g d'amino-5 indane. On obtient, après recristallisation dans un mélange acétate d'éthyle - oxyde de diisopropyle (50-50 en volumes), 4,0 g de N-(indanyl-5) (N-phényl phtalimido-2 acétamido)-2 acétamide fondant à 216°C.

### EXEMPLE 19

On opère d'une manière analogue à celle décrite à l'exemple 1, mais à partir de 1,8g d'(amino-2 N-phényl acétamido)-2 N-(méthyl-2 phényl) acétamide et de 0,82 g d'isocyanate de méthyl-3 phényle. On obtient après recristallisation dans l'acétonitrile, 1,2 g de N-(méthyl-2 phényl) {[(méthyl-3 phényl)-3 uréido]-2 N-phényl acétamido}-2 acétamide fondant à 200°C.

L'(amino-2 N-phényl-acétamido)-2 N-(méthyl-2 phényl) acétamide peut être préparé d'une manière analogue à celle décrite à l'exemple 1, pour la préparation de l'(amino-2 N-phényl-acétamido)-2 N-phényl-acétamide, mais à partir de 6,8 g de N-(méthyl-2 phényl) (N-phényl phtalimido-2 acétamido)-2 acétamide et de 1,55 g d'hydrate d'hydrazine. On obtient ainsi, 1,8 g d'(amino-2 N-phényl acétamido)-2 N-(méthyl-2 phényl) acétamide sous forme d'une huile utilisée telle quelle dans les synthèses ultérieures.

Le N-(méthyl-2 phényl) (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 6,8 g d'acide (N-phényl phtalimido-2 acétamido)-2 acétique, de 2,7 g de dichlorure d'oxalyle et de 5,1 g de méthyl-2 aniline. On obtient ainsi, 6,8 g de N-(méthyl-2 phényl) (N-phényl phtalimido-2 acétamido)-2 acétamide fondant à 160°C.

### EXEMPLE 20

On opère d'une manière analogue à celle décrite à l'exemple 1, mais à partir de 1,4g de N-{[(amino-2 acétyl) phénylamino]-2 acétyl} N-phényl glycinate de tert-butyle et de 0,45 g d'isocyanate de méthyl-3 phényle. Le produit obtenu est purifié par chromatographie sur 80 g de silice (0,04-0,063 mm) contenus dans une colonne de 3 cm de diamètre [éluant : chlorure de méthylène-méthanol (98-2 en volumes)] en utilisant une surpression de 30kPa d'azote et en recueillant des fractions de 35 cm3. Les fractions 19 à 21 sont réunies et concentrées à sec sous pression réduite (2,7kPa) à 40°C. Après battage du résidu obtenu avec 25 cm3 d'heptane, on obtient 0,4g de N-{{[(méthyl-3 phényl)-3 uréido]-2 N-phényl acétamido}-2 acétyl} N-phényl-glycinate de tert-butyle fondant à 76-80°C.

Le N-{[(amino-2 acétyl) phénylamino]-2 acétyl} N-phényl glycinate de tert-butyle peut être préparé d'une manière analogue à celle décrite à l'exemple 1, pour la préparation de l'(amino-2 N-phényl-acétamido)-2 N-phényl-acétamide, mais à partir de 2,3g de N-{[(phtalimido-2 acétyl) phénylamino]-2 acétyl} N-phényl glycinate de tert-butyle et de 0,4g d'hydrate d'hydrazine. On obtient ainsi, 1,4g de N-{[(amino-2 acétyl) phénylamino]-2 acétyl} N-phényl glycinate de tert-butyle sous forme d'une huile épaisse utilisée telle quelle dans les synthèses ultérieures.

Le N-{[(phtalimido-2 acétyl) phénylamino]-2 acétyl} N-phényl glycinate de tert-butyle peut être préparé d'une manière analogue à celle décrite à l'exemple 1, pour la préparation du (N-phényl phtalimido-2 acétamido)-2 N-phényl-acétamide, mais à partir de 5,1g d'acide (N-phényl phtalimido-2 acétamido)-2 acétique, de 2,1g de dichlorure d'oxalyle et de 3,1g de N-phényl glycinate de tert-butyle. On obtient ainsi, 2,3g de N-{[(phtalimido-2 acétyl) phénylamino]-2 acétyl} N-phényl glycinate de tert-butyle fondant à 80°C.

### EXEMPLE 21

On opère d'une manière analogue à celle décrite à l'exemple 1, mais à partir de 1,5 g d'amino-2 N-[(diméthyl-3,3 pipéridino)-1 oxo-1 propyl-2] N-phényl-acétamide-(RS) et de 0,63 g d'isocyanate de méthyl-3 phényle. Le produit obtenu est purifié par chromatographie sur 40 g de silice (0,065-0,200 mm) contenus dans une colonne de 2,2 cm de diamètre [éluant : chlorure de méthylène-acétate d'éthyle (50-50 en volumes)] en recueillant des fractions de 25 cm3. Les fractions 14 à 24 sont réunies et concentrées à sec sous pression réduite (2,7 kPa) à 40 °C. On obtient, après recristallisation dans l'oxyde de diéthyle 1,6 g de N-[(diméthyl-3,3 pipéridino)-1 oxo-1 propyl-2] [(méthyl-3 phényl)-3 uréido]-2 N-phényl-acétamide-(RS) fondant à 149°C.

L'amino-2 N-[(diméthyl-3,3 pipéridino)-1 oxo-1 propyl-2] N-phényl-acétamide-(RS) peut être préparé d'une manière analogue à celle décrite à l'exemple 1, pour la préparation de l'(amino-2 N-phényl-acétamido)-2 N-phényl-acétamide, mais à partir de 10,8 g de N-[(diméthyl-3,3 pipéridino)-1 oxo-1 propyl-2] N-phényl phtalimido-2 acétamide-(RS) et de 2,4 g d'hydrate d'hydrazine. On obtient ainsi, 6,6 g d'amino-2 N-[(diméthyl-3,3 pipéridino)-1 oxo-1 propyl-2] N-phényl-acétamide-(RS) sous forme d'une huile utilisée telle quelle dans les synthèses ultérieures.

Le N-[(diméthyl-3,3 pipéridino)-1 oxo-1 propyl-2] N-phényl phtalimido-2 acétamide peut être préparé d'une manière analogue à celle décrite à l'exemple 1, pour la préparation du (N-phényl phtalimido-2 acétamido)-2 acétate de tert-butyle mais à partir de 6,4 g de diméthyl-3,3 (phénylamino-2 propionyl)-1 pipéridine-(RS) et de 5,5 g de chlorure de phtalimido-2 acétyle. On obtient ainsi 10,9 g de N-[(diméthyl-3,3 pipéridino)-1 oxo-1 propyl-2] N-phényl phtalimido-2 acétamide-(RS) fondant à 171°C.

La diméthyl-3,3 (phénylamino-2 propionyl)-1 pipéridine-(RS) peut être préparée de la manière suivante : à une solution de 8,5 g de chlorure de bromo-2 propionyle dans 90 cm3 d'acétonitrile on ajoute en une heure une solution de 5,7 g de diméthyl-3,3 pipéridine et de 6,0 g de triéthylamine dans 15 cm3 d'acétonitrile. Le mélange réactionnel est agité à une température voisine de 25°C pendant 18 heures, puis on ajoute 7 g d'aniline et 6 g de triéthylamine. L'acétonitrile est distillé et le mélange réactionnel est agité à une température voisine de 100°C pendant 5 heures. Après refroidissement, on ajoute 200 cm3 d'acétate d'éthyle et 200 cm3 d'eau distillée. La phase organique est extraite par 2 fois 100 cm3 d'une solution aqueuse d'acide chlorhydrique 1N. La phase aqueuse obtenue est alcalinisée à pH 10 avec une solution aqueuse de soude 4N et extraite par 2 fois 100 cm3 d'oxyde de diéthyle. Les phases organiques sont réunies, lavées par 100 cm3 d'eau distillée, séchées sur sulfate de magnésium et concentrées à sec sous pression réduite (2,7 kPa) à 30°C. On obtient, après recristallisation dans l'oxyde de diisopropyle, 6,5 g de diméthyl-3,3 (phénylamino-2 propionyl)-1 pipéridine-(RS) fondant à 98°C.

### EXEMPLE 22

On opère d'une manière analogue à celle décrite à l'exemple 1, mais à partir de 1,0 g d'(amino-2 N-phényl-acétamido)-2 N-méthyl N-phényl-propionamide-(RS) et de 0,42 g d'isocyanate de méthyl-3 phényle. On obtient après recristallisation dans un mélange d'acétonitrile et de diméthylformamide ( 93-7 en volumes ) 0,5 g de N-méthyl {[(méthyl-3 phényl)-3 uréido]-2 N-phényl-acétamido}-2 N-phényl-propionamide-(RS) fondant à 214°c.

L'(amino-2 N-phényl-acétamido)-2 N-méthyl N-phénylpropionamide-(RS) peut être préparé d'une manière analogue à celle décrite à l'exemple 1, pour la préparation de l'(amino-2 N-phényl-acétamido)-2 N-phényl-acétamide, mais à partir de 15,4 g de N-méthyl N-phényl (N-phényl phtalimido-2 acétamido)-2 propionamide-(RS) et de 3,4 g d'hydrate d'hydrazine. On obtient ainsi, 9,6 g d'(amino-2 N-phényl-acétamido)-2 N-méthyl N-phényl-propionamide-(RS) sous forme d'une huile utilisée telle quelle dans les synthèses ultérieures.

Le N-méthyl N-phényl (N-phényl phtalimido-2 acétamido)-2 propionamide-(RS) peut être préparé d'une manière analogue à celle décrite à l'exemple 1, pour la préparation du (N-phényl phtalimido-2 acétamido)-2 acétate de tert-butyle mais à partir de 9,5 g d'anilino-2 N-méthyl N-phényl-propionamide-(RS) et de 8,3 g de chlorure de phtalimido-2 acétyle. On obtient ainsi, après recristallisation dans l'oxyde de diéthyle 15,5 g de N-méthyl N-phényl (N-phényl phtalimido-2 acétamido)-2 propionamide-(RS) fondant à 120°C.

L'anilino-2 N-méthyl N-phényl-propionamide-(RS) peut être préparé d'une manière analogue à celle décrite à l'exemple 21, pour la préparation de la diméthyl-3,3 (phénylamino-2 propionyl)-1 pipéridine-(RS), mais à partir de 20 g de chlorure de bromo-2 propionyle, de 20 g de triéthylamine, de 12,5 g de N-méthyl aniline et de 32,6 g d'aniline. Le produit obtenu est purifié par chromatographie sur 700 g de gel de silice (0,065-0,200 mm) contenus dans une colonne de 6 cm de diamètre [éluant : acétate d'éthyle -cyclohexane (30-70 en volumes)] en recueillant des fractions de 200 cm3. Les fractions 9 à 13 sont réunies et concentrées à sec sous pression réduite (2,7 kPa) à 40°C. On obtient, après recristallisation dans le cyclohexane 9,6 g d'anilino-2 N-méthyl N-phényl-propionamide-(RS) fondant à 90°C.

### EXEMPLE 23

A une suspension de 1,6 g de N-(méthyl-3 phényl) (N-phényl phtalimido-2 acétamido)-2 acétamide dans 10 cm3 de méthanol, on ajoute 0,37 g d'hydrate d'hydrazine. Le mélange réactionnel est agité à reflux pendant 40 minutes, puis concentré à sec sous pression réduite (2,7 kPa) à 40°C. Le résidu est agité 30 minutes dans 15 cm3 de tétrahydrofuranne anhydre et le produit insoluble est séparé par filtration. On ajoute alors 0,5 g d'isocyanate de méthyl-3 phényle au filtrat et le mélange est agité 30 minutes à une température voisine de 25°C, puis concentré à sec sous pression réduite (2,7 kPa) à 40°C. Le résidu obtenu est purifié par chromatographie sur 150 g de silice (0,065-0,200 mm) contenus dans une colonne de 3,5 cm de diamètre [éluant : chlorure de méthylène-acétate d'éthyle (80-20 en volumes)] en recueillant des fractions de 25 cm3. Les fractions 21 à 27 sont réunies et concentrées à sec sous pression réduite (2,7 kPa) à 40°C. On obtient ainsi, après recristallisation dans l'acétonitrile, 0,27 g de N-(méthyl-3 phényl) {[(méthyl-3 phényl)-3 uréido]-2 N-phényl-acétamido}-2 acétamide fondant à 187°C.

Le N-(méthyl-3 phényl) (N-phényl phtalimido-2 acétamido)-2 acétamide peut être préparé de la manière suivante : à une suspension de 2,6 g de N-[(imidazolyl-1)-2 oxo-2 éthyl] N-phénylphtalimido-2 acétamide dans 25 cm3 de toluène, on ajoute 0,75 g de méthyl-3 aniline. Le mélange réactionnel est agité à reflux pendant 6 heures, puis concentré à sec sous pression réduite (2,7 kPa) à 40°C. Le résidu est dissous dans 30 cm3 de chlorure de méthylène et la solution obtenue est lavée successivement par 20 cm3 d'une solution aqueuse 1N d'acide chlorhydrique et par 2 fois 15 cm3 d'eau distillée, puis séchée sur sulfate de magnésium et concentrée à sec sous pression réduite (2,7 kPa) à 40°C. On obtient ainsi 1,6 g de N-(méthyl-3 phényl) (N-phényl phtalimido-2 acétamido)-2 acétamide sous forme d'une meringue utilisée telle quelle dans les synthèses ultérieures.

Le N-[(imidazolyl-1)-2 oxo-2 éthyl] N-phényl phtalimido-2 acétamide peut être préparé de la manière suivante : à une suspension de 5 g d'acide (N-phényl phtalimido-2 acétamido)-2 acétique dans 30 cm3 de tétrahydrofuranne anhydre, on ajoute 2,4 g de N,N'-diimidazole carbonyle. Le milieu réactionnel est agité 1 heure à une température voisine de 25°C puis le produit insoluble obtenu est séparé par filtration et lavé par 2 fois 5 cm3 de tétrahydrofuranne et séché à l'air. On obtient ainsi 5,3 g de N-[(imidazolyl-1)-2 oxo-2 éthyl] N-phényl phtalimido-2 acétamide fondant à 206°C.

### EXEMPLE 24

On opère d'une manière analogue à celle décrite à l'exemple 23, mais à partir de 3,1 g de N-[oxo-2 (tétrahydro-1,2,3,4 quinolyl-1)-2 éthyl] N-phényl phtalimido-2 acétamide, de 0,69 g d'hydrate d'hydrazine et de 0,91 g d'isocyanate de méthyl-3 phényle. Le produit obtenu est purifié par chromatographie sur 25 g de silice (0,065-0,200 mm) contenus dans une colonne de 1,7 cm de diamètre [éluant : chlorure de méthylène-acétate d'éthyle (80-20 en volumes)] en recueillant des fractions de 25 cm3. Les fractions 12 à 23 sont réunies et concentrées à sec sous pression réduite (2,7 kPa) à 40 °C. On obtient, après recristallisation dans l'acétate d'éthyle, 0,9 g de [(méthyl-3 phényl)-3 uréido]-2 N-[oxo-2 (tétrahydro-1,2,3,4 quinolyl-1)-2 éthyl] N-phényl-acétamide fondant à 167°C.

Le N-[oxo-2 (tétrahydro-1,2,3,4 quinolyl-1)-2 éthyl] N-phényl phtalimido-2 acétamide, peut être préparé d'une manière analogue à celle décrite à l'exemple 1, pour la préparation du (N-phényl phtalimido-2 acétamido)-2 N-phényl-acétamide, mais à partir de 2,5 g d'acide (N-phényl phtalimido-2 acétamido)-2 acétique, de 0,95 g de dichlorure d'oxalyle et de 1,0 g de tétrahydro-1,2,3,4 quinoléine. On obtient ainsi 3,2 g de N-[oxo-2 (tétrahydro-1,2,3,4 quinolyl-1)-2 éthyl] N-phényl phtalimido-2 acétamide sous forme d'une meringue utilisée telle quelle dans les synthèses ultérieures.

### EXEMPLE 25

On opère d'une manière analogue à celle décrite à l'exemple 23, mais à partir de 1,6 g de N-méthyl N-(méthyl-4 phényl) (N-phényl phtalimido-2 acétamido)-2 acétamide, de 0,36 g d'hydrate d'hydrazine et de 0,3 g d'isocyanate de méthyl-3 phényle. Le produit obtenu est purifié par chromatographie sur 80 g de gel de silice (0,065-0,200 mm) contenus dans une colonne de 2,5 cm de diamètre [éluant : chlorure de méthylène] en recueillant des fractions de 20 cm3. Les fractions 10 à 12 sont réunies et concentrées à sec sous pression réduite (2,7 kPa) à 40°C. On obtient, après recristallisation dans un mélange d'acétate d'éthyle et d'éther diéthylique (40-60 en volumes), 0,7 g de N-méthyl N-(méthyl-4 phényl) {[(méthyl-3 phényl)-3 uréido]-2 N-phényl-acétamido}-2 acétamide fondant à 162°C.

Le N-méthyl N-(méthyl-4 phényl) (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 2,0 g d'acide (N-phényl phtalimido-2 acétamido)-2 acétique, de 0,82 g de dichlorure d'oxalyle et de 1,55 g de N-méthyl méthyl-4 aniline. On obtient, après recristallisation dans le cyclohexane, 9,6 g de N-méthyl N-(méthyl-4 phényl) (N-phényl phtalimido-2 acétamido)-2 acétamide fondant à 90°C.

La N-méthyl méthyl-4 aniline peut être préparée selon la méthode décrite par A. M. HJORT, E. J. DeBEER, J. S. BUCK et W. S. IDE, J. Pharmacol. Exp. Therap., 55, 152 (1935).

### EXEMPLE 26

On opère d'une manière analogue à celle décrite à l'exemple 23, mais à partir de 2,7 g de N-méthyl N-(méthyl-3 phényl) (N-phényl phtalimido-2 acétamido)-2 acétamide, de 0,6 g d'hydrate d'hydrazine et de 0,8 g d'isocyanate de méthyl-3 phényle. Le produit obtenu est purifié par chromatographie sur 25 g de gel de silice (0,065-0,200 mm) contenus dans une colonne de 1,7 cm de diamètre [éluant : chlorure de méthylène-acétate d'éthyle (80-20 en volumes)], en recueillant des fractions de 25 cm3. Les fractions 13 à 27 sont réunies et concentrées à sec sous pression réduite (2,7 kPa) à 40°C. On obtient, après recristallisation dans l'éther diéthylique, 0,86 g de N-méthyl N-(méthyl-3 phényl) {[(méthyl-3 phényl)-3 uréido]-2 N-phényl-acétamido}-2 acétamide fondant à 165°C.

Le N-méthyl N-(méthyl-3 phényl) (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide , mais à partir de 2,0 g d'acide (N-phényl phtalimido-2 acétamido)-2 acétique, de 0,76 g de dichlorure d'oxalyle, de 0,74 g de N-méthyl méthyl-3 aniline et de 0,6 g de triéthylamine. On obtient ainsi 2,7 g de N-méthyl N-(méthyl-3 phényl) (N-phényl phtalimido-2 acétamido)-2 acétamide sous forme d'une huile utilisée telle quelle dans les synthèses ultérieures.

La N-méthyl méthyl-3 aniline peut être préparée selon la méthode décrite par J. A. MARTUS, Hormone, 10, 81 (1937); [C.A., 32 510 (1938)].

### EXEMPLE 27

On opère d'une manière analogue à celle décrite à l'exemple 23, mais à partir de 1,2 g de N-(méthoxy-4 phényl) (N-phényl phtalimido-2 acétamido)-2 acétamide, de 0,28 g d'hydrate d'hydrazine et de 0,38 g d'isocyanate de méthyl-3 phényle. Le produit obtenu est purifié par chromatographie sur 25 g de silice (0,065-0,200 mm) contenus dans une colonne de 1,7 cm de diamètre [éluant : chlorure de méthylène-acétate d'éthyle (80-20 en volumes)] en recueillant des fractions de 25 cm3. Les fractions 2 à 7 sont réunies et concentrées à sec sous pression réduite (2,7 kPa) à 40 °C. On obtient, après recristallisation dans l'acétonitrile, 0,12 g de N-(méthoxy-4 phényl) {[(méthyl-3 phényl)-3 uréido]-2 N-phényl-acétamido}-2 acétamide fondant à 194°C.

Le N-(méthoxy-4 phényl) (N-phényl phtalimido-2 acétamido)-2 acétamide peut être préparé d'une manière analogue à celle décrite à l'exemple 23, pour la préparation du N-(méthyl-3 phényl) (N-phényl phtalimido-2 acétamido)-2 acétamide, mais à partir de 2,6 g de N-[oxo-2 (imidazolyl-1)-2 éthyl] N-phényl phtalimido-2 acétamide et de 0,84 g de méthoxy-4 aniline. On obtient ainsi 1,25 g de N-(méthoxy-4 phényl)(N-phényl phtalimido-2 acétamido)-2 acétamide sous forme d'une meringue utilisée telle quelle dans les synthèses ultérieures.

### EXEMPLE 28

On opère d'une manière analogue à celle décrite à l'exemple 23, mais à partir de 3,1 g de N-éthyl N-phényl (N-phényl phtalimido-2 acétamido)-2 acétamide, de 0,4 g d'hydrate d'hydrazine et de 0,54 g d'isocyanate de méthyl-3 phényle. Le produit obtenu est purifié par chromatographie sur 20 g de silice (0,065-0,200 mm) contenus dans une colonne de 1,7 cm de diamètre [éluant : chlorure de méthylène] en recueillant des fractions de 20 cm3. Les fractions 10 à 23 sont réunies et concentrées à sec sous pression réduite (2,7 kPa) à 40 °C. On obtient, après recristallisation dans l'éther diéthylique, 0,55 g de N-éthyl N-phényl {[(méthyl-3 phényl)-3 uréido]-2 N-phényl-acétamido}-2 acétamide fondant à 158°C.

Le N-éthyl N-phényl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 2,0 g d'acide (N-phényl phtalimido-2 acétamido)-2 acétique, de 0,76 g de dichlorure d'oxalyle et de 1,5 g de N-éthyl aniline. On obtient, après recristallisation dans l'oxyde de diéthyle 1,8 g de N-éthyl N-phényl (N-phényl phtalimido-2 acétamido)-2 acétamide fondant à 105°C.

### EXEMPLE 29

A une solution de 18,0 g d'{[(imidazolyl-1) carboxamido]-2 N-phényl-acétamido}-2 N-méthyl N-phényl-acétamide dans 300 cm3 de toluène, on ajoute 12,6 g d'(hydroxy-1 éthyl)-3 aniline -(RS) et le mélange est agité à reflux pendant 4 heures. Après refroidissement on ajoute 300 cm3 d'acétate d'éthyle, puis la solution obtenue est lavée successivement par 300 cm3 d'eau distillée, par 2 fois 300 cm3 d'une solution aqueuse 1N d'acide chlorhydrique, par 2 fois 300 cm3 d'une solution aqueuse saturée d'hydrogénocarbonate de sodium et par 300 cm3 d'une solution aqueuse saturée de chlorure de sodium. La phase organique est séchée sur sulfate de magnésium puis concentrée à sec sous pression réduite (2,7 kPa) à 40°C. On obtient, après recristallisation dans l'acétate d'éthyle, 13,6 g d'{{[(hydroxy-1 éthyl)-3 phényl]-3 uréido}-2 N-phényl-acétamido}-2 N-méthyl N-phényl-acétamide-(RS) fondant à 168°C.

L' {[(imidazolyl-1) carboxamido]-2 N-phényl-acétamido}-2 N-méthyl N-phényl-acétamide peut être préparé de la manière suivante : à une solution de 31 g de N,N'-diimidazole carbonyle dans 300 cm3 de tétrahydrofuranne anhydre, on ajoute une solution de 37 g d '(amino-2 N-phényl-acétamido)-2 N-méthyl N-phényl-acétamide dans 150 cm3 de tétrahydrofuranne anhydre. La solution est agitée pendant 3 heures à une température voisine de 25°C, puis concentrée à sec sous pression réduite (2,7 kPa) à 40°C. Le résidu est dissous dans 500 cm3 d'acétate d'éthyle et la solution obtenue est lavée successivement par 4 fois 300 cm3 d'eau distillée et par 300 cm3 d'une solution aqueuse saturée de chlorure de sodium. La phase organique est séchée sur sulfate de magnésium puis concentrée à sec sous pression réduite (2,7 kPa) à 40°C. On obtient après recristallisation dans l'acétate d'éthyle 33,3 g d' {[(imidazolyl-1) carboxamido]-2 N-phényl-acétamido}-2 N-méthyl N-phényl-acétamide fondant à 120°C.

### EXEMPLE 30

On opère d'une manière analogue à celle décrite à l'exemple 29, mais à partir de 0,9 g d'{[(imidazolyl-1) carboxamido]-2 N-phényl-acétamido}-2 N-méthyl N-phényl-acétamide et de 0,64 g de méthylthio-3 aniline. Le produit obtenu est purifié par chromatographie sur 100 g de silice (0,065-0,200 mm) contenus dans une colonne de 2 cm de diamètre [éluant : chlorure de méthylène-méthanol (95-5 en volumes)] en recueillant des fractions de 20 cm3. Les fractions 9 à 12 sont réunies et concentrées à sec sous pression réduite (2,7 kPa) à 40°C. On obtient, après recristallisation dans un mélange d'acétate d'éthyle et d'éther diéthylique (85-15 en volumes), 0,22 g de N-méthyl {[(méthylthio-3 phényl)-3 uréido]-2 N-phényl-acétamido}-2 N-phényl-acétamide fondant à 172°C.

### EXEMPLE 31

On opère d'une manière analogue à celle décrite à l'exemple 29, mais à partir de 3,25 g d'[(imidazolyl-1) carboxamido]-2 N-[oxo-2 (tétrahydro-1,2,3,4 quinolyl-1)-2 éthyl] N-phényl-acétamide et de 2,13 g d'(hydroxy-1 éthyl)-3 aniline -(RS). Le produit obtenu est purifié par chromatographie sur 60 g de silice (0,065-0,200 mm) contenus dans une colonne de 2,0 cm de diamètre [éluant : acétate d'éthyle] en recueillant des fractions de 25 cm3. Les fractions 8 à 15 sont réunies et concentrées à sec sous pression réduite (2,7 kPa) à 40 °C. On obtient, après recristallisation dans un mélange d'oxyde de diisopropyle et d'acétate d'éthyle (90-10 en volumes), 1,7 g d'{[(hydroxy-1 éthyl)-3 phényl]-3 uréido}-2 N-[oxo-2 (tétrahydro-1,2,3,4 quinolyl-1)-2 éthyl] N-phényl-acétamide-(RS) fondant à 120°C.

L'[(imidazolyl-1) carboxamido]-2 N-[oxo-2 (tétrahydro-1,2,3,4 quinolyl-1)-2 éthyl] N-phényl-acétamide peut être préparé d'une manière analogue à celle décrite dans l'exemple 29, pour la préparation de l'{[(imidazolyl-1) carboxamido]-2 N-phényl-acétamido}-2 N-méthyl N-phényl-acétamide, mais à partir de 6,7 g d'amino-2 N-[oxo-2 (tétrahydro-1,2,3,4 quinolyl-1)-2 éthyl] N-phényl-acétamide et de 3,36 g de N,N'-diimidazole carbonyle. Le produit obtenu est purifié par chromatographie sur 150 g de silice (0,065-0,200 mm) contenus dans une colonne de 2,7 cm de diamètre [éluant : acétate d'éthyle -dichlorométhane (80-20 en volumes)] en recueillant des fractions de 30 cm3. Les fractions 6 à 13 sont réunies et concentrées à sec sous pression réduite (2,7 kPa) à 40 °C. On obtient ainsi, 6,5 g d'[(imidazolyl-1) carboxamido]-2 N-[oxo-2 (tétrahydro-1,2,3,4 quinolyl-1)-2 éthyl] N-phényl-acétamide sous forme d'une meringue utilisée telle quelle dans les synthèses ultérieures.

L'amino-2 N-[oxo-2 (tétrahydro-1,2,3,4 quinolyl-1)-2 éthyl] N-phényl-acétamide peut être préparé d'une manière analogue à celle décrite à l'exemple 2, pour la préparation de l'(amino-2 N-phényl-acétamido)-2 N-méthyl N-phényl-acétamide, mais à partir de 4,5 g de N-[oxo-2 (tétrahydro-1,2,3,4 quinolyl-1)-2 éthyl] N-phényl phtalimido-2 acétamide et de 1,0 g d'hydrate d'hydrazine. On obtient ainsi 2,2 g d'amino-2 N-[oxo-2 (tétrahydro-1,2,3,4 quinolyl-1)-2 éthyl] N-phényl acétamide sous forme d'une huile utilisée telle quelle dans les synthèses ultérieures.

### EXEMPLE 32

On opère d'une manière analogue à celle décrite à l'exemple 29, mais à partir de 3,25 g d'[(imidazolyl-1) carboxamido]-2 N-[oxo-2 (tétrahydro-1,2,3,4 quinolyl-1)-2 éthyl] N-phényl-acétamide et de 2,16 g de méthylthio-3 aniline. Le produit obtenu est purifié par chromatographie sur 60 g de silice (0,065-0,200 mm) contenus dans une colonne de 1,7 cm de diamètre [éluant : acétate d'éthyle -cyclohexane (75-25 en volumes)] en recueillant des fractions de 25 cm3. Les fractions 11 à 18 sont réunies et concentrées à sec sous pression réduite (2,7 kPa) à 40°C. On obtient, après recristallisation dans l'oxyde de diisopropyle, 1,3 g [(méthylthio-3 phényl)-3 uréido]-2 N-[oxo-2 (tétrahydro-1,2,3,4 quinolyl-1)-2 éthyl] N-phényl-acétamide fondant à 110°C.

### EXEMPLE 33

On opère d'une manière analogue à celle décrite à l'exemple 29, mais à partir de 2,0 g de N-[(diméthyl-3,3 pipéridino)-2 oxo-2 éthyl] [(imidazolyl-1) carboxamido]-2 N-phényl-acétamide et de 1,38 g d'(hydroxy-1 éthyl)-3 aniline -(RS). On obtient après recristallisation dans un mélange d'éthanol et d'éther diisopropylique (50-50 en volumes), 1,4 g de N-[(diméthyl-3,3 pipéridino)-2 oxo-2 éthyl] {[(hydroxy-1 éthyl)-3 phényl]-3 uréido}-2 N-phényl-acétamide-(RS) fondant à 200°C.

Le N-[(diméthyl-3,3 pipéridino)-2 oxo-2 éthyl] [(imidazolyl-1) carboxamido]-2 N-phényl-acétamide peut être préparé d'une manière analogue à celle décrite dans l'exemple 29, pour la préparation de l'{[(imidazolyl-1) carboxamido]-2 N-phényl-acétamido}-2 N-méthyl N-phényl-acétamide, mais à partir de 6,8 g d'amino-2 N-[oxo-2 (diméthyl-3,3 pipéridino)-2 éthyl] N-phényl-acétamide et de 3,7 g de N,N'-diimidazole carbonyle. On obtient ainsi, après recristallisation dans l'éther diisopropylique, 6,4 g de N-[(diméthyl-3,3 pipéridino)-2 oxo-2 éthyl] [(imidazolyl-1) carboxamido]-2 N-phényl-acétamide fondant 104°C.

### EXEMPLE 34

On opère d'une manière analogue à celle décrite à l'exemple 29, mais à partir de 2,0 g de N-[(diméthyl-3,3 pipéridino)-2 oxo-2 éthyl] [(imidazolyl-1) carboxamido]-2 N-phényl-acétamide et de 1,25 g de méthoxy-3 aniline. On obtient après recristallisation dans un mélange d'acétonitrile et d'éther diisopropylique (50-50 en volumes), 0,96 g de N-[(diméthyl-3,3 pipéridino)-2 oxo-2 éthyl] [(méthoxy-3 phényl)-3 uréido]-2 N-phényl-acétamide fondant à 200°C.

### EXEMPLE 35

On opère d'une manière analogue à celle décrite à l'exemple 29, mais à partir de 2,0 g de N-[(diméthyl-3,3 pipéridino)-2 oxo-2 éthyl] [(imidazolyl-1) carboxamido]-2 N-phényl-acétamide et de 1,4 g de méthylthio-3 aniline. On obtient, après recristallisation dans un mélange acétate d'éthyle -oxyde de diisopropyle (50-50 en volumes), 1,1 g de N-[(diméthyl-3,3 pipéridino)-2 oxo-2 éthyl] [(méthylthio-3 phényl)-3 uréido]-2 N-phényl-acétamide fondant à 172°C.

### EXEMPLE 36

On opère d'une manière analogue à celle décrite à l'exemple 29, mais à partir de 1,8 g d'{[(imidazolyl-1) carboxamido]-2 N-phényl-acétamido}-2 N-méthyl N-phényl-propionamide-(RS) et de 1,24 g d'(hydroxy-1 éthyl)-3 aniline -(RS). Le produit obtenu est purifié par chromatographie sur 30 g de silice (0,065-0,200 mm) contenus dans une colonne de 2,2 cm de diamètre [éluant : acétate d'éthyle -cyclohexane (80-20 en volumes)] en recueillant des fractions de 25 cm3. Les fractions 7 à 17 sont réunies et concentrées à sec sous pression réduite (2,7 kPa) à 40°C. On obtient, après recristallisation dans l'acétate d'éthyle, 0,9 g de {{[(hydroxy-1 éthyl)-3 phényl-(RS)]-3 uréido}-2 N-phényl-acétamido}-2 N-méthyl N-phényl-propionamide-(RS) fondant à 185°C.

L'{[(imidazolyl-1) carboxamido]-2 N-phényl-acétamido}-2 N-méthyl N-phényl-propionamide-(RS) peut être préparé d'une manière analogue à celle décrite dans l'exemple 29 pour la préparation de l'{[(imidazolyl-1) carboxamido]-2 N-phényl-acétamido}-2 N-méthyl N-phényl-acétamide mais à partir de 5,1 g d'(amino-2 N-phényl-acétamido)-2 N-méthyl N-phényl-propionamide-(RS) et de 3,1 g de N,N'-diimidazole carbonyle. On obtient ainsi 5,0 g d'{[(imidazolyl-1) carboxamido]-2 N-phényl-acétamido}-2 N-méthyl N-phényl-propionamide-(RS) sous forme d'une meringue utilisée telle quelle dans les synthèses ultérieures.

### EXEMPLE 37

On opère d'une manière analogue à celle décrite à l'exemple 29, mais à partir de 3,2 g d'{[(imidazolyl-1) carboxamido]-2 N-phényl-acétamido}-2 N-méthyl N-phényl-propionamide-(RS) et de 2,2 g de méthylthio-3 aniline. Le produit obtenu est purifié par chromatographie sur 100 g de gel de silice (0,065-0,200 mm) contenus dans une colonne de 3,3 cm de diamètre [éluant acétate d'éthyle - chlorure de méthylène (50-50 en volumes)] en recueillant des fractions de 50 cm3. Les fractions 22 à 32 sont réunies et concentrées à sec sous pression réduite (2,7 kPa) à 40°C. On obtient, après recristallisation dans l'acétonitrile 1,5 g de N-méthyl {[(méthylthio-3 phényl)-3 uréido]-2 N-phényl-acétamido}-2 N-phényl-propionamide-(RS) fondant à 175°C.

### EXEMPLE 38

On opère d'une manière analogue à celle décrite à l'exemple 29, mais à partir de 2,0 g de N-[(diméthyl-3,3 pipéridino)-1 oxo-1 propyl-2] [(imidazolyl-1) carboxamido]-2 N-phényl-acétamide-(RS) et de 1,38 g d'(hydroxy-1 éthyl)-3 aniline-(RS). Le produit obtenu est purifié par chromatographie sur 40 g de silice (0,065-0,200 mm) contenus dans une colonne de 2,4 cm de diamètre [éluant : chlorure de méthylène-acétate d'éthyle (40-60 en volumes)] en recueillant des fractions de 25 cm3. Les fractions 6 à 13 sont réunies et concentrées à sec sous pression réduite (2,7 kPa) à 40°C. On obtient, après recristallisation dans l'acétate d'éthyle, 0,6 g de N-[(diméthyl-3,3 pipéridino)-1 oxo-1 propyl-2-(RS)] {[(hydroxy-1 éthyl)-3 phényl-(RS)]-3 uréido}-2 N-phényl-acétamide fondant à 186°C.

Le N-[(diméthyl-3,3 pipéridino)-1 oxo-1 propyl-2] [(imidazolyl-1) carboxamido]-2 N-phényl-acétamide-(RS) peut être préparé d'une manière analogue à celle décrite dans l'exemple 29, pour la préparation de l'{[(imidazolyl-1) carboxamido]-2 N-phényl-acétamido}-2 N-méthyl N-phényl-acétamide mais à partir de 5,1 g d'amino-2 N-[(diméthyl-3,3 pipéridino)-1 oxo-1 propyl-2] N-phényl-acétamide-(RS) et de 3,1 g de N,N'-diimidazole carbonyle. On obtient ainsi 6,0 g de N-[(diméthyl-3,3 pipéridino)-1 oxo-1 propyl-2] [(imidazolyl-1) carboxamido]-2 N-phényl-acétamide-(RS) sous forme d'une meringue utilisée telle quelle dans les synthèses ultérieures.

### EXEMPLE 39

On opère d'une manière analogue à celle décrite à l'exemple 29, mais à partir de 3,6 g de N-[(diméthyl-3,3 pipéridino)-1 oxo-1 propyl-2] [(imidazolyl-1) carboxamido]-2 N-phényl-acétamide-(RS) et de 2,44 g de méthylthio-3 aniline. Le produit obtenu est purifié par chromatographie sur 90 g de silice (0,065-0,200 mm) contenus dans une colonne de 3,3 cm de diamètre [éluant : chlorure de méthylène-acétate d'éthyle (50-50 en volumes)] en recueillant des fractions de 30 cm3. Les fractions 17 à 24 sont réunies et concentrées à sec sous pression réduite (2,7 kPa) à 40°C. On obtient, après recristallisation dans un mélange d'oxyde de diéthyle et d'acétate d'éthyle ( 60-2 en volumes ), 2,1 g de N-[(diméthyl-3,3 pipéridino)-1 oxo-1 propyl-2] [(méthylthio-3 phényl)-3 uréido]-2 N-phényl-acétamide-(RS) fondant à 130°C.

### EXEMPLE 40

Une suspension de 1,6 g d'(α-phénylamino phénylacétyl)-1 pyrrolidine-(RS) et de 1,2 g d'acide [(méthyl-3 phényl)-3 uréido]-2 acétique dans 50 cm3 de dichloro-1,2 éthane anhydre est chauffée à reflux. On ajoute 0,68 g de chlorure de thionyle en maintenant le reflux jusqu'à la fin du dégagement gazeux. Le mélange réactionnel est alors versé dans 30 cm3 d'une solution aqueuse saturée d'hydrogénocarbonate de sodium, puis on ajoute 50 cm3 de chlorure de méthylène. La phase organique est lavée par 50 cm3 d'eau distillée, séchée sur sulfate de magnésium et concentrée à sec sous pression réduite (2,7 kPa) à 40°C. Le produit obtenu est purifié par chromatographie sur 50 g de silice (0,065-0,200 mm) contenus dans une colonne de 2,2 cm de diamètre [éluant : acétate d'éthyle - chlorure de méthylène (50-50 en volumes)] en recueillant des fractions de 25 cm3. Les fractions 9 à 22 sont réunies et concentrées à sec sous pression réduite (2,7 kPa) à 40 °C. On obtient, après recristallisation dans un mélange d'acétate d'éthyle et d'acétonitrile (90-10 en volumes), 0,55 g de [(méthyl-3 phényl)-3 uréido]-2 N-[oxo-2 phényl-1 (pyrrolidinyl-1)-2 éthyl] N-phényl acétamide-(RS) fondant à 138°C.

L'(α-phénylamino phénylacétyl)-1 pyrrolidine-(RS) peut être préparée d'une manière analogue à celle décrite à l'exemple 21, pour la préparation de la diméthyl-3,3 (phénylamino-2 propionyl)-1 pipéridine-(RS), mais à partir de 2,58 g de chlorure d'α-bromo phénylacétyle, de 0,77 g de pyrrolidine, de 1,52 g de triéthylamine et de 3,0 g d'aniline. On obtient, après recristallisation dans l'éther de pétrole, 1,65 g d'(α-phénylamino phénylacétyl)-1 pyrrolidine-(RS) fondant à 65°C.

L'acide [(méthyl-3 phényl)-3 uréido]-2 acétique peut être préparé de la manière suivante : à une solution de 30 g de glycine et de 53 g d'hydrogénocarbonate de sodium dans 600 cm³ d'eau distillée, on ajoute en 15 minutes 53 g d'isocyanate de méthyl-3 phényle. Le mélange réactionnel est agité 4 heures à une température voisine de 25°C puis lavé par 200 cm3 d'acétate d'éthyle et acidifié à pH 1 avec 200 cm3 d'une solution d'acide chlorhydrique 4N. Le produit obtenu est séparé par filtration, lavé à l'eau et séché à l'air. On obtient ainsi 72 g d'acide [(méthyl-3 phényl)-3 uréido]-2 acétique fondant à 208°C.

### EXEMPLE 41

On opère d'une manière analogue à celle décrite à l'exemple 40, mais à partir de 2,4 g d'anilino-2 N-tert-butyl phénylacétamide-(RS), de 1,2 g d'acide [(méthyl-3 phényl)-3 uréido]-2 acétique et de 1,0 g de chlorure de thionyle. Le produit obtenu est purifié par chromatographie sur 50 g de silice (0,065-0,200 mm) contenus dans une colonne de 1,7 cm de diamètre [éluant : acétate d'éthyle - chlorure de méthylène (20-80 en volumes)] en recueillant des fractions de 30 cm3. Les fractions 5 à 7 sont réunies et concentrées à sec sous pression réduite (2,7 kPa) à 40 °C. On obtient, après recristallisation dans l'acétonitrile, 0,8 g de N-tert-butyl {[(méthyl-3 phényl)-3 uréido]-2 N-phényl acétamido}-2 phénylacétamide-(RS) fondant à 216°C.

L'anilino-2 N-tert-butyl phénylacétamide-(RS) peut être préparé d'une manière analogue à celle décrite à l'exemple 21, pour la préparation de la diméthyl-3,3 (phénylamino-2 propionyl)-1 pipéridine-(RS) mais à partir de 2,6 g de chlorure d'α-bromo phénylacétyle, de 0,77 g de tert-butylamine, de 1,51 g de triéthylamine et de 3,1 g d'aniline. On obtient 2,55 g d'anilino-2 N-tert-butyl phénylacétamide-(RS) sous forme d'une huile utilisée telle quelle dans les synthèses ultérieures.

### EXEMPLE 42

On opère d'une manière analogue à celle décrite à l'exemple 40, mais à partir de 0,62 g d'acide [(méthyl-3 phényl)-3 uréido]-2 acétique, de 0,9 g d'anilino-2 [(pyrrolidinyl-1) carbonyl]-2 acétate de tert-butyle-(RS) et de 0,21 cm3 de chlorure de thionyle. On obtient ainsi, après recristallisation dans 8 cm3 d'un mélange d'oxyde de diéthyle et d'acétate d'éthyle (90-10 en volumes), 0,65 g de {[(méthyl-3 phényl)-3 uréido]-2 N-phényl-acétamido}-2 [(pyrrolidinyl-1)carbonyl]-2 acétate de tert-butyle-(RS) fondant à 148°C.

L'anilino-2 [(pyrrolidinyl-1) carbonyl]-2 acétate de tert-butyle-(RS) peut être préparé de la manière suivante : à une solution de 4,4 g de bromo-2 [(pyrrolidinyl-1) carbonyl]-2 acétate de tert-butyle-(RS) dans 15 cm3 d'acétonitrile, on ajoute 1,8 cm3 d'aniline et le mélange réactionnel est maintenu à reflux pendant 5 heures puis pendant 20 heures à une température voisine de 20°C. Le produit insoluble est séparé par filtration et le filtrat est concentré à sec sous pression réduite (2,7 kPa). Après recristallisation dans 10 cm3 d'oxyde de diisopropyle, on obtient 0,95 g d'anilino-2 [(pyrrolidinyl-1) carbonyl]-2 acétate de tert-butyle-(RS) fondant à 80°C.

Le bromo-2 [(pyrrolidinyl-1) carbonyl]-2 acétate de tert-butyle-(RS) peut être préparé de la manière suivante : à une solution de 2,6 g de [(pyrrolidinyl-1) carbonyl]-2 acétate de tert-butyle et de 1,18 g d'acétamide dans 45 cm3 de chloroforme maintenue à reflux, on ajoute goutte à goutte 0,51 cm3 de brome et le mélange est chauffé 11 heures à reflux. Après refroidissement, l'insoluble est séparé par filtration et le filtrat est lavé par 2 fois 10 cm3 d'une solution saturée d'hydrogénocarbonate de sodium, séché sur sulfate de sodium, puis concentré à sec sous pression réduite (2,7 kPa) à 35°C. On obtient ainsi 4,4 g de bromo-2 [(pyrrolidinyl-1) carbonyl]-2 acétate de tert-butyle-(RS) sous forme d'huile utilisée telle quelle dans les synthèses ultérieures.

Le [(pyrrolidinyl-1) carbonyl]-2 acétate de tert-butyle peut être préparé de la façon suivante : à une solution de 1,6 g de malonate acide de tert-butyle dans 16 cm3 de dichlorométhane maintenue à une température voisine de 0°C, on ajoute 0,84 cm3 de pyrrolidine puis une solution de 2,1 g de dicyclohexylcarbodiimide dans 10 cm3 de dichlorométhane. Le mélange est agité pendant 7 jours à une température voisine de 25°C, l'insoluble est séparé par filtration et le filtrat est concentré à sec sous pression réduite (2,7 kPa) à 35°C. On obtient ainsi 2,6 g de [(pyrrolidinyl-1) carbonyl]-2 acétate de tert-butyle à l'état d'huile utilisée telle quelle dans les synthèses ultérieures.

Le malonate acide de tert-butyle peut être préparé selon la méthode décrite dans Acta Chem. Scand. B29, 687 (1975).

### EXEMPLE 43

On opère d'une manière analogue à celle décrite à l'exemple 40, mais à partir de 2,2 g d'acide [(méthyl-3 phényl)-3 uréido]-2 acétique, de 3,4 g d'anilino-2 phénylcarbamoyl-2 acétate de tert-butyle-(RS) et de 0,75 cm3 de chlorure de thionyle. On obtient ainsi, après recristallisation dans 8 cm3 d'un mélange d'oxyde de diéthyle et d'acétate d'éthyle (90-10 en volumes), 0,45 g de {[(méthyl-3 phényl)-3 uréido]-2 N-phényl-acétamido}-2 phénylcarbamoyl-2 acétate de tert-butyle-(RS) fondant à 176°C.

L'anilino-2 phénylcarbamoyl-2 acétate de tert-butyle-(RS) peut être préparé d'une manière analogue à celle décrite à l'exemple 42 pour la préparation de l'anilino-2 [(pyrrolidinyl-1) carbonyl]-2 acétate de tert-butyle-(RS), mais à partir de 20,4 g de bromo-2 phénylcarbamoyl-2 acétate de tert-butyle-(RS) et de 9,1 cm3 d'aniline dans 100 cm3 d'acétonitrile. Après recristallisation dans 25 cm3 d'oxyde de diisopropyle, on obtient 3,4 g d'anilino-2 phénylcarbamoyl-2 acétate de tert-butyle-(RS) fondant à 125°C.

Le bromo-2 phénylcarbamoyl-2 acétate de tert-butyle-(RS) peut être préparé d'une manière analogue à celle décrite à l'exemple 42 pour la préparation du bromo-2 [(pyrrolidinyl-1) carbonyl]-2 acétate de tert-butyle-(RS) mais à partir de 16 g de phénylcarbamoyl-2 acétate de tert-butyle, de 5,9 g d'acétamide et de 8 cm3 de brome. On obtient ainsi 20,4 g de bromo-2 phénylcarbamoyl-2 acétate de tert-butyle-(RS) à l'état d'huile utilisée telle quelle dans les synthèses ultérieures.

Le phénylcarbamoyl-2 acétate de tert-butyle peut être préparé d'une manière analogue à celle décrite à l'exemple 42 pour la préparation du [(pyrrolidinyl-1) carbonyl]-2 acétate de tert-butyle mais à partir de 8 g de malonate acide de tert-butyle, de 4,5 cm3 d'aniline et de 10,5 g de dicyclohexylcarbodiimide. On obtient ainsi 16 g de phénylcarbamoyl-2 acétate de tert-butyle à l'état d'huile utilisée telle quelle dans les synthèses ultérieures.

### EXEMPLE 44

On opère d'une manière analogue à celle décrite à l'exemple 29, mais à partir de 1,95 g d'{[(imidazolyl-1) carboxamido]-2 N-phényl-acétamido}-2 N-méthyl N-phényl-acétamide et de 1,2 g d'éthyl-3 aniline. Le produit brut obtenu est purifié par chromatographie sur 50 g de silice (0,065-0,200 mm) contenus dans une colonne de 2 cm de diamètre [éluant : cyclohexane - acétate d' éthyle (40-60 en volumes)] en recueilllant des fractions de 25 cm3. Les fractions 7 à 13 sont réunies et concentrées à sec sous pression réduite (2,7 kPa) à 40°C. On obtient, après recristallisation dans l'oxyde de diisopropyle, 0,75 g d'{[(éthyl-3 phényl)-3 uréido]-2 N-phényl-acétamido}-2 N-méthyl N-phényl-acétamide fondant à 105°C.

### EXEMPLE 45

On opère d'une manière analogue à celle décrite à l'exemple 29, mais à partir de 1,95 g d'{[(imidazolyl-1) carboxamido]-2 N-phényl-acétamido}-2 N-méthyl N-phényl-acétamide et de 1,2 g d'alcool amino-3 benzylique. Le produit obtenu est purifié par chromatographie sur 50 g de silice (0,065-0,200mm) contenus dans une colonne de 1,5 cm de diamètre [éluant : chlorure de méthylène-éthanol (95-5 en volumes)] en recueillant des fractions de 20 cm3. Les fractions 6 à 13 sont réunies et concentrées à sec sous pression réduite (2,7kPa) à 40°C. On obtient, après recristallisation dans l'oxyde de diisopropyle, 1 g d'{[(hydroxyméthyl-3 phényl)-3 uréido]-2 N-phényl-acétamido}-2 N-méthyl N-phényl acétamide fondant à 120°C.

### EXEMPLE 46

On opère d'une manière analogue à celle décrite à l'exemple 29, mais à partir de 2,0 g d'{[(imidazolyl-1) carboxamido]-2 N-phényl-acétamido}-2 N-méthyl N-phényl-acétamide et de 1,55 g d'(amino-3 phényl)-1 butanol-(RS). Le produit brut obtenu est purifié par chromatographie sur 50 g de silice (0,065-0,200 mm) contenus dans une colonne de 1,5 cm de diamètre [éluant : dichlorométhane-acétate d'éthyle (50-50 en volumes)] en recueillant des fractions de 25 cm3. Les fractions 20 à 29 sont réunies et concentrées à sec sous pression réduite (2,7kPa) à 40°C. On obtient ainsi, après recristallisation dans l'acétate d'éthyle, 0,9 g d'{{[(hydroxy-1 butyl)-3 phényl]-3 uréido}-2 N-phényl-acétamido}-2 N-méthyl N-phényl-acétamide-(RS) fondant à 154°C.

L'(amino-3 phényl)-1 butanol-(RS) peut être préparé de la manière suivante : à une solution de 2,2 g de (nitro-3 phényl)-1 butanol-(RS) dans 40 cm3 d'éthanol, on ajoute 0,2 g de palladium sur noir à 5%. La suspension est agitée 2 heures à une température voisine de 25°C sous atmosphère d'hydrogène (100kPa). Le catalyseur est ensuite séparé et le filtrat est concentré à sec sous pression réduite (2,7kPa) à 40°C. On obtient ainsi 1,8 g d'(amino-3 phényl)-1 butanol-(RS) sous forme d'une huile utilisée telle quelle dans les synthèses ultérieures.

Le (nitro-3 phényl)-1 butanol-(RS) peut être préparé selon la méthode décrite par E. FELDER et coll. J. Med. Chem., 13 559 (1970).

### EXEMPLE 47

On opère d'une manière analogue à celle décrite à l'exemple 29, mais à partir de 2,0 g d'{[(imidazolyl-1) carboxamido]-2 N-phényl-acétamido}-2 N-méthyl N-phényl-acétamide et de 1,1 g d'amino-3 phénol. On obtient, après recristallisation dans le méthanol, 0,8 g d'{[(hydroxy-3 phényl)-3 uréido]-2 N-phényl-acétamido}-2 N-méthyl N-phényl-acétamide fondant à 180°C.

### EXEMPLE 48

On opère d'une manière analogue à celle décrite à l'exemple 29, mais à partir de 2,35 g d'{[(imidazolyl-1) carboxamido]-2 N-phényl-acétamido}-2 N-méthyl N-phényl-acétamide et de 1,65 g d'(amino-3 phényl)-2 éthanol. Le produit brut obtenu est purifié par chromatographie sur 150 g de silice (0,065-0,200mm) contenus dans une colonne de 4,1 cm de diamètre [éluant : chlorure de méthylène-éthanol (96-4 en volumes)] en recueillant des fractions de 20 cm3. Les fractions 17 à 32 sont réunies et concentrées à sec sous pression réduite (2,7kPa) à 40°C. On obtient, après recristallisation dans l'acétonitrile, 0,89 g d'{{[(hydroxy-2 éthyl)-3 phényl]-3 uréido}-2 N-phényl-acétamido}-2 N-méthyl N-phényl-acétamide fondant à 152°C.

L'(amino-3 phényl)-2 éthanol peut être préparé selon la méthode décrite par B. CARNMALM et coll., Acta Pharm. Suecica, 11, 33 (1974).

### EXEMPLE 49

On opère d'une manière analogue à celle décrite à l'exemple 29, mais à partir de 1,58 g de N-[(diméthyl-3,3 pipéridino)-2 oxo-2 éthyl] [(imidazolyl-1) carboxamido]-2 N-phényl-acétamide et de 0,98 g d'hydroxyméthyl-3 aniline. On obtient après recristallisation dans un mélange diméthylformamide-acétonitrile (25-75 en volumes), 1,0 g de N-[(diméthyl-3,3 pipéridino)-2 oxo-2 éthyl] [(hydroxyméthyl-3 phényl-3) uréido]-2 N-phényl-acétamide fondant à 248°C.

### EXEMPLE 50

On opère d'une manière analogue à celle décrite à l'exemple 29, mais à partir de 2,0 g de N-[(diméthyl-3,3 pipéridino)-2 oxo-2 éthyl] [(imidazolyl-1) carboxamido]-2 N-phényl-acétamide et de 1,4 g d'(amino-3 phényl)-2 éthanol. On obtient après recristallisation dans l'acétonitrile, 0,45 g de N-[(diméthyl-3,3 pipéridino)-2 oxo-2 éthyl] {[(hydroxy-2 éthyl)-3 phényl]-3 uréido}-2 N-phényl-acétamide fondant à 175°C.

### EXEMPLE 51

On opère d'une manière analogue à celle décrite à l'exemple 1, mais à partir de 1,5 g d'amino-2 N-[oxo-1 (tétrahydro-1,2,3,4 quinolyl-1)-1 propyl-2] N-phényl-acétamide-(RS) et de 0,59 g d'isocyanate de méthyl-3 phényle. On obtient, après recristallisation dans un mélange acétonitrile-diméthylformamide (96-4 en volumes), 1,4 g de [(méthyl-3 phényl)-3 uréido]-2 N-[oxo-1 (tétrahydro-1,2,3,4 quinolyl-1)-1 propyl-2] N-phényl-acétamide-(RS) fondant à 215°C.

L'amino-2 N-[oxo-1 (tétrahydro-1,2,3,4 quinolyl-1)-1 propyl-2 ] N-phényl-acétamide-(RS) peut être préparé d'une manière analogue à celle décrite à l'exemple 1, pour la préparation de l'(amino-2 N-phényl-acétamido)-2 N-phényl-acétamide , mais à partir de 9,7 g de N-[oxo-1 (tétrahydro-1,2,3,4 quinolyl-1)-1 propyl-2] N-phényl phtalimido-2 acétamide-(RS) et de 2,07 g d'hydrate d'hydrazine. On obtient ainsi, 6,8 g d'amino-2 N-[oxo-1 (tétrahydro-1,2,3,4 quinolyl-1)-1 propyl-2] N-phényl-acétamide- (RS) sous forme d'une huile utilisée telle quelle dans les synthèses ultérieures.

Le N-[oxo-1 (tétrahydro-1,2,3,4 quinolyl-1)-1 propyl-2] N-phényl phtalimido-2 acétamide-(RS) peut être préparé d'une manière analogue à celle décrite à l'exemple 1, pour la préparation du (N-phényl phtalimido-2 acétamido)-2 acétate de tert-butyle mais à partir de 7,8 g de (phénylamino-2 propionyl)-1 tétrahydro-1,2,3,4 quinoléine-(RS) et de 6,2 g de chlorure de phtalimido-2 acétyle. On obtient ainsi 9,8 g de N-[(oxo-1 (tétrahydro-1,2,3,4 quinolyl-1)-1 propyl-2 ] N-phényl phtalimido-2 acétamide-(RS) fondant à 191°C.

La (phénylamino-2 propionyl)-1 tétrahydro-1,2,3,4 quinoléine-(RS) peut être préparée d'une manière analogue à celle décrite à l'exemple 21 pour la préparation de la diméthyl-3,3 (phénylamino-2 propionyl)-1 pipéridine-(RS), mais à partir de 8,5 g de chlorure de bromo-2 propionyle, de 6,6 g de tétrahydro-1,2,3,4 quinoléine, de 6,1 g de triéthylamine et de 7 g d'aniline. On obtient, après recristallisation dans le cyclohexane, 7,8 g de (phénylamino-2 propionyl)-1 tétrahydro-1,2,3,4 quinoléine-(RS) fondant à 98°C.

### EXEMPLE 52

On opère d'une manière analogue à celle décrite à l'exemple 29, mais à partir de 2 g d'[(imidazolyl-1) carboxamido]-2 N-[oxo-1 (tétrahydro-1,2,3,4 quinolyl-1)-1 propyl-2] N-phényl-acétamide-(RS) et de 1,3 g d'(amino-3 phényl)-1 éthanol-(RS). Le produit brut obtenu est purifié par chomatographie sur 40 g de silice (0,065-0,200mm) contenus dans une colonne de 2,4 cm de diamètre [éluant : dichlorométhane-acétate d'éthyle (60-40 en volumes)] en recueillant des fractions de 25 cm3. Les fractions 15 à 21 sont réunies et concentrées à sec sous pression réduite (2,7kPa) à 40°C. On obtient après recristallisation dans l'acétate d'éthyle 0,9 g d'{[(hydroxy-1 éthyl)-3 phényl-(RS)]-3 uréido}-2 N-[oxo-1 (tétrahydro-1,2,3,4 quinolyl-1)-1 propyl-2 ] N-phényl-acétamide-(RS) fondant à 170°C.

L'[(imidazolyl-1) carboxamido]-2 N-[oxo-1 (tétrahydro-1,2,3,4 quinolyl-1)-1 propyl-2] N-phényl-acétamide-(RS) peut être préparé d'une manière analogue à celle décrite à l'exemple 29 pour la préparation de l'{[(imidazolyl-1) carboxamido]-2 N-phényl-acétamido}-2 N-méthyl N-phényl-acétamide, mais à partir de 5,3 g d'amino-2 N-[oxo-1 (tétrahydro-1,2,3,4 quinolyl-1)-1 propyl-2] N-phényl-acétamide-(RS) et de 3,07 g de N,N'-diimidazole carbonyle. On obtient ainsi 6,5 g d'[(imidazolyl-1) carboxamido]-2 N-[oxo-1 (tétrahydro-1,2,3,4 quinolyl-1)-1 propyl-2] N-phényl-acétamide-(RS) sous forme d'une meringue utilisée telle quelle dans les synthèses ultérieures.

### EXEMPLE 53

On opère d'une manière analogue à celle décrite à l'exemple 29 mais à partir de 2,0 g d'[(imidazolyl-1) carboxamido]-2 N-[oxo-1 (tétrahydro-1,2,3,4 quinolyl-1)-1 propyl-2] N-phényl-acétamide-(RS) et de 1,28 g de méthylthio-3 aniline. Le produit brut obtenu est purifié par chromatographie sur 40 g de silice (0,065-0,200mm) contenus dans une colonne de 2,4 cm de diamètre [éluant : dichlorométhane-acétate d'éthyle) (70-30 en volumes)] en recueillant des fractions de 25 cm3. Les fractions 12 à 20 sont réunies et concentrées à sec sous pression réduite (2,7kPa) à 40°C. On obtient, après recristallisation dans un mélange oxyde de diéthyle-acétate d'éthyle (90-10 en volumes), 0,75 g de [(méthylthio-3 phényl)-3 uréido]-2 N-[oxo-1 (tétrahydro-1,2,3,4 quinolyl-1)-1 propyl-2] N-phényl-acétamide-(RS) fondant à 165°C.

### EXEMPLE 54

A une solution de 3 g d'(amino-2 N-phényl-acétamido)-2 N-méthyl N-phényl-acétamide dans 50 cm3 dichloro-1,2 éthane à une température voisine de 25°C, on ajoute 1,5 g de triéthylamine, puis 2,7 g de chlorure d'indolecarbonyle-2 en solution dans 20 cm3 de dichloro-1,2 éthane et le mélange réactionnel est agité pendant 18 heures à une température voisine de 25°C. On ajoute alors 50 cm3 de dichlorométhane puis 30 cm3 d'une solution aqueuse saturée d'hydrogénocarbonate de sodium. La phase organique est lavée par 2 fois 30 cm3 d'eau distillée, séchée sur sulfate de magnésium et concentrée à sec sous pression réduite (2,7kPa) à 40°C. On obtient, après recristallisation dans un mélange diméthylformamide-méthanol (65-35 en volumes), 0,6 g de N-[N-(N-méthyl N-phényl-carbamoylméthyl) N-phényl-carbamoylméthyl] indolecarboxamide-2 fondant à 255°C.

Le chlorure d'indolecarbonyle-2 peut être préparé de la manière suivante : à une suspension de 4,0 g d'acide indolecarboxylique-2 dans 80 cm3 d'oxyde de diéthyle anhydre à une température voisine de 5°C, on ajoute 0,2 cm3 de diméthylformamide, puis 3,25 g de dichlorure d'oxalyle en solution dans 20 cm3 d'oxyde de diéthyle anhydre. Le mélange réactionnel est agité à une température voisine de 25°C pendant 2 heures puis la phase éthérée est concentrée à sec sous pression réduite (2,7kPa) à 30°C. On obtient ainsi 4,0 g de chlorure d'indolecarbonyle-2 fondant à 120°C.

### EXEMPLE 55

A une suspension de 2,4 g d'acide naphtalènecarboxylique-2 dans 50 cm3 d'oxyde de diéthyle anhydre, on ajoute en 5 minutes 1,8 g de dichlorure d'oxalyle. Le mélange est agité pendant 3 heures à une température voisine de 25°C, puis concentré à sec sous pression réduite (2,7kPa) à 30°C. Le résidu obtenu est dissous dans 25 cm3 de dichloro-1,2 éthane et la solution est ajoutée en 10 minutes à un mélange de 3,8 g d'(amino-2 N-phényl-acétamido)-2 N-méthyl N-phényl-acétamide et de 2,0 g de triéthylamine dans 50 cm3 de dichlorométhane maintenu à une température voisine de 5°C. Le mélange est agité pendant 16 heures à une température voisine de 25°C. On ajoute alors 50 cm3 de dichlorométhane et 30 cm3 d'une solution aqueuse saturée d'hydrogénocarbonate de sodium. La phase organique est lavée par 2 fois 30 cm3 d'eau distillée, séchée sur sulfate de magnésium et concentrée à sec sous pression réduite (2,7kPa) à 40°C. On obtient, après recristallisation dans l'acétate d'éthyle, 1,4 g de N-[N-(N-méthyl N-phényl-carbamoylméthyl) N-phényl-carbamoylméthyl] naphtalènecarboxamide-2 fondant à 134°C.

### EXEMPLE 56

On opère d'une manière analogue à celle décrite à l'exemple 1, mais à partir de 3,1 g d'amino-2 N-[(dihydro-3,4 2H-benzothiazine-1,4 yl-4)-2 oxo-2 éthyl] N-phényl-acétamide et de 1,2 g d'isocyanate de méthyl-3 phényle. On obtient après recristallisation dans l'acétate d'éthyle, 2,4 g de N-[(dihydro-3,4 2H-benzothiazine-1,4 yl-4)-2 oxo-2 éthyl] [(méthyl-3 phényl)-3 uréido]-2 N-phényl-acétamide fondant à 170°C.

L'amino-2 N-[(dihydro-3,4 2H-benzothiazine-1,4 yl-4) oxo-2 éthyl] N-phényl-acétamide peut être préparé d'une manière analogue à celle décrite à l'exemple 1, pour la préparation de l'(amino-2 N-phényl-acétamido)-2 N-phényl-acétamide, mais à partir de 4,5 g de N-[(dihydro-3,4 2H-benzothiazine-1,4 yl-4)-2 oxo-2 éthyl] N-phényl phtalimido-2 acétamide et de 0,95 g d'hydrate d'hydrazine. On obtient ainsi, 3,1 g d'amino-2 N-[(dihydro-3,4 2H-benzothiazine-1,4 yl-4)-2 oxo-2 éthyl] N-phényl-acétamide sous forme d'une huile utilisée telle quelle dans les synthèses ultérieures.

Le N-[(dihydro-3,4 2H-benzothiazine-1,4 yl-4)-2 oxo-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 5,1 g d'acide (N-phényl phtalimido-2 acétamido)-2 acétique, de 2,1 g de dichlorure d'oxalyle et de 6 g de dihydro-3,4 2H-benzothiazine-1,4. On obtient, après recristallisation dans l'éthanol, 4,5 g de N-[(dihydro-3,4 2H-benzothiazine-1,4 yl-4)-2 oxo-2 éthyl] N-phényl phtalimido-2 acétamide fondant à 120°C.

La dihydro-3,4 2H-benzothiazine-1,4 peut être préparée selon la méthode décrite par C.C.J. CULVENOR et coll., J.Chem. Soc., 278 (1949).

### EXEMPLE 57

On opère d'une manière analogue à celle décrite à l'exemple 1, mais à partir de 1,2 g d'amino-2 N-[(diméthyl-2,6 pipéridino)-2 oxo-2 éthyl] N-phényl-acétamide-cis et de 0,61 g d'isocyanate de méthyl-3 phényle. On obtient après recristallisation dans un mélange acétate d'éthyle-acétonitrile (50-50 en volumes), 0,75 g de N-[(diméthyl-2,6 pipéridino)-2 oxo-2 éthyl] [(méthyl-3 phényl)-3 uréido]-2 N-phényl-acétamide-cis fondant à 212°C.

L'amino-2 N-[(diméthyl-2,6 pipéridino)-2 oxo-2 éthyl] N-phényl-acétamide-cis peut être préparé d'une manière analogue à celle décrite à l'exemple 1, pour la préparation de l'(amino-2 N-phényl-acétamido)-2 N-phényl-acétamide, mais à partir de 3,3 g de N-[(diméthyl-2,6 pipéridino)-2 oxo-2 éthyl] N-phényl phtalimido-2 acétamide-cis et de 0,82 g d'hydrate d'hydrazine. On obtient ainsi, 1,2 g d'amino-2 N-[(diméthyl-2,6 pipéridino)-2 oxo-2 éthyl] N-phényl-acétamide-cis sous forme d'une huile utilisée telle quelle dans les synthèses ultérieures.

Le N-[(diméthyl-2,6 pipéridino)-2 oxo-2 éthyl] N-phényl phtalimido-2 acétamide-cis peut être préparé d'une manière analogue à celle décrite à l'exemple 1, pour la préparation du (N-phényl phtalimido-2 acétamido)-2 N-phényl-acétamide, mais à partir de 3,38 g d'acide (N-phényl phtalimido-2 acétamido)-2 acétique, de 1,4 g de dichlorure d'oxalyle et de 2,5 g de diméthyl-2,6 pipéridine-cis. On obtient ainsi, 3,3 g de N-[(diméthyl-2,6 pipéridino)-2 oxo-2 éthyl] N-phényl phtalimido-2 acétamide-cis , sous forme d'une meringue utilisée telle quelle dans les synthèses ultérieures.

### EXEMPLE 58

On opère d'une manière analogue à celle décrite à l'exemple 1, mais à partir de 2,4 g d'amino-2 N-[(dihydro-3,4 2H-benzoxazine-1,4 yl-4)-2 oxo-2 éthyl] N-phényl-acétamide et de 0,98 g d'isocyanate de méthyl-3 phényle. On obtient après recristallisation dans l'acétonitrile, 1,6 g de N-[(dihydro-3,4 2H-benzoxazine-1,4 yl-4)-2 oxo-2 éthyl] [(méthyl-3 phényl)-3 uréido]-2 N-phényl-acétamide fondant à 185°C.

L'amino-2 N-[(dihydro-3,4 2H-benzoxazine-1,4 yl-4)-2 oxo-2 éthyl] N-phényl-acétamide peut être préparé d'une manière analogue à celle décrite à l'exemple 1, pour la préparation de l'(amino-2 N-phényl-acétamido)-2 N-phényl-acétamide, mais à partir de 3,8 g de N-[(dihydro-3,4 2H-benzoxazine-1,4 yl-4)-2 oxo-2 éthyl] N-phényl phtalimido-2 acétamide et de 0,83 g d'hydrate d'hydrazine. On obtient ainsi, 2,4 g d'amino-2 N-[(dihydro-3,4 2H-benzoxazine-1,4 yl-4)-2 oxo-2 éthyl] N-phényl-acétamide sous forme d'une huile utilisée telle quelle dans les synthèses ultérieures.

Le N-[(dihydro-3,4 2H-benzoxazine-1,4 yl-4)-2 oxo-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 5,1 g d'acide (N-phényl phtalimido-2 acétamido)-2 acétique, de 2,1 g de dichlorure d'oxalyle et de 4,8 g de dihydro-3,4 2H-benzoxazine-1,4. Le produit brut obtenu est purifié par chromatographie sur 80 g de silice (0,065-0,200mm) contenus dans une colonne de 3,2 cm de diamètre [éluant : chlorure de méthylène-acétate d'éthyle [90-10 en volumes)] en recueillant des fractions de 20 cm3. Les fractions 17 à 24 sont réunies et concentrées à sec sous pression réduite (2,7kPa) à 40°C. On obtient, après recristallisation dans un mélange acétate d'éthyle-oxyde d'isopropyle (50-50 en volumes), 3,9 g de N-[(dihydro-3,4 2H-benzoxazine-1,4 yl-4)-2 oxo-2 éthyl] N-phényl phtalimido-2 acétamide fondant à 173°C.

La dihydro-3,4 2H-benzoxazine-1,4 peut être préparée selon la méthode décrite par SHIRAI HIDEAKI et Coll., C.A., 78, 25378z.

### EXEMPLE 59

On opère d'une manière analogue à celle décrite à l'exemple 1, mais à partir de 1,7 g d'amino-2 N-[(éthyl-4 méthyl-4 pipéridino)-2 oxo-2 éthyl] N-phényl-acétamide et de 0,7 g d'isocyanate de méthyl-3 phényle. On obtient après recristallisation dans l'acétate d'éthyle, 0,5 g de N-[(éthyl-4 méthyl-4 pipéridino)-2 oxo-2 éthyl] [(méthyl-3 phényl)-3 uréido]-2 N-phényl-acétamide fondant à 204°C.

L'amino-2 N-[(éthyl-4 méthyl-4 pipéridino)-2 oxo-2 éthyl] N-phényl-acétamide peut être préparé d'une manière analogue à celle décrite à l'exemple 1, pour la préparation de l'(amino-2 N-phényl-acétamido)-2 N-phényl-acétamide, mais à partir de 2,7 g de N-[(éthyl-4 méthyl-4 pipéridino)-2 oxo-2 éthyl] N-phényl phtalimido-2 acétamide et de 0,6 g d'hydrate d'hydrazine. On obtient ainsi, 1,7 g d'amino-2 N-[(éthyl-4 méthyl-4 pipéridino)-2 oxo-2 éthyl] N-phényl-acétamide sous forme d'une huile utilisée telle quelle dans les synthèses ultérieures.

Le N-[(éthyl-4 méthyl-4 pipéridino)-2 oxo-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 5,0 g d'acide (N-phényl phtalimido-2 acétamido)-2 acétique, de 2,3 g de dichlorure d'oxalyle et de 3,8 g d'éthyl-4 méthyl-4 pipéridine. On obtient, après recristallisation dans un mélange cyclohexane -acétate d'éthyle (50-50 en volumes), 2,8 g de N-[(éthyl-4 méthyl-4 pipéridino)-2 oxo-2 éthyl] N-phényl phtalimido-2 acétamide fondant à 166°C.

L'éthyl-4 méthyl-4 pipéridine peut être préparée selon la méthode décrite par J.M.Mc. MANUS et coll., J. Med. Chem., 774 (1965).

### EXEMPLE 60

On opère d'une manière analogue à celle décrite à l'exemple 1, mais à partir de 1,0 g d'amino-2 N-[(diméthyl-4,4 pipéridino)-2 oxo-2 éthyl] N-phényl-acétamide et de 0,44 g d'isocyanate de méthyl-3 phényle. On obtient après recristallisation dans un mélange acétate d'éthyle-acétonitrile (50-50 en volumes) 0,7 g de N-[(diméthyl-4,4 pipéridino)-2 oxo-2 éthyl] [(méthyl-3 phényl)-3 uréido]-2 N-phényl-acétamide fondant à 205°C.

L'amino-2 N-[(diméthyl-4,4 pipéridino)-2 oxo-2 éthyl] N-phényl-acétamide peut être préparé d'une manière analogue à celle décrite à l'exemple 1, pour la préparation de l'(amino-2 N-phényl-acétamido)-2 N-phényl-acétamide, mais à partir de 6,2 g de N-[(diméthyl-4,4 pipéridino)-2 oxo-2 éthyl] N-phényl phtalimido-2 acétamide et de 1,45 g d'hydrate d'hydrazine. On obtient ainsi, 2,6 g d'amino-2 N-[(diméthyl-4,4 pipéridino)-2 oxo-2 éthyl] N-phényl-acétamide sous forme d'une huile utilisée telle quelle dans les synthèses ultérieures.

Le N-[(diméthyl-4,4 pipéridino)-2 oxo-2 éthyl] N-phényl phtalimido-2 acétamide peut être préparé d'une manière analogue à celle décrite à l'exemple 1, pour la préparation du (N-phényl phtalimido-2 acétamido)-2 N-phényl-acétamide, mais à partir de 9,0 g d'acide (N-phényl phtalimido-2 acétamido)-2 acétique, de 3,8 g de dichlorure d'oxalyle et de 6 g de diméthyl-4,4 pipéridine. On obtient ainsi, après recristallisation dans l'acétate d'éthyle, 4,25g de N-[(diméthyl-4,4 pipéridino)-2 oxo-2 éthyl] N-phényl phtalimido-2 acétamide fondant à 131°C.

La diméthyl-4,4 pipéridine peut être préparée selon la méthode décrite par J.M.Mc. MANUS et coll., J. Med. Chem., 774 (1965).

### EXEMPLE 61

On opère d'une manière analogue à celle décrite à l'exemple 1, mais à partir de 2,6 g d'amino-2 N-[(diméthyl-3,5 pipéridino)-2 oxo-2 éthyl) N-phényl-acétamide, mélange des isomères cis et trans et de 1,06 g d'isocyanate de méthyl-3 phényle. Le produit brut obtenu est purifié par chromatographie sur 50 g de silice (0,065-0,200mm) contenus dans une colonne de 2,5 cm de diamètre (éluant : chlorure de méthylène-méthanol (99-1 en volumes) en recueillant des fractions de 20 cm3. Les fractions 27 à 36 sont réunies et concentrées à sec sous pression réduite (2,7kPa) à 40°C. On obtient après recristallisation dans l'éthanol, 1,4 g de N-[(diméthyl-3,5 pipéridino)-2 oxo-2 éthyl] [(méthyl-3 phényl)-3 uréido]-2 N-phényl-acétamide, mélange des isomères cis et trans fondant à 210°C.

L'amino-2 N-[(diméthyl-3,5 pipéridino)-2 oxo-2 éthyl] N-phényl-acétamide, mélange des isomères cis et trans peut être préparé d'une manière analogue à celle décrite à l'exemple 1, pour la préparation de l'(amino-2 N-phényl-acétamido)-2 N-phényl-acétamide, mais à partir de 6,8 g de N-[(diméthyl-3,5 pipéridino)-2 oxo-2 éthyl] N-phényl phtalimido-2 acétamide et de 1,56 g d'hydrate d'hydrazine. On obtient ainsi, 2,6 g d'amino-2 N-[(diméthyl-3,5 pipéridino)-2 oxo-2 éthyl] N-phényl-acétamide, mélange des isomères cis et trans sous forme d'une huile utilisée telle quelle dans les synthèses ultérieures.

Le N-[(diméthyl-3,5 pipéridino)-2 oxo-2 éthyl] N-phényl phtalimido-2 acétamide mélange des isomères cis et trans peut être préparé d'une manière analogue à celle décrite à l'exemple 1, pour la préparation du (N-phényl phtalimido-2 acétamido)-2 N-phényl-acétamide, mais à partir de 10 g d'acide (N-phényl phtalimido-2 acétamido)-2 acétique, de 3,74 g de dichlorure d'oxalyle et de 6,6 g de diméthyl-3,5 pipéridine (mélange des isomères cis et trans). Le produit brut obtenu est purifié par chromatographie sur 80 g de silice (0,065-0,200mm) contenus dans une colonne de 3,5 cm de diamètre [éluant : chlorure de méthylène-méthanol (99-1 en volumes)] en recueillant des fractions de 20 cm3. Les fractions 4 à 28 sont réunies et concentrées à sec sous pression réduite (2,7kPa) à 40°C. On obtient ainsi 6,8 g de N-[(diméthyl-3,5 pipéridino)-2 oxo-2 éthyl] N-phényl phtalimido-2 acétamide, mélange des isomères cis et trans sous forme d'une meringue utilisée telle quelle dans les synthèses ultérieures.

### EXEMPLE 62

On opère d'une manière analogue à celle décrite à l'exemple 1, mais à partir de 2,0 g d'amino-2 N-[(éthyl-5 méthyl-2 pipéridino)-2 oxo-2 éthyl] N-phényl-acétamide, mélange des isomères cis et trans et de 0,8 g d'isocyanate de méthyl-3 phényle. Le produit brut obtenu est purifié par chromatographie sur 50 g de silice (0,065-0,200mm) contenus dans une colonne de 1,5 cm de diamètre [éluant : chlorure de méthylène-méthanol (98-2 en volumes)] en recueillant des fractions de 20 cm3. Les fractions 2 à 8 sont réunies et concentrées à sec sous pression réduite (2,7kPa) à 40°C. On obtient, après recristallisation dans un mélange acétate d'éthyle-oxyde de diéthyle (40-60 en volumes), 0,5 g de N-[(éthyl-5 méthyl-2 pipéridino)-2 oxo-2 éthyl] [(méthyl-3 phényl)-3 uréido]-2 N-phényl-acétamide, mélange des isomères cis et trans fondant à 158°C.

L'amino-2 N-[(éthyl-5 méthyl-2 pipéridino)-2 oxo-2 éthyl] N-phényl-acétamide, mélange des isomères cis et trans peut être préparé d'une manière analogue à celle décrite à l'exemple 1 pour la préparation de l'amino-2 N-phényl-acétamido)-2 N-phényl-acétamide, mais à partir de 2,7 g de N-[(éthyl-5 méthyl-2 pipéridino)-2 oxo-2 éthyl] N-phényl phtalimido-2 acétamide mélange des isomères cis et trans et de 0,6 g d'hydrate d'hydrazine. On obtient ainsi, 2,0 g d'amino-2 N-[(éthyl-5 méthyl-2 pipéridino)-2 oxo-2 éthyl] N-phényl-acétamide, mélange des isomères cis et trans sous forme d'une huile utilisée telle quelle dans les synthèses ultérieures.

Le N-[(éthyl-5 méthyl-2 pipéridino)-2 oxo-2 éthyl] N-phényl phtalimido-2 acétamide, mélange des isomères cis et trans peut être préparé d'une manière analogue à celle décrite à l'exemple 1, pour la préparation du (N-phényl phtalimido-2 acétamido)-2 N-phényl-acétamide, mais à partir de 5,0 g d'acide (N-phényl phtalimido-2 acétamido)-2 acétique, de 2,3 g de dichlorure d'oxalyle et de 3,8 g d'éthyl-5 méthyl-2 pipéridine (mélange des isomères cis et trans). Le produit brut obtenu est purifié par chromatographie sur 80 g de silice (0,065-0,200mm) contenus dans une colonne de 2,5 cm de diamètre [éluant : chlorure de méthylène-méthanol (98-2 en volumes)] en recueillant des fractions de 30 cm3. Les fractions 8 à 18 sont réunies et concentrées à sec sous pression réduite (2,7 kPa) à 40°C. On obtient ainsi 2,7 g de N-[(éthyl-5 méthyl-2 pipéridino)-2 oxo-2 éthyl] N-phényl phtalimido-2 acétamide, mélange des isomères cis et trans sous forme d'une meringue utilisée telle quelle dans les synthèses ultérieures.

### EXEMPLE 63

On opère d'une manière analogue à celle décrite à l'exemple 1, mais à partir de 1,9 g d'(amino-2 N-phényl-acétamido)-2 N-(chloro-2 phényl) N-méthyl-acétamide et de 0,76 g d'isocyanate de méthyl-3 phényle. Le produit obtenu est purifié par chromatographie sur 100 g de silice (0,065-0,200mm) contenus dans une colonne de 3,2 cm de diamètre [éluant : acétate d'éthyle-cyclohexane (50-50 en volumes)] en recueillant des fractions de 25 cm3. Les fractions 7 à 15 sont réunies et concentrées à sec sous pression réduite (2,7kPa) à 40°C. On obtient, après recristallisation dans un mélange oxyde de diéthyle-acétate d'éthyle (75-25 en volumes), 1,0 g de N-(chloro-2 phényl) N-méthyl {[(méthyl-3 phényl)-3 uréido]-2 N-phényl-acétamido]-2 acétamide fondant à 175°C.

L'(amino-2 N-phényl-acétamido)-2 N-(chloro-2 phényl) N-méthyl-acétamide peut être préparé d'une manière analogue à celle décrite à l'exemple 1, pour la préparation de l'(amino-2 N-phényl acétamido)-2 N-phényl-acétamide, mais à partir de 2,6 g de N-(chloro-2 phényl) N-méthyl (N-phényl phtalimido-2 acétamido)-2 acétamide et de 0,6 g d'hydrate d'hydrazine. On obtient ainsi, 1,9 g d'(amino-2 N-phényl-acétamido)-2 N-(chloro-2 phényl) N-méthyl-acétamide sous forme d'une huile utilisée telle quelle dans les synthèses ultérieures.

Le N-(chloro-2 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 4,15 g d'acide (N-phényl phtalimido-2 acétamido)-2 acétique, de 1,9 g de dichlorure d'oxalyle et de 3,5 g de chloro-2 N-méthyl-aniline. Le produit brut obtenu est purifié par chromatographie sur 60 g de silice (0,065-0,200mm) contenus dans une colonne de 2,2 cm de diamètre [éluant : acétate d'éthyle-cyclohexane (50-50 en volumes)] en recueillant des fractions de 25 cm3. Les fractions 6 à 10 sont réunies et concentrées à sec sous pression réduite (2,7kPa) à 40°C. On obtient après recristallisation dans l'oxyde de diisopropyle, 2,6 g de N-(chloro-2 phényl) N-méthyl (N-phényl phtalimido-2 acétamido)-2 acétamide fondant à 175°C.

La chloro-2 N-méthyl-aniline peut être préparée selon la méthode décrite par R. STOERMER, Chem. Ber., 31, 2531 (1898).

### EXEMPLE 64

On opère d'une manière analogue à celle décrite à l'exemple 1, mais à partir de 10,0 g d'(amino-2 N-phényl-acétamido)-2 N-(chloro-3 phényl) N-méthyl-acétamide et de 3,9 g d'isocyanate de méthyl-3 phényle. Le produit brut obtenu est purifié par chromatographie sur 150 g de silice (0,065-0,200mm) contenus dans une colonne de 3,5 cm de diamètre [éluant : dichlorométhane-méthanol (99-1 en volumes)] en recueillant des fractions de 50 cm3. Les fractions 5 à 18 sont réunies et concentrées à sec sous pression réduite (2,7kPa) à 40°C. On obtient, après recristallisation dans un mélange acétate d'éthyle-oxyde de diéthyle (60-40 en volumes), 5,5 g de N-(chloro-3 phényl) N-méthyl {[(méthyl-3 phényl)-3 uréido]-2 N-phényl-acétamido}-2 acétamide fondant à 196°C.

L'(amino-2 N-phényl-acétamido)-2 N-(chloro-3 phényl) N-méthyl-acétamide peut être préparé d'une manière analogue à celle décrite à l'exemple 1, pour la préparaton de l'(amino-2 N-phényl-acétamido)-2 N-phényl-acétamide, mais à partir de 15,6 g de N-(chloro-3 phényl) N-méthyl (N-phényl phtalimido-2 acétamido)-2 acétamide et de 3,3 g d'hydrate d'hydrazine. On obtient ainsi, 10,0 g d'(amino-2 N-phényl-acétamido)-2 N-(chloro-3 phényl) N-méthyl-acétamide sous forme d'une huile utilisée telle quelle dans les synthèses ultérieures.

Le N-(chloro-3 phényl) N-méthyl (N-phényl phtalimido-2 acétamido)-2 acétamide peut être préparé d'un manière analogue à celle décrite à l'exemple 1, pour la préparation du (N-phényl phtalimido-2 acétamido)-2 N-phényl acétamide mais à partir de 16,7 g d'acide (N-phényl phtalimido-2 acétamido)-2 acétique, de 6,7 g de dichlorure d'oxalyle et de 14,0 g de chloro-3 N-méthyl-aniline. On obtient, après recristallisation dans un mélange acétate d'éthyle-oxyde de diéthyle (70-30 en volumes), 15,6 g de N-(chloro-3 phényl) N-méthyl (N-phényl phtalimido-2 acétamido)-2 acétamide fondant à 218°C.

La chloro-3 N-méthyl-aniline peut être préparée selon la méthode décrite par R. STOERMER, Chem. Ber., 31, 2531 (1898).

### EXEMPLE 65

On opère d'une manière analogue à celle décrite à l'exemple 1, mais à partir de 5,3 g d'(amino-2 N-phényl-acétamido)-2 N-(fluoro-2 phényl) N-méthyl-acétamide et de 2,3 g d'isocyanate de méthyl-3 phényle. Le produit brut obtenu est purifié par chromatographie sur 140 g de silice (0,065-0,200mm) contenus dans une colonne de 3,5 cm de diamètre [éluant dichlorométhane-méthanol (99-1 en volumes)] en recueillant des fractions de 30 cm3. Les fractions 8 à 48 sont réunies et concentrées à sec sous pression réduite (2,7kPa) à 40°C. On obtient, après recristallisation dans l'acétate d'éthyle, 1,8 g de N-(fluoro-2 phényl) N-méthyl {[(méthyl-3 phényl)-3 uréido]-2 N-phényl-acétamido}-2 acétamide fondant à 172°C.

L'(amino-2 N-phényl-acétamido)-2 N-(fluoro-2 phényl) N-méthyl-acétamide peut être préparé d'une manière analogue à celle décrite à l'exemple 1, pour la préparation de l'(amino-2 N-phényl-acétamido)-2 N-phényl-acétamide, mais à partir de 8,4 g de N-(fluoro-2 phényl) N-méthyl (N-phényl phtalimido-2 acétamido)-2 acétamide et de 2,0 g d'hydrate d'hydrazine. On obtient ainsi, 5,3 g d'(amino-2 N-phényl-acétamido)-2 N-(fluoro-2 phényl) N-méthyl-acétamide sous forme d'une huile utilisée telle quelle dans les synthèses ultérieures.

Le N-(fluoro-2 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 9,4 g d'acide (N-phényl phtalimido-2 acétamido)-2 acétique, de 4,3 g de dichlorure d'oxalyle et de 7,2 g de fluoro-2 N-méthyl aniline. On obtient, après recristallisation dans un mélange acétate d'éthyle-cyclohexane (50-50 en volumes), 8,4 g de N-(fluoro-2 phényl) N-méthyl (N-phényl phtalimido-2 acétamido)-2 acétamide fondant à 184°C.

La fluoro-2 N-méthyl-aniline peut être préparée de la manière suivante : à une suspension de 4,9 g d'hydrure de lithium-aluminium dans 100 cm3 de tétrahydrofuranne anhydre maintenue à une température voisine de 25°C, on ajoute en 15 minutes une solution de 12,2 g de fluoro-2 formanilide dans 100 cm3 de tétrahydrofuranne anhydre. Le mélange est agité à une température voisine de 25°C pendant 3 heures. Après refroidissement à une température voisine de 5°C, on ajoute successivement 5,7 cm3 d'eau, 4,2 cm3 d'une solution aqueuse 5N de soude puis 19 cm3 d'eau. La suspension obtenue est agitée pendant 30 minutes et on y ajoute 150 cm3 d'oxyde de diéthyle. Le produit insoluble est séparé par filtration et le filtrat est concentré à sec sous pression réduite (2,7kPa) à 40°C. Le résidu est dissous dans 60 cm3 de dichlorométhane et la phase organique est séchée sur sulfate de magnésium puis concentrée à sec sous pression réduite (2,7kPa) à 40°C. On obtient ainsi 7,2 g de fluoro-2 N-méthyl-aniline sous forme d'une huile utilisée telle quelle dans les synthèses ultérieures.

Le fluoro-2 formanilide peut être préparé de la manière suivante : à une solution de 10,8 g de méthylate de sodium dans 100 cm3 de diméthylformamide anhydre, on ajoute 11 g de fluoro-2 aniline. Le mélange est chauffé à reflux pendant 2 heures en distillant le méthanol formé, puis concentré à sec sous pression réduite (0,1kPa) à 60°C. Le résidu est repris par 1 litre d'eau et 300 cm3 d'oxyde de diéthyle. La phase organique est séparée, séchée sur sulfate de magnésium et concentrée à sec sous pression réduite (2,7kPa) à 30°C. On obtient ainsi 12,2 g de fluoro-2 formanilide sous forme d'une huile utilisée telle quelle dans les synthèses ultérieures.

### EXEMPLE 66

On opère d'une manière analogue à celle décrite à l'exemple 1, mais à partir de 2,6 g d'(amino-2 N-phényl-acétamido)-2 N-(fluoro-4 phényl) N-méthyl-acétamide et de 1,1 g d'isocyanate de méthyl-3 phényle. Le produit brut obtenu est purifié par chromatographie sur 60 g de silice (0,065-0,200mm) contenus dans une colonne de 2,0 cm de diamètre [éluant : acétate d'éthyle-cyclohexane (50-50 en volumes) en recueillant des fractions de 30 cm3. Les fractions 6 à 10 sont réunies et concentrées à sec sous pression réduite (2,7kPa) à 40°C. On obtient, après recristallisation dans l'acétate d'éthyle, 1,8 g de N-(fluoro-4 phényl) N-méthyl {[(méthyl-3 phényl)-3 uréido]-2 N-phényl-acétamido}-2 acétamide fondant à 135°C.

L'(amino-2 N-phényl-acétamido)-2 N-(fluoro-4 phényl) N-méthyl-acétamide peut être préparé d'une manière analogue à celle décrite à l'exemple 1, pour la préparation de l'(amino-2 N-phényl-acétamido)-2 N-phényl-acétamide, mais à partir de 4,5 g de N-(fluoro-4 phényl) N-méthyl (N-phényl phtalimido-2 acétamido)-2 acétamide et de 1,1 g d'hydrate d'hydrazine. On obtient ainsi, 2,6 g d'(amino-2 N-phényl-acétamido)-2 N-(fluoro-4 phényl) N-méthyl-acétamide sous forme d'une huile utilisée telle quelle dans les synthèses ultérieures.

Le N-(fluoro-4 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 5,0 g d'acide (N-phényl phtalimido-2 acétamido)-2 acétique, de 2,3 g de dichlorure d'oxalyle et de 3,75 g de fluoro-4 N-méthyl-aniline. On obtient, après recristallisation dans un mélange acétate d'éthyle-cyclohexane (50-50 en volumes), 4,5 g de N-(fluoro-4 phényl) N-méthyl (N-phényl phtalimido-2 acétamido)-2 acétamide fondant à 195°C.

La fluoro-4 N-méthyl-aniline peut être préparée d'une manière analogue à celle décrite à l'exemple 65, pour la préparation de la fluoro-2 N-méthyl-aniline, mais à partir de 23,5 g de fluoro-4 formanilide et de 14 g d'hydrure de lithium-aluminium. On obtient ainsi, 21 g de fluoro-4 N-méthyl aniline sous forme d'une huile utilisée telle quelle dans les synthèses ultérieures.

Le fluoro-4 formanilide peut être préparé d'une manière analogue à celle décrite à l'exemple 65 pour la préparation du fluoro-2 formanilide, mais à partir de 33 g de fluoro-4 aniline, de 33,6 g de méthylate de sodium et de 250 cm3 de diméthylformamide. On obtient ainsi, 23,5 g de fluoro-4 formanilide fondant à 60°C.

### EXEMPLE 67

On opère d'une manière analogue à celle décrite à l'exemple 1, mais à partir de 9,0 g d'(amino-2 N-phényl-acétamido)-2 N-(fluoro-3 phényl) N-méthyl-acétamide et de 3,8 g d'isocyanate de méthyl-3 phényle. Le produit brut obtenu est purifié par chromatographie sur 200 g de silice (0,065-0,200mm) contenus dans une colonne de 3,5 cm de diamètre [éluant : dichlorométhane-méthanol (99-1 en volumes) en recueillant des fractions de 40 cm3. Les fractions 26 à 46 sont réunies et concentrées à sec sous pression réduite (2,7kPa) à 40°C. On obtient, après recristallisation dans l'acétonitrile, 3,7 g de N-(fluoro-3 phényl) N-méthyl {[(méthyl-3 phényl)-3 uréido]-2 N-phényl-acétamido}-2 acétamide fondant à 180°C.

L'(amino-2 N-phényl-acétamido)-2 N-(fluoro-3 phényl) N-méthyl-acétamide peut être préparé d'une manière analogue à celle décrite à l'exemple 1, pour la préparation de l'(amino-2 N-phényl-acétamido)-2 N-phényl-acétamide, mais à partir de 14,7 g de N-(fluoro-3 phényl) N-méthyl (N-phényl phtalimido-2 acétamido)-2 acétamide et de 3,5 g d'hydrate d'hydrazine. On obtient ainsi, 9,0 g d'(amino-2 N-phényl-acétamido)-2 N-(fluoro-3 phényl) N-méthyl-acétamide sous forme d'une huile utilisée telle quelle dans les synthèses ultérieures.

Le N-(fluoro-3 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 12,0 g d'acide (N-phényl phtalimido-2 acétamido)-2 acétique, de 5,4 g de dichlorure d'oxalyle et de 9,1 g de fluoro-3 N-méthyl-aniline. On obtient, après recristallisation dans un mélange acétate d'éthyle-cyclohexane (50-50 en volumes), 14,7 g de N-(fluoro-3 phényl) N-méthyl (N-phényl phtalimido-2 acétamido)-2 acétamide fondant à 188°C.

La fluoro-3 N-méthyl-aniline peut être préparée d'une manière analogue à celle décrite à l'exemple 65 pour la préparation de la fluoro-2 N-méthyl-aniline, mais à partir de 12,6 g de fluoro-3 formanilide et de 5,2 g d'hydrure de lithium-aluminium. On obtient ainsi, 9,1 g de fluoro-3 N-méthyl-aniline sous forme d'une huile utilisée telle quelle dans les synthèses ultérieures.

Le fluoro-3 formanilide peut être préparé d'une manière analogue à celle décrite à l'exemple 65 pour la préparation du fluoro-2 formanilide, mais à partir de 11 g de fluoro-3 aniline, de 10,8 g de méthylate de sodium et de 100 cm3 de diméthylformamide. On obtient ainsi 12,6 g de fluoro-3 formanilide sous forme d'une huile utilisée telle quelle dans les synthèses ultérieures.

### EXEMPLE 68

On opère d'une manière analogue à celle décrite à l'exemple 1, mais à partir de 3,8 g d'(amino-2 N-phényl-acétamido)-2 N-(difluoro-3,4 phényl) N-méthyl-acétamide et de 1,4 g d'isocyanate de méthyl-3 phényle. Le produit brut obtenu est purifié par chromatographie sur 80 g de silice (0,065-0,200mm) contenus dans une colonne de 2,5 cm de diamètre [éluant : acétate d'éthyle-cyclohexane (75-25 en volumes)] en recueillant des fractions de 20 cm3. Les fractions 3 à 8 sont réunies et concentrées à sec sous pression réduite (2,7kPa) à 40°C. On obtient après recristallisation dans un mélange cyclohexane-acétate d'éthyle (50-50 en volumes) 0,5 g de N-(difluoro-3,4 phényl) N-méthyl {[(méthyl-3 phényl)-3 uréido]-2 N-phényl-acétamido}-2 acétamide fondant à 163°C.

L'(amino-2 N-phényl-acétamido)-2 N-(difluoro-3,4 phényl) N-méthyl-acétamide peut être préparé d'une manière analogue à celle décrite à l'exemple 1, pour la préparation de l'(amino-2 N-phényl-acétamido)-2 N-phényl-acétamide, mais à partir de 5,0 g de N-(difluoro-3,4 phényl) N-méthyl (N-phényl phtalimido-2 acétamido)-2 acétamide et de 1,0 g d'hydrate d'hydrazine. On obtient ainsi, 3,8 g d'(amino-2 N-phényl-acétamido)-2 N-(difluoro-3,4 phényl) N-méthyl-acétamide sous forme d'une huile utilisée telle quelle dans les synthèse ultérieures.

Le N-(difluoro-3,4 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide , mais à partir de 13,8 g d'acide (N-phényl phtalimido-2 acétamido)-2 acétique, de 5,9 g de dichlorure d'oxalyle et de 11,4 g de difluoro-3,4 N-méthyl-aniline. On obtient, après recristallisation dans l'acétonitrile, 5,5 g de N-(difluoro-3,4 phényl) N-méthyl (N-phényl phtalimido-2 acétamido)-2 acétamide fondant à 205°C.

La difluoro-3,4 N-méthyl-aniline peut être préparée d'une manière analogue à celle décrite à l'exemple 65 pour la préparation de la fluoro-2 N-méthyl-aniline, mais à partir de 14,2 g de difluoro-3,4 formanilide et de 3,8 g d'hydrure de lithium-aluminium. On obtient ainsi, 11,4 g de difluoro-3,4 N-méthyl-aniline sous forme d'une huile brune utilisée telle quelle dans les synthèses ultérieures.

Le difluoro-3,4 formanilide peut être préparé d'une manière analogue à celle décrite à l'exemple 65 pour la préparation du fluoro-2 formanilide, mais à partir de 12,8 g de difluoro-3,4 aniline, de 10,8 g de méthylate de sodium et de 100 cm3 de diméthylformamide. On obtient ainsi, 14,2 g de difluoro-3,4 formanilide sous forme d'une huile utilisée telle quelle dans les synthèses ultérieures.

### EXEMPLE 69

On opère d'une manière analogue à celle décrite à l'exemple 1, mais à partir de 3,5 g d'(amino-2 N-phényl-acétamido)-2 N-(difluoro-2,5 phényl) N-méthyl-acétamide et de 1,3 g d'isocyanate de méthyl-3 phényle. Le produit brut obtenu est purifié par chromatographie sur 60 g de silice (0,065-0,200mm) contenus dans une colonne de 2,5 cm de diamètre [éluant : acétate d'éthyle-cyclohexane (70-30 en volumes)] en recueillant des fractions de 20 cm3. Les fractions 3 à 13 sont réunies et concentrées à sec sous pression réduite (2,7kPa) à 40°C. On obtient, après recristallisation dans l'oxyde de diéthyle, 2,4 g de N-(difluoro-2,5 phényl) N-méthyl{[(méthyl-3 phényl)-3 uréido]-2 N-phényl-acétamido}-2 acétamide fondant à 118°C.

L'(amino-2 N-phényl-acétamido)-2 N-(difluoro-2,5 phényl) N-méthyl-acétamide peut être préparé d'une manière analogue à celle décrite à l'exemple 1, pour la préparation de l'(amino-2 N-phényl-acétamido)-2 N-phényl-acétamide, mais à partir de 5,0 g de N-(difluoro-2,5 phényl) N-méthyl (N-phényl phtalimido-2 acétamido)-2 acétamide et de 1,0 g d'hydrate d'hydrazine. On obtient ainsi, 3,5 g d'(amino-2 N-phényl-acétamido)-2 N-(difluoro-2,5 phényl) N-méthyl-acétamide sous forme d'une huile utilisée telle quelle dans les synthèses ultérieures.

Le N-(difluoro-2,5 phényl) N-méthyl (N-phényl phtalimido-2 acétamido)-2 acétamide peut être préparé d'une manière analogue à celle décrite à l'exemple 1, pour la préparation du (N-phényl phtalimido-2 acétamido)-2 N-phényl-acétamide, mais à partir de 12,8 g d'acide (N-phényl phtalimido-2 acétamido)-2 acétique, de 5,9 g de dichlorure d'oxalyle et de 11,0 g de de difluoro-2,5 N-méthyl aniline. On obtient après recristallisation dans l'acétonitrile, 7,6 g de N-(difluoro-2,5 phényl) N-méthyl (N-phényl phtalimido-2 acétamido)-2 acétamide fondant à 220°C.

La difluoro-2,5 N-méthyl-aniline peut être préparée d'une manière analogue à celle décrite à l'exemple 65 pour la préparation de la fluoro-2 N-méthyl aniline, mais à partir de 16,6 g de difluoro-2,5 formanilide et de 4,8 g d'hydrure de lithium-aluninium. On obtient ainsi, 11,0 g de difluoro-2,5 N-méthyl-aniline sous forme d'une huile utilisée telle quelle dans les synthèses ultérieures.

Le difluoro-2,5 formanilide peut être préparé d'une manière analogue à celle décrite à l'exemple 65 pour la préparation du fluoro-2 formanilide, mais à partir de 12,8 g de difluoro-2,5 aniline, de 10,8 g de méthylate de sodium et de 100 cm3 de diméthylformamide. On obtient ainsi, 16,6 g de difluoro-2,5 formanilide sous forme d'une huile utilisée telle quelle dans les synthèses ultérieures.

### EXEMPLE 70

On opère d'une manière analogue à celle décrite à l'exemple 40, mais à partir de 2,1 g d'acide [(méthyl-3 phényl)-3 uréido]-2 acétique, de 3,0 g d'anilino-2 phénylcarbamoyl-2 acétate d'éthyle-(RS) et de 0,7 cm3 de chlorure de thionyle. Le produit brut obtenu est chromatographié sur 60 g de silice (0,063-0,200mm) contenus dans une colonne de 2 cm de diamètre [éluant : dichlorométhane (100 cm3) puis dichlorométhane-méthanol (99-1 en volumes)] en recueillant des fractions de 20 cm3. Les fractions 54 à 80 sont réunies et concentrées à sec sous presion réduite (2,7kPa) à 60°C. On obtient, après recristallisation dans 300 cm3 d'éther de pétrole, 1,9 g de {[(méthyl-3 phényl-3 uréido]-2 N-phényl-acétamido}-2 phénylcarbamoyl-2 acétate d'éthyle-(RS) fondant à 125°C.

L'anilino-2 phénylcarbamoyl-2 acétate d'éthyle-(RS) peut être préparé d'une manière analogue à celle décrite à l'exemple 42 pour la préparation de l'anilino-2 [(pyrrolidinyl-1) carbonyl]-2 acétate de tert-butyle-(RS), mais à partir de 5,7 g de bromo-2 phénylcarbamoyl-2 acétate d'éthyle-(RS) et de 3,7 g d'aniline dans 40 cm3 d'acétonitrile. On obtient ainsi 6,2 g d'anilino-2 phénylcarbamoyl-2 acétate d'éthyle-(RS) à l'état d'huile utilisée telle quelle dans les synthèses ultérieures.

Le bromo-2 phénylcarbamoyl-2 acétate d'éthyle-(RS) peut être préparé d'une manière analogue à celle décrite à l'exemple 42 pour le bromo-2 [(pyrrolidinyl-1) carbonyl]-2 acétate de tert-butyle-(RS), mais à partir de 10,4 g de phénylcarbamoyl-2 acétate d'éthyle, de 5,9 g d'acétamide et de 2,6 cm3 de brome dans 230 cm3 de chloroforme. On obtient ainsi, 11,5 g de bromo-2 phénylcarbamoyl-2 acétate d'éthyle-(RS) fondant à 90°C.

Le phénylcarbamoyl-2 acétate d'éthyle peut être préparé selon la méthode décrite par F. D. COATTAWAY et coll., J. Chem. Soc., 97, 939 (1910).

### EXEMPLE 71

On opère comme à l'exemple 40, mais à partir de 2,5 g d'acide [(méthyl-3 phényl)-3 uréido]-2 acétique, de 4,2 g d'anilino-2 [(diméthyl-3,3 pipéridino-1) carbonyl]-2 acétate de tert-butyle-(RS) et de 0,87 g de chlorure de thionyle. Le produit brut obtenu est chromatographié sur 130 g de silice (0,063-0,208mm) dans une colonne de 2,4 cm de diamètre [éluants : dichlorométhane (1000 cm3) puis dichlorométhane-méthanol (99-1 en volumes)] en recueillant des fractions de 20 cm3. Les fractions 55 à 99 sont réunies et concentrées à sec sous pression réduite (2,7kPa) à 60°C. On obtient après recristallisation dans 30 cm3 d'éther de pétrole, 0,5 g [(diméthyl-3,3 pipéridino-1) carbonyl]-2 {[(méthyl-3 phényl)-3 uréido]-2 N-phényl-acétamido}-2 acétate de tert-butyle-(RS) fondant à 130°C.

L'anilino-2 [(diméthyl-3,3 pipéridino-1) carbonyl]-2 acétate de tert-butyle-(RS) peut être préparé de la manière suivante : à une solution de 8,0 g de bromo-2 [(diméthyl-3,3 pipéridino-1) carbonyl]-2 acétate de tert-butyle-(RS) dans 3,4 cm3 de triéthylamine, on ajoute 2,2 g d'aniline et le mélange réactionnel est chauffé à 90°C pendant 5 heures puis à une température voisine de 20°C pendant 20 heures. La masse solide est traitée avec 250 cm3 d'oxyde de diéthyle, le produit insoluble est séparé par filtration, lavé par 2 fois 100 cm3 d'oxyde de diéthyle. Le filtrat est concentré à sec sous pression réduite (2,7kPa) à 50°C. On obtient ainsi 6,0 g d'anilino-2 [(diméthyl-3,3 pipéridino-1) carbonyl]-2 acétate de tert-butyle-(RS) fondant à 70°C.

Le bromo-2 [(diméthyl-3,3 pipéridino-1) carbamoyl]-2 acétate de tert-butyle-(RS) peut être préparé d'une manière analogue à celle décrite à l'exemple 42 pour le bromo-2 [(pyrrolidinyl-1) carbonyl]-2 acétate de tert-butyle-(RS), mais à partir de 10,2 g de [(diméthyl-3,3 pipéridino-1) carbonyl]-2 acétate de tert-butyle, de 4,7 g d'acétamide et de 2,05 cm3 de brome dans 150 cm3 de chloroforme. On obtient ainsi 10,8 g de bromo-2 [(diméthyl-3,3 pipéridino-1) carbonyl]-2 acétate de tert-butyle-(RS) sous forme d'une huile utilisée telle quelle dans les synthèses ultérieures.

Le [(diméthyl-3,3 pipéridino-1) carbonyl]-2 acétate de tert-butyle peut être préparé de la manière suivante : à une solution de 16 g de malonate acide de tert-butyle dans 450 cm3 de tétrahydrofuranne anhydre, on ajoute par portions 16,2 g de N,N'-diimidazole carbonyle. Le mélange réactionnel est agité à une température voisine de 20°C pendant 4 heures puis on ajoute 11,7 cm3 de diméthyl-3,3 pipéridine. Le mélange réactionnel est encore agité pendant 20 heures à une température voisine de 20°C puis dilué par 1 litre d'eau et extrait par 3 fois 200 cm3 de chlorure de méthylène. Les phases organiques sont réunies, lavées par 2 fois 100 cm3 d'eau, séchées sur sulfate de magnésium et concentrées à sec sous pression réduite (2,7kPa) à 60°C. On obtient ainsi 22,0 g de [(diméthyl-3,3 pipéridino-1) carbonyl]-2 acétate de tert-butyle sous forme d'une huile utilisée telle quelle dans les synthèses ultérieures.

### EXEMPLE 72

A une solution de 7,8 g d'{[(imidazolyl-1)carboxamido]-2 N-phényl-acétamido}-2 N-méthyl N-phényl-acétamide dans 100 cm3 de toluène, on ajoute 6,6 g d'amino-3 benzoate d'éthyle. Le mélange est agité à reflux pendant 2 heures, puis concentré à sec sous pression réduite (2,7 kPa) à 45°C. Le résidu est dissous dans 200 cm3 d'acétate d'éthyle et la solution obtenue est lavée par 50 cm3 d'une solution aqueuse 2N d'acide chlorhydrique puis par 2 fois 50 cm3 d'eau. La phase organique est séchée sur sulfate de magnésium et concentrée à sec sous pression réduite (2,7 kPa) à 40°C. Le produit brut obtenu est purifié par chromatographie sur 100 g de silice (0,065-0,200 mm) contenus dans une colonne de 2,5 cm de diamètre [éluant : acétate d'éthyle] en recueillant des fractions de 20 cm3. Les fractions 4 à 11 sont réunies et concentrées à sec sous pression réduite (2,7 kPa) à 40°C. On obtient, après recristallisation dans l'acétate d'éthyle, 3,8 g de {[N-(N-méthyl N-phényl-carbamoylméthyl) N-phényl-carbamoylméthyl]-3 uréido}-3 benzoate d'éthyle fondant à 174°C.

### EXEMPLE 73

On opère d'une manière analogue à celle décrite à l'exemple 72, mais à partir de 1,3 g d'{[(imidazolyl-1) carboxamido]-2 N-phényl-acétamido}-2 N-méthyl N-phényl-acétamide et de 0,78 g d'amino-3 benzonitrile. Le produit brut obtenu est purifié par chromatographie sur 25 g de silice (0,065-0,200 mm) contenus dans une colonne de 1,8 cm de diamètre [éluant dichlorométhane-acétate d'éthyle (50/50 en volumes)] en recueillant des fractions de 25 cm3. Les fractions 7 à 17 sont réunies et concentrées à sec sous pression réduite (2,7 kPa) à 40°C. On obtient, après recristallisation dans l'acétonitrile, 0,7 g de {[(cyano-3 phényl)-3 uréido]-2 N-phényl-acétamido}-2 N-méthyl N-phényl-acétamide fondant à 204°C.

### EXEMPLE 74

A une solution de 2,4 g de {[N-(N-méthyl N-phényl-carbamoylméthyl) N-phényl-carbamoylméthyl]-3 uréido}-3 benzoate d'éthyle dans 90 cm3 d'un mélange eau-tétrahydrofuranne-dioxanne (30/40/30 en volumes) on ajoute 5 cm3 d'une solution aqueuse N de soude. Le mélange est agité pendant 16 heures à une température voisine de 25°C, puis concentré à environ 50 cm3 sous pression réduite (2,7 kPa) à 40°C. La solution obtenue est diluée avec 50 cm3 d'eau, lavée par 2 fois 50 cm3 d'acétate d'éthyle, acidifiée à pH 3 avec une solution aqueuse 4N d'acide chlorhydrique et extraite par 2 fois 30 cm3 d'acétate d'éthyle. Les phases organiques sont réunies, lavées par 30 cm3 d'eau, séchées sur sulfate de magnésium et concentrées à sec sous pression réduite (2,7 kPa) à 30°C. On obtient, après recristallisation dans l'acétate d'éthyle, 1,4 g d'acide {[N-(N-méthyl N-phényl-carbamoylméthyl) N-phényl-carbamoylméthyl]-3 uréido}-3 benzoïque fondant à 232°C.

### EXEMPLE 75

On opère d'une manière analogue à celle décrite à l'exemple 72, mais à partir de 3 g d'{[(imidazolyl-1)carboxamido]-2 N-phényl-acétamido}-2 N-méthyl N-phényl-acétamide et de 3,3 g d'amino-3 benzophénone. On obtient, après recristallisation dans le dichlorométhane, 1,3 g de {[(benzoyl-3 phényl)-3 uréido]-2 N-phényl-acétamido}-2 N-méthyl N-phényl-acétamide fondant à 215°C.

L'amino-3 benzophénone peut être préparée selon la méthode décrite par G.J. ESSELEN, Jr et L. CLARKE, J. Amer. Chem. Soc., 36, 322 (1914).

### EXEMPLE 76

A une suspension de 2 g de {[(benzoyl-3 phényl)-3 uréido]-2 N-phényl-acétamido}-2 N-méthyl N-phényl-acétamide dans un mélange méthanol-tétrahydrofuranne-eau (70/28/2 en volumes), on ajoute en 1 heure 0,3 g de borohydrure de sodium. Le mélange est agité pendant 30 minutes à une température voisine de 25°C, puis concentré à sec sous pression réduite (2,7 kPa) à 35°C. Le résidu est dissous dans 50 cm3 de dichlorométhane et la solution obtenue est lavée par 30 cm3 d'une solution aqueuse 2N d'acide chlorhydrique, puis par 30 cm3 d'eau. La phase organique est séchée sur sulfate de magnésium puis concentrée à sec sous pression réduite (2,7 kpa) à 35°C. On obtient, après recristallisation dans un mélange acétate d'éthyle-oxyde de diéthyle (60/40 en volumes) 1,4 g d'{[(α-hydroxy benzyl-3 phényl)-3 uréido]-2 N-phényl-acétamido}-2 N-méthyl N-phényl-acétamide -(RS) fondant à 160°C.

### EXEMPLE 77

On opère de manière analogue à celle décrite à l'exemple 72, mais à partir de 3,5 g d'{[(imidazolyl-1) carboxamido]-2 N-phényl-acétamido}-2 N-méthyl N-phényl-acétamide et de 2,5 g de nitro-3 aniline. Le produit brut obtenu est purifié par chromatographie sur 60 g de silice (0,065-0,200 mm) contenus dans une colonne de 2,2 cm de diamètre [éluant : dichlorométhane-acétate d'éthyle (80/20 en volumes)] en recueillant des fractions de 20 cm3. Les fractions 12 à 20 sont réunies et concentrées à sec sous pression réduite (2,7 kPa) à 40°C. On obtient, après recristallisation dans un mélange acétate d'éthyle-acétonitrile (90/10 en volumes), 1,1 g de N-méthyl {[(nitro-3 phényl)-3 uréido]-2 N-phényl-acétamido}-2 N-phényl-acétamide fondant à 152°C.

### EXEMPLE 78

On opère d'une manière analogue à celle décrite à l'exemple 72, mais à partir de 2,95 g d'{[(imidazolyl-1) carboxamido]-2 N-phényl-acétamido}-2 N-méthyl N-phényl-acétamide et de 2,64 g d'(amino-3 phényl)-1 pipéridine. Le produit brut obtenu est purifié par chromatographie sur 35 g de silice (0,065-0,200 mm) contenus dans une colonne de 1,5 cm de diamètre [éluant : dichlorométhane-acétate d'éthyle (50/50 en volumes)] en recueillant des fractions de 25 cm3. Les fractions 20 à 26 sont réunies et concentrées à sec sous pression réduite (2,7 kPa) à 40°C. On obtient, après recristallisation dans un mélange acétonitrile-diméthylformamide (90/10 en volumes), 0,75 g de N-méthyl N-phényl {[(pipéridino-3 phényl)-3 uréido]-2 N-phényl-acétamido}-2 acétamide fondant à 235°C.

L'(amino-3 phényl)-1 pipéridine peut être préparée selon la méthode décrite par G. ADRIANT et C. GLACET, Bull. Soc. Chim. Fr., 1511 (1970).

### EXEMPLE 79

On opère d'une manière analogue à celle décrite à l'exemple 72, mais à partir de 2,9 g d'{[(imidazolyl-1) carboxamido]-2 N-phényl-acétamido}-2 N-méthyl N-phényl-acétamide et de 2,6 g d'amino-3 benzènesulfonamide. Le produit brut obtenu est purifié par chromatographie sur 45 g de silice (0,065-0,200 mm) contenus dans une colonne de 1,5 cm de diamètre (éluant : acétate d'éthyle) en recueillant des fractions de 25 cm3. Les fractions 3 à 7 sont réunies et concentrées à sec sous pression réduite (2,7 kPa) à 40°C. On obtient, après recristallisation dans un mélange acétonitrile-acétate d'éthyle (70/30 en volumes), 0,9 g de N-méthyl N-phényl {[(sulfamoyl-3 phényl)-3 uréido]-2 N-phényl-acétamido}-2 acétamide fondant à 175°C.

L'amino-3 benzènesulfonamide peut être préparé selon la méthode décrite par W.A. JACOBS et M. HEIDELBERGER, J. Am. Chem. Soc., 39, 2428 (1917).

### EXEMPLE 80

On opère d'une manière analogue à celle décrite à l'exemple 72, mais à partir de 1,96 g d'{[(imidazolyl-1) carboxamido]-2 N-phényl-acétamido}-2 N-méthyl N-phényl-acétamide et de 1,36 g d'amino-3 benzamide. On obtient, après recristallisation dans un mélange acétonitrile-acétate d'éthyle (55/45 en volumes), 1,25 g de {[N-(N-méthyl N-phényl-carbamoylméthyl) N-phényl-carbamoylméthyl]-3 uréido}-3 benzamide fondant à 170°C.

L'amino-3 benzamide peut être préparé selon la méthode décrite par W.A. JACOBS et M. HEIDELBERGER, J. Am. Chem. Soc., 39, 1438 (1917).

### EXEMPLE 81

A une solution de 3,0 g d'(amino-2 N-phényl-acétamido)-2 N-méthyl N-phényl-acétamide dans 50 cm3 de tétrahydrofuranne anhydre, on ajoute à une température voisine de 25°C, 1,64 g d'isocyanate de nitro-4 phényle. Le mélange est agité pendant 12 heures à une température voisine de 25°C et le produit insoluble est séparé par filtration. On obtient, après recristallisation dans l'acétate d'éthyle 3,2 g de N-méthyl {[(nitro-4 phényl)-3 uréido]-2 N-phényl-acétamido}-2 N-phényl-acétamide fondant à 215°C.

### EXEMPLE 82

A une solution de 1,8 g de N-méthyl {[(nitro-3 phényl)-3 uréido]-2 N-phényl-acétamido}-2 N-phényl-acétamide dans 100 cm3 d'éthanol on ajoute 0,3 g de palladium sur noir à 5 %. La suspension obtenue est agitée pendant 2 heures à une température voisine de 50°C sous atmosphère d'hydrogène (100 kPa). Après refroidissement, le catalyseur est séparé par filtration et la solution est concentrée à sec sous pression réduite (2,7 kPa) à 35°C. Le résidu obtenu est dissous dans 60 cm3 d'un mélange acétonitrile-oxyde de diisopropyle (20/80 en volumes) et on ajoute 5 cm3 d'une solution 3,5N d'acide chlorhydrique dans l'oxyde de diisopropyle. Le produit insoluble formé est séparé par filtration, séché à l'air et recristallisé dans l'éthanol. On obtient ainsi 0,9 g de chlorhydrate d'{[(amino-3 phényl)-3 uréido]-2 N-phényl-acétamido}-2 N-méthyl N-phényl-acétamide fondant à 220°C.

### EXEMPLE 83

On opère d'une manière analogue à celle décrite à l'exemple 72, mais à partir de 1,28 g d'{[(imidazolyl-1) carboxamido]-2 N-phényl-acétamido}-2 N-méthyl N-phényl-acétamide et de 1,05 g d'acide amino-5 salicylique. Le produit brut obtenu est purifié par chromatographie sur 40 g de silice (0,065-0,200 mm) contenus dans une colonne de 3 cm de diamètre [éluant : tétrahydrofuranne-eau (99/1 en volumes)] en recueillant des fractions de 25 cm3. Les fraction 2 à 4 sont réunies et concentrées à sec sous pression réduite (2,7 kPa) à 40°C. On obtient, après recristallisation dans un mélange eau-acétonitrile (55/45 en volumes), 0,18 g d'acide {[N-(N-méthyl N-phényl-carbamoylméthyl) N-phényl-carbamoylméthyl]-3 uréido]-5 salicylique fondant à 189°C.

### EXEMPLE 84

A une solution de 0,8 g d'{[(hydroxy-1 éthyl)-3 phényl]-3 uréido}-2 N-[oxo-2 (tétrahydro-1,2,3,4 quinolyl-1)-2 éthyl] N-phényl-acétamide-(RS) dans 50 cm3 de chlorure de méthylène, on ajoute en 2 fois, à 20 heures d'intervalle, 2,3 g de dioxyde de manganèse. Le mélange est agité à une température voisine de 25°C pendant 70 heures. Le produit insoluble est séparé par filtration, et le filtrat est concentré à sec sous pression réduite (2,7 kPa) à 40°C. On obtient, après recristallisation dans l'oxyde de diisopropyle, 0,3 g d'[(acétyl-3 phényl)-3 uréido]-2 N-[oxo-2 (tétrahydro-1,2,3,4 quinolyl-1)-2 éthyl] N-phényl-acétamide fondant à 130°C.

### EXEMPLE 85

On opère d'une manière analogue à celle décrite à l'exemple 84, mais à partir de 1,4 g d'{{[(hydroxy-1 éthyl)-3 phényl]-3 uréido}-2 N-phényl-acétamido}-2 N-méthyl N-phényl-acétamide-(RS) et de 4,7 g de dioxyde de manganèse. On obtient, après recristallisation dans l'acétate d'éthyle, 0,65 g d'{[(acétyl-3 phényl)-3 uréido]-2 N-phényl-acétamido}-2 N-méthyl N-phényl-acétamide fondant à 170°C.

### EXEMPLE 86

A une solution de 1,1 g d'{[(acétyl-3 phényl)-3 uréido]-2 N-phényl-acétamido}-2 N-méthyl N-phényl-acétamide dans 10 cm3 d'éthanol, on ajoute 2 cm3 d'eau distillée et 0,17 g de chlorhydrate d'hydroxylamine. Le mélange est agité à reflux pendant 3 heures. Après refroidissement, le produit insoluble est séparé par filtration, lavé par 3 fois 10 cm3 d'eau et séché à l'air. On obtient, après recristallisation dans le méthanol, 0,45 g d'{{[(hydroxyimino-1 éthyl)-3 phényl]-3 uréido}-2 N-phényl-acétamido}-2 N-méthyl N-phényl-acétamide-(E) fondant à 170°C.

### EXEMPLE 87

A une solution de 1,1 g de [(méthylthio-3 phényl)-3 uréido]-2 N-[oxo-2 (tétrahydro-1,2,3,4 quinolyl-1)-2 éthyl] N-phényl-acétamide dans 10 cm3 de chlorure de méthylène, maintenue à une température voisine de 0°C, on coule en 10 minutes 1,5 g d'acide m.chloroperbenzoïque en solution dans 10 cm3 de chlorure de méthylène. Le mélange est agité pendant 2 heures à une température voisine de 25°C, puis dilué par 25 cm3 de chlorure de méthylène. La solution est lavée par 20 cm3 d'une solution aqueuse saturée de thiosulfate de sodium, 20 cm3 d'une solution aqueuse saturée d'hydrogénocarbonate de sodium, puis 20 cm3 d'une solution aqueuse saturée de chlorure de sodium. La phase organique est séchée sur sulfate de magnésium et concentrée à sec sous pression réduite (2,7 kPa) à 40°C. Le produit brut obtenu est purifié par chromatographie sur 40 g de silice (0,062-0,200 mm) contenus dans une colonne de 1,2 cm de diamètre [éluant : acétate d'éthyle-méthanol (95/5 en volumes)] en recueillant des fractions de 10 cm3. Les fractions 6 à 13 sont réunies et concentrées à sec sous pression réduite (2,7 kPa) à 40°C. On obtient, après recristallisation dans un mélange oxyde de diisopropyle-acétate d'éthyle (90/10 en volumes) 0,5 g de [(méthylsulfinyl-3 phényl)-3 uréido]-2 N-[oxo-2 (tétrahydro-1,2,3,4 quinolyl-1)-2 éthyl] N-phényl-acétamide-(RS) fondant à 140°C.

### EXEMPLE 88

A une solution de 0,92 g d'{[(acétyl-3 phényl)-3 uréido]-2 N-phényl-acétamido}-2 N-méthyl N-phényl-acétamide dans 10 cm3 d'éthanol on ajoute 0,16 g de chlorhydrate de 0-méthyl hydroxylamine en solution dans 2 cm3 d'eau. Le mélange est agité à reflux pendant 3 heures, puis à une température voisine de 25°C pendant 12 heures et concentré à un volume d'environ 3 cm3 sous pression réduite (2,7 kPa) à 40°C. On ajoute alors 20 cm3 d'eau distillée et le produit insoluble est séparé par filtration et séché à l'air. On obtient après recristallisation dans un mélange méthanol-oxyde de diisopropyle (60/40 en volumes) 0,31 g de {{[(méthoxyimino-1 éthyl)-3 phényl]-3 uréido}-2 N-phényl-acétamido}-2 N-méthyl N-phényl-acétamide-(Z) fondant à 165°C.

### EXEMPLE 89

A une solution de 1 g de {[(formyl-3 phényl)-3 uréido]-2 N-phényl-acétamido}-2 N-méthyl N-phényl-acétamide dans 6 cm3 de méthanol, on ajoute 0,18 g de pyridine puis 0,17 g de chlorhydrate d'hydroxylamine en solution dans 3 cm3 d'eau. Le mélange est chauffé à reflux pendant 3 heures. Après refroidissement, le produit insoluble est séparé par filtration, lavé par 3 fois 3 cm3 d'eau et séché à l'air. On obtient, après recristallisation dans un mélange diméthylformamide-eau (50/50 en volumes) 0,5 g d'{[(hydroxyiminométhyl-3 phényl)-3 uréido]-2 N-phényl-acétamido}-2 N-méthyl N-phényl-acétamide-(E) fondant à 219°C.

Le {[(formyl-3 phényl)-3 uréido]-2 N-phényl-acétamido}-2 N-méthyl N-phényl-acétamide peut être préparé d'une manière analogue à celle décrite à l'exemple 84 pour la préparation de l'[(acétyl-3 phényl)-3 uréido]-2 N-[oxo-2 (tétrahydro-1,2,3,4 quinolyl-1)-2 éthyl] N-phényl-acétamide, mais à partir de 1,3 g d'{[(hydroxyméthyl-3 phényl)-3 uréido]-2 N-phényl-acétamido}-2 N-méthyl N-phényl-acétamide et de 2,7 g de dioxyde de manganèse. Le produit brut obtenu est purifié par chromatographie sur 25 g de silice (0,065-0,200 mm) contenus dans une colonne de 1,8 cm de diamètre (éluant : acétate d'éthyle) en recueillant des fractions de 25 cm3. Les fractions 3 à 7 sont réunies et concentrées à sec sous pression réduite (2,7 kPa) à 40°C. On obtient ainsi 1,1 g de {[(formyl-3 phényl)-3 uréido]-2 N-phényl-acétamido}-2 N-méthyl N-phényl-acétamide sous forme d'une meringue utilisée telle quelle dans les synthèses ultérieures.

### EXEMPLE 90

A une solution de 1,38 g d'{{[(hydroxy-1 éthyl)-3 phényl]-3 uréido}-2 N-phényl-acétamido}-2 N-méthyl N-phényl-acétamide-(RS) dans 30 cm3 de chlorure de méthylène maintenue à une température voisine de 5°C, on ajoute 0,30 g de triéthylamine puis 0,24 g de chlorure d'acétyle. Le mélange est agité à une température voisine de 5°C pendant 1 heure, puis à une température voisine de 25°C pendant 12 heures. La solution obtenue est lavée par 2 fois 10 cm3 d'eau. La phase organique est séchée sur sulfate de magnésium et concentrée à sec sous pression réduite (2,7 kPa) à 40°C. Le produit brut obtenu est purifié par chromatographie sur 60 g de silice (0,065-0,200 mm) contenus dans une colonne de 2,5 cm de diamètre [(éluant : chlorure de méthylène-acétate d'éthyle (50/50 en volumes)] en recueillant des fractions de 25 cm3. Les fractions 6 à 11 sont réunies et concentrées à sec sous pression réduite (2,7 kPa) à 40°C. On obtient, après recristallisation dans un mélange acétate d'éthyle-oxyde de diéthyle (70/30 en volumes), 0,32 g d'acétate de {{[N-(N-méthyl N-phénylcarbamoylméthyl) N-phényl-carbamoylméthyl]-3 uréido}-3 phényl-1} éthanol-(RS) fondant à 168°C.

### EXEMPLE 91

On opère d'une manière analogue à celle décrite à l'exemple 72, mais à partir de 1,96 g d'{[(imidazolyl-1) carboxamido]-2 N-phényl-acétamido}-2 N-méthyl N-phényl-acétamide et de 1,5 g de (méthoxy-1 éthyl)-3 aniline-(RS). On obtient, après recristallisation dans l'acétonitrile, 0,71 g de {{[(méthoxy-1 éthyl)-3 phényl]-3 uréido}-2 N-phényl-acétamido}-2 N-méthyl N-phényl-acétamide-(RS) fondant à 194°C.

La (méthoxy-1 éthyl)-3 aniline-(RS) peut être préparée de la manière suivante : à une solution de 20 g d'(hydroxy-1 éthyl)-3 aniline-(RS) dans 300 cm3 de diméthylformamide anhydre, maintenue à une température voisine de 10°C, on ajoute en 5 minutes 7 g d'une suspension huileuse (50 % en poids) d'hydrure de sodium. Le mélange est agité pendant 30 minutes à une température voisine de 10°C, puis on y ajoute en 10 minutes 20,7 g d'iodure de méthyle. Le mélange est alors agité à une température voisine de 40°C pendant 2 heures, refroidi puis versé dans 500 cm3 d'eau. La phase aqueuse est extraite par 3 fois 250 cm3 d'acétate d'éthyle, puis les phases organiques réunies sont lavées par 2 fois 200 cm3 d'eau, séchées sur sulfate de magnésium et concentrées à sec sous pression réduite (2,7 kPa) à 40°C. Le résidu brut obtenu est purifié par chromatographie sur 300 g de silice (0,065-0,200 mm) contenus dans une colonne de 4,2 cm de diamètre (éluant : chlorure de méthylène-acétate d'éthyle (50/50 en volumes)) en recueillant des fractions de 50 cm3. Les fractions 7 à 12 sont réunies et concentrées à sec sous pression réduite (2,7 kPa) à 40°C. On obtient ainsi 5 g de (méthoxy-1 éthyl)-3 aniline-(RS) sous forme d'une huile utilisée telle quelle dans les synthèses ultérieures.

### EXEMPLE 92

On opère d'une manière analogue à celle décrite à l'exemple 72, mais à partir de 4,0 g de N-[(diméthyl-3,3 pipéridino)-2 oxo-2 éthyl] [(imidazolyl-1) carboxamido]-2 N-phényl-acétamide et de 3,3 g d'amino-3 benzoate d'éthyle. Le produit brut est purifié par chromatographie sur 50 g de silice (0,065-0,200 mm) contenus dans une colonne de 2,0 cm de diamètre [éluant : chlorure de méthylène-acétate d'éthyle (40/60 en volumes)] en recueillant des fractions de 20 cm3. Les fractions 5 à 13 sont réunies et concentrées à sec sous pression réduite (2,7 kPa) à 40°C. On obtient, après recristallisation dans un mélange acétate d'éthyle-oxyde de diisopropyle (60/40 en volumes), 2,6 g de {{N-[(diméthyl-3,3 pipéridino)-2 oxo-2 éthyl] N-phényl-carbamoylméthyl}-3 uréido}-3 benzoate d'éthyle fondant à 164°C.

### EXEMPLE 93

On opère d'une manière analogue à celle décrite à l'exemple 74, mais à partir de 2,0 g de {{N-[(diméthyl-3,3 pipéridino)-2 oxo-2 éthyl] N-phényl-carbamoylméthyl]-3 uréido}-3 benzoate d'éthyle et de 4 cm3 d'une solution aqueuse de soude 1N. On obtient, après recristallisation dans un mélange acétate d'éthyle-diméthylformamide (90/10 en volumes) 1,1 g d'acide {{N-(diméthyl-3, 3 pipéridino)-2 oxo-2 éthyl] N-phényl-carbamoylméthyl}-3 uréido}-3 benzoïque fondant à 270°C.

### EXEMPLE 94

On opère d'une manière analogue à celle décrite à l'exemple 74, mais à partir de 1,5 g de {[N-(N-méthyl N-phényl-carbamoylméthyl) N-phényl-carbamoylméthyl]-3 uréido}-3 phénylacétate d'éthyle et de 3 cm3 d'une solution aqueuse de soude 1N. On obtient, après recristallisation dans l'acétate d'éthyle, 0,9 g d'acide {[N-(N-méthyl N-phényl-carbamoylméthyl) N-phényl-carbamoylméthyl]-3 uréido}-3 phénylacétique fondant à 228°C.

Le {[N-(N-méthyl N-phényl-carbamoylméthyl) N-phényl-carbamoylméthyl]-3 uréido}-3 phénylacétate d'éthyle peut être préparé d'une manière analogue à celle décrite à l'exemple 72 pour la préparation du {[N-méthyl N-phényl-carbamoylméthyl) N-phényl-carbamoylméthyl]-3 uréido}-3 benzoate d'éthyle mais à partir de 1,75 g d'{[(imidazolyl-1) carboxamido]-2 N-phényl-acétamido}-2 N-méthyl N-phényl-acétamide et de 1,6 g d'amino-3 phénylacétate d'éthyle. Le produit brut obtenu est purifié par chromatographie sur 30 g de silice (0,065-0,200 mm) contenus dans une colonne de 1,8 cm de diamètre [éluant : dichlorométhane-acétate d'éthyle (50/50 en volumes)] en recueillant des fractions de 20 cm3 . Les fractions 13 à 23 sont réunies et concentrées à sec sous pression réduite (2,7 kPa) à 40°C. On obtient ainsi, 1,5 g de {[N-(N-méthyl N-phényl-carbamoylméthyl) N-phényl-carbamoylméthyl]-3 uréido}-3 phénylacétate d'éthyle sous forme d'une huile utilisée telle quelle dans les synthèses ultérieures.

L'amino-3 phénylacétate d'éthyle peut être préparé de la manière suivante : à une solution de 2,0 g de nitro-3 phénylacétate d'éthyle dans 20 cm3 d'éthanol, on ajoute 0,1 g de palladium sur noir à 5%. La suspension est agitée pendant 2 heures à une température voisine de 25°C sous atmosphère d'hydrogène (100 kPa). Le catalyseur est ensuite séparé par filtration et le filtrat est concentré à sec sous pression réduite (2,7 kPa) à 40°C. On obtient ainsi 1,7 g d'amino-3 phénylacétate d'éthyle sous forme d'une huile utilisée telle quelle dans les synthèses ultérieures.

Le nitro-3 phénylacétate d'éthyle peut être préparé selon la méthode décrite par SEGERS et A. BRUYLANTS, Bul. Soc. Chim. Belg., 64, 87 (1955).

### EXEMPLE 95

A une suspension de 1,5 g de {[(N-(N-méthyl N-phénylcarbamoylméthyl) N-phényl-carbamoylméthyl]-3 uréido}-3 phénoxy acétate de tert-butyle dans 20 cm3 de dichloro-1,2 éthane, on ajoute 2,5 g d'acide trifluoroacétique et le mélange est chauffé à reflux pendant une heure, puis laissé pendant 12 heures à une température voisine de 25°C et concentré à sec sous pression réduite (2,7 kPa) à 40°C. Le résidu obtenu est purifié par chromatographie sur 40 g de silice (0,065-0,200 mm) contenus dans une colonne de 3 cm de diamètre [éluant : chlorure de méthylène-méthanol (80/20 en volumes)] en recueillant des fractions de 20 cm3. Les fractions 6 à 17 sont réunies et concentrées à sec sous pression réduite (2,7 kPa) à 40°C. Après dissolution du solide obtenu dans 10 cm3 d'une solution aqueuse de soude 4N et filtration de la solution obtenue, on ajoute 10 cm3 d'une solution aqueuse d'acide chlorhydrique 4N. Le produit insoluble est séparé par filtration, lavé par 5 fois 5 cm3 d'eau et séché à l'air. On obtient, ainsi 0,55 g d'acide {[N-(N-méthyl N-phényl-carbamoylméthyl) N-phényl-carbamoylméthyl]-3 uréido}-3 phénoxyacétique fondant à 130°C.

Le {[N-(N-méthyl N-phényl-carbamoylméthyl) N-phényl-carbamoylméthyl]-3 uréido}-3 phénoxyacétate de tert-butyle peut être préparé d'une manière analogue à celle décrite à l'exemple 72, pour la préparation du {[N-(N-méthyl N-phényl-carbamoylméthyl) N-phényl carbamoylméthyl]-3 uréido-}-3 benzoate d'éthyle, mais à partir de 3,5 g d'{[(imidazolyl-1) carboxamido]-2 N-phényl-acétamido}-2 N-méthyl N-phényl-acétamide et de 4 g d'amino-3 phénoxyacétate de tert-butyle. Le produit brut obtenu est purifié par chromatographie sur 90 g de silice (0,065-0,200 mm) contenus dans une colonne de 2,5 cm de diamètre [éluant : chlorure de méthylène-acétate d'éthyle (80/20 en volumes)] en recueillant des fractions de 25 cm3. Les fractions 15 à 29 sont réunies et concentrées à sec sous pression réduite (2,7 kPa) à 40°C. On obtient, après recristallisation dans l'isopropanol, 1,3 g de {[N-(N-méthyl N-phényl-carbamoylméthyl) N-phényl-carbamoylméthyl]-3 uréido}-3 phénoxyacétate de tert-butyle fondant à 174°C.

L'amino-3 phénoxyacétate de tert-butyle peut être préparé de la manière suivante : à une solution de 5,4 g de nitro-3 phénoxy acétate de tert-butyle dans 50 cm3 d'éthanol, on ajoute 0,2 g de palladium sur noir à 5 %. La suspension est agitée pendant 2 heures à une température voisine de 25°C sous atmosphère d'hydrogène (100 kPa). Le catalyseur est ensuite séparé par filtration et le filtrat est concentré à sec sous pression réduite (2,7kPa) à 40°C. On obtient ainsi 4,8 g d'amino-3 phénoxyacétate de tert-butyle sous forme d'une huile utilisée telle quelle dans les synthèses ultérieures.

Le nitro-3 phénoxyacétate de tert-butyle peut être préparé de la manière suivante : à une solution de 4,17 g de nitro-3 phénol dans 50 cm3 de diméthylformamide anhydre, on ajoute en 10 minutes, 1,44 g d'une suspension huileuse (50 % en poids) d'hydrure de sodium. Le mélange obtenu est agité à une température voisine de 25°C pendant 30 minutes, puis on ajoute en 10 minutes 5,85 g de bromoacétate de tert-butyle. Le mélange est agité pendant 2 heures à une température voisine de 25°C, puis versé dans 150 cm3 d'eau et extrait par 2 fois 50 cm3 d'acétate d'éthyle. Les phases organiques réunies sont séchées sur sulfate de magnésium et concentrées à sec sous pression réduite (2,7 kPa) à 35°C. On obtient, après recristallisation dans le cyclohexane, 5,4 g de nitro-3 phénoxy acétate de tert-butyle fondant à 52°C.

### EXEMPLE 96

On opère d'une manière analogue à celle décrite à l'exemple 74, mais à partir de 0,8 g de {{N-[oxo-2 (tétrahydro-1,2,3,4 quinolyl-1)-2 éthyl] N-phényl-carbamoylméthyl}-3 uréido}-3 benzoate d'éthyle et de 1,55 cm3 d'une solution aqueuse de soude 1N. Le produit brut obtenu est dissous dans 10 cm3 d'une solution aqueuse 1N de soude. La solution est lavée par 10 cm3 d'acétate d'éthyle, puis acidifiée à pH 1 avec 11 cm3 d'une solution aqueuse d'acide chlorhydrique 1N. Le produit insoluble est séparé par filtration, lavé par 3 fois 2 cm3 d'eau et séché à l'air. On obtient ainsi, 0,35 g d'acide {{N-[oxo-2(tétrahydro-1,2,3,4 quinolyl-1)-2 éthyl] N-phényl-carbamoylméthyl}-3 uréido}-3 benzoïque fondant à 280°C.

### EXEMPLE 97

On opère d'une manière analogue à celle décrite à l'exemple 72, mais à partir de 3 g d'[(imidazolyl-1) carboxamido]-2 N-[oxo-2 (tétrahydro-1,2,3,4 quinolyl-1)-2 éthyl] N-phényl acétamide et de 2,4 g d'amino-3 benzoate d'éthyle. Le produit brut obtenu est purifié par chromatographie sur 80 g de silice (0,065-0,200 mm) contenus dans une colonne de 2,5 cm de diamètre [éluant : chlorure de méthylène-acétate d'éthyle (40/60 en volumes)) en recueillant des fractions de 25 cm3. Les fractions 8 à 16 sont réunies et concentrées à sec sous pression réduite (2,7 kPa) à 40°C. On obtient, après recristallisation dans un mélange acétate d'éthyle-oxyde de diisopropyle (70/30 en volumes), 1,2 g de {{N-[oxo-2 (tétrahydro-1,2,3,4 quinolyl-1)-2 éthyl] N-phényl-carbamoylméthyl}-3 uréido}-3 benzoate d'éthyle fondant à 174°C.

### EXEMPLE 98

On opère d'une manière analogue à celle décrite à l'exemple 72, mais à partir de 1,74 g de N-[(diméthyl-3,3 pipéridino)-2 oxo-2 éthyl] [(imidazolyl-1) carboxamido]-2 N-phényl-acétamide et de 1 g d'(hydroxyimino-1 éthyl)-3 aniline. Le produit brut obtenu est purifié par chromatographie sur 50 g de silice (0,065-0,200 mm) contenus dans une colonne de 2,2 cm de diamètre [éluant : acétate d'éthyle] en recueillant des fractions de 20 cm3. Les fractions 6 à 13 sont réunies et concentrées à sec sous pression réduite (2,7 kPa) à 40°C. On obtient, après recristallisation dans l'acétonitrile, 0,3 g de N-[(diméthyl-3,3 pipéridino)-2 oxo-2 éthyl] {[(hydroxyimino-1 éthyl)-3 phényl]-3 uréido}-2 N-phényl-acétamide-(E) fondant à 248°C.

L'(hydroxyimino-1 éthyl)-3 aniline peut être préparée de la manière suivante : à une solution de 5,4 g d'amino-3 acétophénone dans 20 cm3 de méthanol, on ajoute 3,16 g de pyridine puis 3,06 g de chlorhydrate d'hydroxylamine en solution dans 10 cm3 d'eau. Le mélange est chauffé à reflux pendant 5 heures, refroidi à une température voisine de 20°C et versé dans 20 cm3 d'eau. La solution obtenue est amenée à pH 8 avec une solution aqueuse 1N de soude. Le produit insoluble est séparé par filtration, lavé par 5 fois 20 cm3 d'eau et séché à l'air. On obtient ainsi, 4,2 g d'(hydroxyimino-1 éthyl)-3 aniline fondant à 133°C.

### EXEMPLE 99

On opère d'une manière analogue à celle décrite à l'exemple 74, mais à partir de 2,2 g de {[N-(N-méthyl N-phényl-carbamoylméthyl) N-phényl-carbamoylméthyl]-3 uréido}-3 mandélate de méthyle-(RS) et de 4,35 cm3 d'une solution aqueuse de soude 1N. On obtient, après recristallisation dans un mélange eau-diméthylformamide (63/35 en volumes) 1,6 g d'acide {[N-(N-méthyl N-phényl-carbamoylméthyl) N-phényl-carbamoylméthyl]-3 uréido}-3 mandélique-(RS) fondant à 246°C.

Le {[N-(N-méthyl N-phényl-carbamoylméthyl) N-phényl-carbamoylméthyl]-3 uréido}-3 mandélate de méthyle-(RS) peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 3,5 g d'{[(imidazolyl-1) carboxamido]-2 N-phényl-acétamido}-2 N-méthyl N-phényl-acétamide et de 3,24 g d'amino-3 mandélate de méthyle-(RS). Le produit brut obtenu est purifié par chromatographie sur 70 g de silice (0,065-0,200mm) contenus dans une colonne de 3 cm de diamètre [éluant : chlorure de méthylène-acétate d'éthyle (50/50 en volumes)] en recueillant des fractions de 30 cm3. Les fractions 18 à 29 sont réunies et concentrées à sec sous pression réduite (2,7kPa) à 40°C. On obtient ainsi 2,5 g de {[N-(N-méthyl N-phényl-carbamoylméthyl) N-phényl-carbamoylméthyl]-3 uréido}-3 mandélate de méthyle-(RS) sous forme d'une meringue utilisée telle quelle dans les synthèses ultérieures.

L'amino-3 mandélate de méthyle-(RS) peut être préparé de la manière suivante : à une solution de 15 g de nitro-3 mandélate de méthyle-(RS) dans 150 cm3 d'éthanol, on ajoute 0,5 g de palladium sur noir à 5%. La suspension est agitée pendant 2 heures à une température voisine de 25°C sous atmosphère d'hydrogène (100kPa). Le catalyseur est ensuite séparé par filtration et le filtrat est concentré à sec sous pression réduite (2,7kPa) à 40°C. On obtient ainsi 13,1 g d'amino-3 mandélate de méthyle-(RS) sous forme d'une huile utilisée telle quelle dans les synthèses ultérieures.

Le nitro-3 mandélate de méthyle-(RS) peut être préparé selon la méthode décrite par L.S. FOSDICK et J.C. CALANDRA, J. Am. Chem. Soc., 63, 1101 (1941).

### EXEMPLE 100

On opère d'une manière analogue à celle décrite à l'exemple 74, mais à partir de 3 g de {{[N-(N-méthyl N-phényl-carbamoylméthyl) N-phényl-carbamoylméthyl]-3 uréido}-3 phényl}-3 propionate d'éthyle et de 5,8 cm3 d'une solution aqueuse de soude 1N. On obtient après recristallisation dans un mélange acétate d'éthyle-diméthylformamide (90/10 en volumes), 0,9 g d'acide {{[N-(N-méthyl N-phényl-carbamoylméthyl) N-phényl-carbamoylméthyl]-3 uréido}-3 phényl}-3 propionique fondant à 208°C.

Le {{[N-(N-méthyl N-phényl-carbamoylméthyl) N-phényl-carbamoylméthyl]-3 uréido}-3 phényl}-3 propionate d'éthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 3,13 g d'{[(imidazolyl-1) carboxamido]-2 N-phényl-acétamido}-2 N-méthyl N-phényl-acétamide et de 3,0 g d'(amino-3 phényl)-3 propionate d'éthyle. Le produit brut est purifié par chromatographie sur 50 g de silice (0,065-0,200mm) contenus dans une colonne de 2 cm de diamètre (éluant : acétate d'éthyle) en recueillant des fractions de 20 cm3. Les fractions 4 à 7 sont réunies et concentrées à sec sous pression réduite (2,7kPa) à 40°C. On obtient ainsi 3 g de {{[N-(N-méthyl N-phényl-carbamoylméthyl) N-phényl-carbamoylméthyl]-3 uréido}-3 phényl)-3 propionate d'éthyle sous forme d'une huile utilisée telle quelle dans les synthèses ultérieures.

L'(amino-3 phényl)-3 propionate d'éthyle peut être préparé d'une manière analogue à celle décrite à l'exemple 99, pour la préparation de l'amino-3 mandélate de méthyle-(RS), mais à partir de 6,6 g de nitro-3 cinnamate d'éthyle-(E) et de 0,2 g de palladium sur noir à 5%. On obtient ainsi, 7,5 g d'(amino-3 phényl)-3 propionate d'éthyle sous forme d'une huile utilisée telle quelle dans les synthèses ultérieures.

Le nitro-3 cinnamate d'éthyle-(E) peut être préparé de la manière suivante : à une solution de 31 g d'acide nitro-3 cinnamique-(E) dans 300 cm3 d'éthanol on ajoute 5 cm3 d'acide sulfurique pur. Le mélange est agité à reflux pendant 3 heures. Après refroidissement et addition de 50 cm3 d'eau, la solution est concentrée à environ 60 cm3 sous pression réduite (2,7kPa) à 40°C. On ajoute 250 cm3 d'acétate d'éthyle puis la phase organique est lavée par 2 fois 100 cm3 d'une solution aqueuse de soude 2N et 2 fois 100 cm3 d'eau, séchée sur sulfate de magnésium et concentrée à sec sous pression réduite (2,7kPa) à 40°C. On obtient ainsi, 32 g de nitro-3 cinnamate d'éthyle-(E) fondant à 70°C.

L'acide nitro-3 cinnamique-(E) peut être préparé de la manière suivante : un mélange de 30,2 g de nitro-3 benzaldéhyde, de 20,8 g d'acide malonique, de 15,8 g de pyridine et de 5 gouttes de pipéridine est chauffé à reflux pendant 1 heure. Après refroidissement, on ajoute 50 cm3 d'eau et le produit insoluble est séparé par filtration, lavé par 3 fois 50 cm3 d'eau et séché à l'air. On obtient ainsi 31 g d'acide nitro-3 cinnamique-(E) fondant à 205°C.

### EXEMPLE 101

On opère d'une manière analogue à celle décrite à l'exemple 74, mais à partir de 1,0 g de {[N-(N-méthyl N-phényl-carbamoylméthyl) N-phényl-carbamoylméthyl]-3 uréido}-3 cinnamate d'éthyle-(E) et de 2 cm3 d'une solution aqueuse de soude 1N. On obtient après recristallisation dans l'acétate d'éthyle, 0,4 g d'acide {[N-(N-méthyl N-phényl-carbamoylméthyl) N-phényl-carbamoylméthyl]-3 uréido}-3 cinnamique-(E) fondant à 210°C.

Le {[N-(N-méthyl N-phényl-carbamoylméthyl) N-phényl-carbamoylméthyl]-3 uréido}-3 cinnamate d'éthyle-(E) peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 3,6 g d'{[(imidazolyl-1) carboxamido]-2 N-phényl-acétamido}-2 N-méthyl N-phényl-acétamide et de 3,5 g d'amino-3 cinnamate d'éthyle-(E). Le produit brut est purifié par chromatographie sur 50 g de silice (0,065-0,200mm) contenus dans une colonne de 2 cm de diamètre (éluant : acétate d'éthyle) en recueillant des fractions de 20 cm3. Les fractions 5 à 17 sont réunies et concentrées à sec sous pression réduite (2,7kPa) à 40°C. On obtient ainsi 2,9 g de {N-(N-méthyl N-phényl-carbamoylméthyl) N-phényl-carbamoylméthyl]-3 uréido}-3 cinnamate d'éthyle-(E) sous forme d'une meringue utilisée telle quelle dans les synthèses ultérieures.

L'amino-3 cinnamate d'éthyle-(E) peut être préparé selon la méthode décrite par BAYER A. G., demande de brevet NL 74 16 449 (C.A. 84, 58 882q).

### EXEMPLE 102

On opère d'une manière analogue à celle décrite à l'exemple 72 mais à partir de 3,1 g d'{[(imidazolyl-1) carboxamido]-2 N-phényl-acétamido}-2 N-méthyl N-phényl-acétamide et de 7 g d'amino-3 benzènesulfonate de tétra-n-butyl ammonium. Le produit brut obtenu est dissous dans 6 cm3 d'acétone et on ajoute une solution de 0,9 g de nonafluorobutanesulfonate de potassium dans 4 cm3 d'acétone. Le produit insoluble est séparé par filtration, lavé par 2 fois 3 cm3 d'acétone et 4 fois 3 cm3 d'oxyde de diisopropyle, puis purifié par chromatographie sur 30 g de silice (0,065-0,200mm) contenus dans une colonne de 2,4 cm de diamètre [éluant : tétrahydrofuranne-eau (92/8 en volumes)] en recueillant des fractions de 10 cm3. Les fractions 4 à 11 sont réunies et concentrées à sec sous pression réduite (2,7kPa) à 45°C. Le résidu est agité pendant 30 minutes dans 6 cm3 d'acétone et le produit insoluble est séparé par filtration, lavé par 2 fois 2 cm3 d'acétone et 4 fois 2 cm3 d'oxyde de diisopropyle puis séché à l'air. On obtient ainsi 0,2 g de {[N-(N-méthyl N-phényl-carbamoylméthyl) N-phényl-carbamoylméthyl]-3 uréido}-3 benzènesulfonate de potassium fondant à environ 280°C.

L'amino-3 benzènesulfonate de tétra-n-butyl ammonium peut être préparé d'une manière analogue à celle décrite à l'exemple 99, pour la préparation de l'amino-3 mandélate de méthyle, mais à partir de 13 g de nitro-3 benzènesulfonate de tétra-n-butyl ammonium et de 0,2 g de palladium sur noir à 5%. On obtient ainsi, 12,4 g d'amino-3 benzènesulfonate de tétra-n-butyl ammonium sous forme d'une huile utilisée telle quelle dans les synthèses ultérieures.

Le nitro-3 benzènesulfonate de tétra-n-butyl ammonium peut être préparé de la manière suivante : à 800 cm3 d'une solution aqueuse de dihydrogénophosphate de potassium 0,5M, on ajoute 6,6 g de nitro-3 benzènesulfonate de sodium, puis 9,8 g d'hydrogénosulfate de tétra-n-butyl ammonium. Le mélange est extrait par 500 cm3 de chlorure de méthylène. La phase organique est séchée sur sulfate de magnésium et concentrée à sec sous pression réduite (2,7kPa) à 40°C. On obtient ainsi, 13 g de nitro-3 benzènesulfonate de tétra-n-butyl ammonium sous forme d'une huile utilisée telle quelle dans les synthèses ultérieures.

### EXEMPLE 103

On opère d'une manière analogue à celle décrite à l'exemple 72, mais à partir de 2,35 g d'{[(imidazolyl-1)carboxamido]-2 N-phényl-acétamido}-2 N-méthyl N-phényl-acétamide et de 1,8 g d'(amino-3 phényl)-2 hydroxy-2 éthanol-(RS). On obtient, après recristallisation dans l'éthanol, 1,3g de {{[(dihydroxy-1,2 éthyl)-3 phényl]-3 uréido}-2 N-phényl-acétamido}-2 N-méthyl N-phényl-acétamide-(RS) fondant à 190°C.

L'(amino-3 phényl)-2 hydroxy-2 éthanol-(RS) peut être préparé d'une manière analogue à celle décrite à l'exemple 99 pour la préparation de l'amino-3 mandélate de méthyle-(RS), mais à partir de 2,3 g d'hydroxy-2 (nitro-3 phényl)-2 éthanol-(RS) et de 0,15 g de palladium sur noir à 5%. On obtient ainsi, 1,8 g d'(amino-3 phényl)-2 hydroxy-2 éthanol-(RS) sous forme d'une huile utilisée telle quelle dans les synthèses ultérieures.

L'hydroxy-2 (nitro-3 phényl)-2 éthanol-(RS) peut être préparé de la manière suivante : on ajoute 2,2 g de (nitro-3 phényl)-2 oxiranne à 90 cm3 d'une solution aqueuse d'acide sulfurique 2N. Le mélange obtenu est agité à une température voisine de 95°C pendant 3 heures. Après refroidissement et addition de 18 cm3 d'une solution aqueuse de soude 10N, l'huile insoluble est extraite par 2 fois 150 cm3 d'acétate d'éthyle. Les phases organiques réunies sont lavées par 100 cm3 d'eau, séchées sur sulfate de magnésium puis concentrées à sec sous pression réduite (2,7kPa) à 40°C. On obtient ainsi 2,3 g d'hydroxy-2 (nitro-3 phényl)-2 éthanol-(RS) fondant à 45°C.

Le (nitro-3 phényl)-2 oxiranne peut être préparé de la manière suivante : à une solution de 4,9 g de bromure de nitro-3 phénacyle dans 100 cm3 d'éthanol on ajoute 0,68 g de borohydrure de sodium. Le mélange est agité pendant 12 heures à une température voisine de 25°C puis versé dans 150 cm3 d'eau. La solution obtenue est concentrée à environ 150 cm3 sous pression réduite (2,7kPa) à 40°C puis extraite par 3 fois 100 cm3 d'acétate d'éthyle. Les phases organiques réunies sont séchées sur sulfate de magnésium et concentrées à sec sous pression réduite (2,7kPa) à 40°C. Le résidu obtenu est purifié par chromatographie sur 100 g de silice (0,065-0,200mm) contenus dans une colonne de 3,2 cm de diamètre (éluant : chlorure de méthylène) en recueillant des fractions de 30 cm3. Les fractions 4 à 8 sont réunies et concentrées à sec sous pression réduite (2,7kPa) à 40°C. On obtient ainsi, 2,2 g de (nitro-3 phényl)-2 oxiranne sous forme d'une huile utilisée telle quelle dans les synthèses ultérieures.

### EXEMPLE 104

On opère d'une manière analogue à celle décrite à l'exemple 74, mais à partir de 0,9 g de {[N-(N-méthyl N-phényl-carbamoylméthyl) N-phényl-carbamoylméthyl]-3 uréido}-3 phénylglyoxylate de méthyle et de 1,8 cm3 d'une solution aqueuse de soude de 1N. Le produit brut est dissous dans 20 cm3 d'une solution aqueuse de soude 4N. La solution obtenue est lavée par 2 fois 20 cm3 d'acétate d'éthyle, puis on ajoute 20 cm3 d'une solution aqueuse d'acide chlorhydrique 4N. Le produit insoluble est séparé par filtration, lavé par 5 fois 5 cm3 d'eau et séché à l'air. On obtient ainsi 0,66 g d'acide {[N-(N-méthyl N-phényl-carbamoylméthyl) N-phényl-carbamoyméthyl]-3 uréido}-3 phénylglyoxylique fondant à 220°C.

Le {[N-(N-méthyl N-phényl-carbamoylméthyl) N-phényl-carbamoylméthyl]-3 uréido}-3 phénylglyoxylate de méthyle peut être préparé d'une manière analogue à celle décrite à l'exemple 84 pour la préparation de l'[(acétyl-3 phényl)-3 uréido]-2 N-[oxo-2 (tétrahydro-1,2,3,4 quinolyl-1)-2 éthyl] N-phényl-acétamide, mais à partir de 1,7 g de {[N-(N-méthyl N-phényl-carbamoylméthyl) N-phényl-carbamoylméthyl]-3 uréido}-3 mandélate de méthyle-(RS) et de 5,3 g de dioxyde de manganèse. Le produit brut est purifié par chromatographie sur 70 g de silice (0,065-0,200mm) contenus dans une colonne de 2,5 cm de diamètre [éluant : chlorure de méthylène -acétate d'éthyle (60-40 en volumes)] en recueillant des fractions de 25 cm3. Les fractions 25 à 33 sont réunies et concentrées à sec sous pression réduite (2,7kPa) à 40°C.On obtient ainsi, 1,3 g de {[N-(N-méthyl N-phényl-carbamoylméthyl) N-phényl-carbamoylméthyl]-3 uréido}-3 phénylglyoxylate de méthyle sous forme d'une meringue utilisée telle quelle dans les synthèses ultérieures.

### EXEMPLE 105

On opère d'une manière analogue à celle décrite à l'exemple 81, mais à partir de 0,6 g d'(amino-2 N-phényl-acétamido)-2 N-isopropyl N-phényl-acétamide et de 0,24 g d'isocyanate de méthyl-3 phényle. Le produit brut obtenu est purifié par chromatographie sur 30 g de silice (0,065-0,200mm) contenus dans une colonne de 1,5 cm de diamètre [éluant : dichlorométhane-acétate d'éthyle (80-20 en volumes) en recueillant des fractions de 20 cm3. Les fractions 6 à 10 sont réunies et concentrées à sec sous pression réduite (2,7kPa) à 40°C. On obtient, après recristallisation dans l'oxyde de diéthyle, 0,3 g de N-isopropyl {[(méthyl-3 phényl)-3 uréido]-2 N-phényl-acétamido}-2 N-phényl-acétamide fondant à 170°C.

L'(amino-2 N-phényl-acétamido)-2 N-isopropyl N-phényl-acétamide peut être préparé d'une manière analogue à celle décrite à l'exemple 72, pour la préparation de l'(amino-2 N-phényl-acétamido)-2 N-méthyl N-phényl-acétamide, mais à partir de 1,3 g de N-isopropyl N-phényl (N-phényl phtalimido-2 acétamido)-2 acétamide et de 0,28 g d'hydrate d'hydrazine. On obtient ainsi, 0,6 g d'(amino-2 N-phényl-acétamido)-2 N-isopropyl N-phényl-acétamide sous forme d'une huile utilisée telle quelle dans les synthèses ultérieures.

Le N-isopropyl N-phényl (N-phényl phtalimido-2 acétamido)-2 acétamide peut être préparé d'une manière analogue à celle décrite à l'exemple 72, pour la préparation du N-méthyl N-phényl (N-phényl phtalimido-2 acétamido)-2 acétamide, mais à partir de 3,4 g d'acide (N-phényl phtalimido-2 acétamido)-2 acétique, de 1,4 g de dichlorure d'oxalyle et de 3,0 g de N-isopropyl aniline. On obtient ainsi, après recristallisation dans l'oxyde de diéthyle, 1,3 g de N-isopropyl N-phényl (N-phényl phtalimido-2 acétamido)-2 acétamide fondant à 152°C.

La N-isopropyl aniline peut être préparée selon la méthode décrite par K. A. SCHELLENBERG, J. Org. Chem., 28, 3259 (1963).

### EXEMPLE 106

A une solution de 1,2 g d'amino-2 N-[(aza-8 spiro [4,5] décanyl-8)-2 oxo-2 éthyl] N-phényl-acétamide dans 50 cm3 de tétrahydrofuranne anhydre, on ajoute à une température voisine de 25°C 0,49 g d'isocyanate de méthyl-3 phényle. Le mélange est agité 12 heures à une température voisine de 25°C et le produit insoluble est séparé par filtration. On obtient, après recristallisation dans l'acétate d'éthyle, 0,7 g de N-[(aza-8 spiro [4,5] décanyl-8)-2 oxo-2 éthyl] [(méthyl-3 phényl)-3 uréido]-2 N-phényl-acétamide fondant à 183°C.

L'amino-2 N-[(aza-8 spiro [4,5] décanyl-8)-2 oxo-2 éthyl] N-phényl-acétamide peut être préparé de la manière suivante : à une solution de 2 g de N-[(aza-8 spiro [4,5] décanyl-8)-2 oxo-2 éthyl] N-phényl phtalimido-2 acétamide dans 100 cm3 de méthanol, on ajoute 0,43 g d'hydrate d'hydrazine. Le mélange réactionnel est chauffé à reflux pendant 2 heures. Après refroidissement et addition de 25 cm3 d'une solution aqueuse N d'acide chlorhydrique, le produit insoluble est séparé par filtration. Le filtrat est concentré sous pression réduite (2,7 kPa) à 30°C. Le résidu est dissous dans 75 cm3 d'eau puis la solution est lavée par 40 cm3 d'éther diéthylique, alcalinisée au moyen de soude et extraite par 2 fois 40 cm3 d'acétate d'éthyle. Les phases organiques réunies sont séchées sur sulfate de magnésium puis concentrées à sec sous pression réduite (2,7 kPa) à 30°C. On obtient ainsi 1,2 g d'amino-2 N-[(aza-8 spiro [4,5] décanyl-8)-2 oxo-2 éthyl] N-phényl-acétamide sous forme d'une huile utilisée telle quelle dans les synthèses ultérieures.

Le N-[(aza-8 spiro [4,5] décanyl-8)-2 oxo-2 éthyl] N-phényl phtalimido-2 acétamide peut être préparé de la manière suivante : à une suspension de 4 g d'acide (phtalimido-2 N-phényl acétamido)-2 acétique dans 40 cm3 de dichloro-1,2 éthane, on ajoute 1,65 g de dichlorure d'oxalyle puis 0,1 cm3 de diméthylformamide. Le mélange est agité 2 heures à une température voisine de 25°C puis on ajoute 3,3 g d'aza-8 spiro [4,5] décane. La solution est agitée 2 heures à une température voisine de 25°C puis lavée par 2 fois 40 cm3 d'eau, séchée sur sulfate de magnésium et concentrée à sec sous pression réduite (2,7 kPa) à 40°C. Le produit obtenu est purifié par chromatographie sur 70 g de silice (0,065-0,200 mm) contenus dans une colonne de 2,5 cm de diamètre [éluant : acétate d'éthyle-cyclohexane (75/25 en volumes)] en recueillant des fractions de 25 cm3. Les fractions 2 à 5 sont réunies et concentrées à sec sous pression réduite (2,7 kPa) à 40°C. On obtient, après recristallisation dans un mélange acétate d'éthyle-cyclohexane (50/50 en volumes) 2,1 g de N-[(aza-8 spiro [4,5] décanyl-8)-2 oxo-2 éthyl] N-phényl phtalimido-2 acétamide fondant à 190°C.

L'aza-8 spiro [4,5] décane peut être préparé selon la méthode décrite par J.M. Mc MANUS et coll., J. Med. Chem., 774 (1965).

### EXEMPLE 107

En opérant d'une manière analogue à celle décrite à l'exemple 106, mais à partir de 1,8 g d'amino-2 N-[(N,N-diéthylcarbamoyl-3 pipéridino)-2 oxo-2 éthyl] N-phényl-acétamide-(RS) et de 0,64 g d'isocyanate de méthyl-3 phényle, on obtient après recristallisation dans un mélange acétate d'éthyle-oxyde de diisopropyle (50/50 en volumes), 0,92 g de N-[(N,N-diéthylcarbamoyl-3 pipéridino)-2 oxo-2 éthyl] [(méthyl-3 phényl)-3 uréido]-2 N-phényl-acétamide-(RS) fondant à 156°C.

L'amino-2 N-[(N,N-diéthylcarbamoyl-3 pipéridino)-2 oxo-2 éthyl] N-phényl-acétamide-(RS) peut être préparé d'une manière analogue à celle décrite à l'exemple 106 pour la préparation de l'amino-2 N-[(aza-8 spiro [4,5] décanyl-8)-2 oxo-2 éthyl] N-phényl-acétamide, mais à partir de 2,9 g de N-[(N,N-diéthylcarbamoyl-3 pipéridino)-2 oxo-2 éthyl] N-phényl phtalimido-2 acétamide-(RS) et de 0,57 g d'hydrate d'hydrazine. On obtient ainsi, 1,8 g d'amino-2 N-[(N,N-diéthylcarbamoyl-3 pipéridino)-2 oxo-2 éthyl] N-phényl-acétamide-(RS) sous forme d'une huile utilisée telle quelle dans les synthèses ultérieures.

Le N-[(N,N-diéthylcarbamoyl-3 pipéridino)-2 oxo-2 éthyl] N-phényl phtalimido-2 acétamide-(RS) peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide mais à partir de 3,4 g d'acide (N-phényl phtalimido-2 acétamido)-2 acétique, de 1,4 g de dichlorure d'oxalyle et de 4,6 g de N,N-diéthylnipécotamide-(RS). On obtient ainsi, 2,9 g de N-[(N,N-diéthylcarbamoyl-3 pipéridino)-2 oxo-2 éthyl] N-phényl phtalimido-2 acétamide, sous forme d'une poudre amorphe utilisée telle quelle dans les synthèses ultérieures.

### EXEMPLE 108

On opère d'une manière analogue à celle décrite à l'exemple 106, mais à partir de 2,0 g d'amino-2 N-[oxo-2 (tétrahydro-1,2,3,4 isoquinolyl-2)-2 éthyl] N-phényl-acétamide et de 0,8 g d'isocyanate de méthyl-3 phényle. Le produit brut est purifié par chromatographie sur 70 g de silice (0,065-0,200 mm) contenus dans une colonne de 2,5 cm de diamètre [éluant : chlorure de méthylène-méthanol (98/2 en volumes)] en recueillant des fractions de 20 cm3. Les fractions 12 à 26 sont réunies et concentrées à sec sous pression réduite (2,7 kPa) à 40°C. On obtient, après recristallisation dans l'acétate d'éthyle, 1,0 g de [(méthyl-3 phényl)-3 uréido]-2 [oxo-2 (tétrahydro-1,2,3,4 isoquinolyl-2)-2 éthyl] N-phényl-acétamide fondant à 135°C.

L'amino-2 N-[oxo-2 (tétrahydro-1,2,3,4 isoquinolyl-2)-2 éthyl] N-phényl-acétamide peut être préparé d'une manière analogue à celle décrite à l'exemple 106, pour la préparation de l'amino-2 N-[(aza-8 spiro [4,5] décanyl-8)-2 oxo-2 éthyl] N-phényl-acétamide, mais à partir de 2,9 g de N-[oxo-2 (tétrahydro-1,2,3,4 isoquinolyl-2)-2 éthyl] N-phényl phtalimido-2 acétamide et de 0,63 g d'hydrate d'hydrazine. On obtient ainsi, 2,0 g d'amino-2 N-[oxo-2 (tétrahydro-1,2,3,4 isoquinolyl-2)-2 éthyl] N-phényl-acétamide sous forme d'une huile utilisée telle quelle dans les synthèses ultérieures.

Le N-[oxo-2 (tétrahydro-1,2,3,4 isoquinolyl-2)-2 éthyl] N-phényl phtalimido-2 acétamide peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide, mais à partir de 4,3 g d'acide (N-phényl phtalimido-2 acétamido)-2 acétique, de 1,65 g de dichlorure d'oxalyle et de 3,6 g de tétrahydro-1,2,3,4 isoquinoléine. Le produit brut obtenu est purifié par chromatographie sur 80 g de silice (0,065-0,200 mm) contenus dans une colonne de 2,5 cm de diamètre [éluant : chlorure de méthylène-méthanol (98/2 en volumes)] en recueillant des fractions de 30 cm3. Les fractions 6 à 15 sont réunies et concentrées à sec sous pression réduite (2,7 kPa) à 40°C. On obtient ainsi, 2,9 g de N-[oxo-2 (tétrahydro-1,2,3,4 isoquinolyl-2)-2 éthyl] N-phényl phtalimido-2 acétamide, sous forme d'une poudre amorphe utilisée telle quelle dans les synthèses ultérieures.

### EXEMPLE 109

A une suspension de 4 g de (N-phényl phtalimido-2 acétamido)-2 N-(quinolyl-8) acétamide dans 40 cm3 de méthanol, on ajoute 0,86 g d'hydrate d'hydrazine. Le mélange réactionnel est chauffé à reflux pendant 40 minutes, puis concentré à sec sous pression réduite (2,7 kPa) à 40°C. Le résidu est agité pendant 30 minutes dans 30 cm3 de tétrahydrofuranne anhydre et le produit insoluble est séparé par filtration. On ajoute alors 0,75 g d'isocyanate de méthyl-3 phényle au filtrat et le mélange est agité 30 minutes à une température voisine de 25°C, puis concentré à sec sous pression réduite (2,7 kPa) à 40°C. Le résidu obtenu est purifié par chromatographie sur 50 g de silice (0,065-0,200 mm) contenus dans une colonne de 2,2 cm de diamètre [éluant : chlorure de méthylène] en recueillant des fractions de 25 cm3. Les fractions 11 à 13 sont réunies et concentrées à sec sous pression réduite (2,7 kPa) à 40°C. On obtient, après recristallisation dans un mélange acétonitrile-acétate d'éthyle (55/45 en volumes), 0,4 g de {[(méthyl-3 phényl)-3 uréido]-2 N-phényl-acétamido}-2 N-(quinolyl-8) acétamide fondant à 170°C.

Le (N-phényl phtalimido-2 acétamido)-2 N-(quinolyl-8) acétamide peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide, mais à partir de 3,4 g d'acide (N-phényl phtalimido-2 acétamido)-2 acétique, de 1,5 g de dichlorure d'oxalyle et de 1,76 g d'amino-8 quinoléine. On obtient ainsi, 4 g de (N-phényl phtalimido-2 acétamido)-2 N-(quinolyl-8) acétamide sous forme d'une poudre amorphe utilisée telle quelle dans les synthèses ultérieures.

### EXEMPLE 110

On opère d'une manière analogue à celle décrite à l'exemple 106, mais à partir de 5,9 g d'[(amino-2 N-phényl-acétamido)-2 acétyl]-1 pipéridine carboxylate-3 d'éthyle-(RS) et de 2,26 g d'isocyanate de méthyl-3 phényle. Le produit brut obtenu est purifié par chromatographie sur 250 g de silice (0,062-0,200 mm) contenus dans une colonne de 4,2 cm de diamètre [éluant : chlorure de méthylène-acétate d'éthyle (50/50 en volumes)] en recueillant des fractions de 40 cm3. Les fractions 4 à 10 sont réunies et concentrées à sec sous pression réduite (2,7 kPa) à 40°C. On obtient, après recristallisation dans l'acétate d'éthyle, 1,4 g de {{[(méthyl-3 phényl)-3 uréido]-2 N-phényl-acétamido}-2 acétyl}-1 pipéridinecarboxylate-3 d'éthyle-(RS) fondant à 166°C.

L'[(amino-2 N-phényl-acétamido)-2 acétyl]-1 pipéridine carboxylate-3 d'éthyle-(RS) peut être préparé d'une manière analogue à celle décrite à l'exemple 106, pour la préparation de l'amino-2 N-[(aza-8 spiro [4,5] décanyl-8)-2 oxo-2 éthyl] N-phényl-acétamide, mais à partir de 8,1 g de [(N-phényl phtalimido-2 acétamido)-2 acétyl]-1 pipéridinecarboxylate-3 d'éthyle-(RS) et de 0,85 g d'hydrate d'hydrazine. On obtient ainsi, 5,6 g d'[(amino-2 N-phényl-acétamido)-2 acétyl]-1 pipéridinecarboxylate-3 d'éthyle-(RS) sous forme d'une huile utilisée telle quelle dans les synthèses ultérieures.

Le [(N-phényl phtalimido-2 acétamido)-2 acétyl]-1 pipéridinecarboxylate-3 d'éthyle-(RS) peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide, mais à partir de 6,8 g d'acide (N-phényl phtalimido-2 acétamido)-2 acétique, de 2,8 g de dichlorure d'oxalyle et de 7,85 g de nipécotate d'éthyle-(RS). On obtient ainsi 9,4 g de [(N-phényl phtalimido-2 acétamido)-2 acétyl]-1 pipéridinecarboxylate-3 d'éthyle-(RS) sous forme d'une poudre amorphe utilisée telle quelle dans les synthèses ultérieures.

### EXEMPLE 111

En opérant d'une manière analogue à celle décrite à l'exemple 106, mais à partir de 1,8 g d'amino-2 N-[oxo-2 (perhydroquinolyl-1)-2 éthyl] N-phényl-acétamide et de 0,73 g d'isocyanate de méthyl-3 phényle, on obtient après recristallisation dans l'acétonitrile, 1,4 g de [(méthyl-3 phényl)-3 uréido]-2 N-[oxo-2 (perhydroquinolyl-1)-2 N-phényl-acétamide (mélange des isomères cis et trans) fondant à 223°C.

L'amino-2 N-[oxo-2 (perhydroquinolyl-1)-2 éthyl] N-phényl-acétamide peut être préparé d'une manière analogue à celle décrite à l'exemple 106, pour la préparation de l'amino-2 N-[(aza-8 spiro [4,5] décanyl-8)-2 oxo-2 éthyl] N-phényl-acétamide, mais à partir de 2,7 g de N-[oxo-2 (perhydroquinolyl-1)-2 éthyl] N-phényl phtalimido-2 acétamide et de 0,6 g d'hydrate d'hydrazine. On obtient ainsi, 1,8 g d'amino-2 N-[oxo-2 (perhydroquinolyl-1)-2 éthyl] N-phényl-acétamide (mélange des isomères cis et trans) sous forme d'une huile utilisée telle quelle dans les synthèses ultérieures.

Le N-[oxo-2 (perhydroquinolyl-1)-2 éthyl] N-phényl phtalimido-2 acétamide peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide, mais à partir de 3,4 g d'acide (N-phényl phtalimido-2 acétamido)-2 acétique, de 1,4 g de dichlorure d'oxalyle et de 4,17 g de perhydroquinoléine (mélange des isomères cis et trans). Le produit brut obtenu est purifié par chromatographie sur 50 g de silice (0,065-0,200 mm) contenus dans une colonne de 2,0 cm de diamètre [éluant : chlorure de méthylène-acétate d'éthyle (80/20 en volumes)] en recueillant des fractions de 20 cm3. Les fractions 10 à 17 sont réunies et concentrées à sec sous pression réduite (2,7 kPa) à 40°C. On obtient ainsi 2,7 g de N-[oxo-2 (perhydroquinolyl-1)-2 éthyl] N-phényl phtalimido-2 acétamide (mélanges des isomères cis et trans) sous forme d'une poudre amorphe utilisée telle quelle dans les synthèses ultérieures.

### EXEMPLE 112

En opérant d'une manière analogue à celle décrite à l'exemple 106 , mais à partir de 1,8 g d'amino-2 N-[oxo-2 (phényl-2 pyrrolidinyl-1)-2 éthyl] N-phényl-acétamide-(RS) et de 0,7 g d'isocyanate de méthyl-3 phényle, on obtient après recristallisation dans un mélange acétate d'éthyle-cyclohexane (50/50 en volumes), 1,8 g de N-[oxo-2 (phényl-2 pyrrolidinyl-1)-2 éthyl] [(méthyl-3 phényl)-3 uréido]-2 N-phényl-acétamide-(RS) fondant à 152°C.

L'amino-2 N-[oxo-2 (phényl-2 pyrrolidinyl-1)-2 éthyl] N-phényl-acétamide-(RS) peut être préparé d'une manière analogue à celle décrite à l'exemple 106, pour la préparation de l'amino-2 N-[(aza-8 spiro [4,5] décanyl-8)-2 oxo-2 éthyl] N-phényl-acétamide, mais à partir de 2,8 g de N-[oxo-2 (phényl-2 pyrrolidinyl-1)-2 éthyl] N-phényl phtalimido-2 acétamide-(RS) et de 0,6 g d'hydrate d'hydrazine. On obtient ainsi, 1,8 g d'amino-2 N-[oxo-2 (phényl-2 pyrrolidinyl-1)-2 éthyl] N-phényl-acétamide-(RS) sous forme d'une huile utilisée telle quelle dans les synthèses ultérieures.

Le N-[oxo-2 (phényl-2 pyrrolidinyl-1)-2 éthyl] N-phényl phtalimido-2 acétamide-(RS) peut être préparé d'une manière analogue à celle décrite à l'exemple 106 pour la préparation du N-[(aza-8 spiro [4,5] décanyl-8)-2 oxo-2 éthyl] N-phényl phtalimido-2 acétamide, mais à partir de 4,0 g d'acide (N-phényl phtalimido-2 acétamido)-2 acétique, de 1,65 g de dichlorure d'oxalyle et de 3,5 g de phényl-2 pyrrolidine-(RS). Le produit brut obtenu est purifié par chromatographie sur 70 g de silice (0,065-0,200 mm) contenus dans une colonne de 2,5 cm de diamètre [éluant : acétate d'éthyle-cyclohexane (75/25 en volumes)] en recueillant des fractions de 20 cm3. Les fractions 8 à 14 sont réunies et concentrées à sec sous pression réduite (2,7 kPa) à 40°C. On obtient ainsi 2,8 g de N-[oxo-2 (phényl-2 pyrrolidinyl-1)-2 éthyl] N-phényl phtalimido-2 acétamide-(RS) sous forme d'une meringue utilisée telle quelle dans les synthèses ultérieures.

La phényl-2 pyrrolidine-(RS) peut être préparée selon la méthode décrite par A. ETIENNE et Y. CORREIA, Bull. Soc. Chim. Fr., 3704 (1969).

### EXEMPLE 113

On opère d'une manière analogue à celle décrite à l'exemple 106, mais à partir de 5,0 g d'(amino-2 N-phényl-acétamido)-2 N-(diméthylamino-4 phényl) N-méthyl-acétamide et de 1,95 g d'isocyanate de méthyl-3 phényle. On obtient, après recristallisation dans l'acétonitrile, 1,7 g de N-(diméthylamino-4 phényl) N-méthyl {[(méthyl-3 phényl)-3 uréido]-2 N-phényl-acétamido}-2 acétamide fondant à 190°C.

L'(amino-2 N-phényl-acétamido)-2 N-(diméthylamino-4 phényl) N-méthyl-acétamide peut être préparé d'une manière analogue à celle décrite à l'exemple 106, pour la préparation de l'amino-2 N-[(aza-8 spiro [4,5] décanyl-8)-2 oxo-2 éthyl] N-phényl-acétamide, mais à partir de 8,6 g de N-(diméthylamino-4 phényl) N-méthyl (N-phényl phtalimido-2 acétamido)-2 acétamide et de 1,8 g d'hydrate d'hydrazine. On obtient ainsi, 5,0 g d'(amino-2 N-phényl-acétamido)-2 N-(diméthylamino-4 phényl) N-méthyl-acétamide sous forme d'une huile utilisée telle quelle dans les synthèses ultérieures.

Le N-(diméthylamino-4 phényl) N-méthyl (N-phényl phtalimido-2 acétamido)-2 acétamide peut être préparé d'une manière analogue à celle décrite à l'exemple 106, pour la préparation du N-[(aza-8 spiro [4,5] décanyl-8)-2 oxo-2 éthyl] N-phényl phtalimido-2 acétamide, mais à partir de 8,4 g d'acide (N-phényl phtalimido-2 acétamido)-2 acétique, de 3,4 g de dichlorure d'oxalyle et de 8,0 g de diméthylamino-4 N-méthyl aniline. On obtient, après recristallisation dans un mélange acétate d'éthyle-cyclohexane (50/50 en volumes), 8,6 g de N-(diméthylamino-4 phényl) N-méthyl (N-phényl phtalimido-2 acétamido)-2 acétamide fondant à 190°C.

La diméthylamino-4 N-méthyl-aniline peut être préparée de la manière suivante : à une suspension de 4,04 g d'hydrure de lithium-aluminium dans 100 cm3 de tétrahydrofuranne anhydre, agitée à une température voisine de 25°C, on ajoute en 15 minutes une solution de 9,4 g de diméthylamino-4 formanilide dans 80 cm3 de tétrahydrofuranne anhydre. Le mélange est agité à une température voisine de 25°C pendant 3 heures. Après refroidissement à une température voisine de 5°C, on ajoute successivement 4,6 cm3 d'eau, 3,4 cm3 d'une solution aqueuse 5N de soude, puis 15,5 cm3 d'eau. La suspension obtenue est agitée pendant 30 minutes et on ajoute 150 cm3 d'oxyde de diéthyle. Le produit insoluble est séparé par filtration et le filtrat est concentré à sec sous pression réduite (2,7 kPa) à 40°C. Le résidu est dissous dans 60 cm3 de dichlorométhane et la phase organique est séchée sur sulfate de magnésium et concentrée à sec sous pression réduite (2,7 kPa) à 40°C. On obtient ainsi 8 g de diméthylamino-4 N-méthyl-aniline sous forme d'une huile utilisée telle quelle dans les synthèses ultérieures.

Le diméthylamino-4 formanilide peut être préparé de la manière suivante : à une solution de 10,8 g de méthylate de sodium dans 100 cm3 de diméthylformamide anhydre, on ajoute 13,6 g de diméthylamino-4 aniline. Le mélange est chauffé à reflux pendant 2 heures en distillant le méthanol formé, puis concentré à sec sous pression réduite (0,1 kPa) à 60°C. Le résidu est repris par 1 litre d'eau et 300 cm3 d'oxyde de diéthyle. La phase organique est séparée par décantation, séchée sur sulfate de magnésium et concentrée à sec sous pression réduite (2,7 kPa) à 30°C. On obtient ainsi 9,4 g de diméthylamino-4 formanilide fondant à 105°C.

### EXEMPLE 114

En opérant de manière analogue à celle décrite à l'exemple 106, mais à partir de 1,0 g d'amino-2 N-[(aza-8 dioxa-1,4 spiro [4,5] décanyl-8)-2 oxo-2 éthyl] N-phényl-acétamide et de 0,4 g d'isocyanate de méthyl-3 phényle, on obtient après recristallisation dans un mélange cyclohexane-acétate d'éthyle (50/50 en volumes) 0,6 g de N-[(aza-8 dioxa-1,4 spiro [4,5] décanyl-8)-2 oxo-2 éthyl] [(méthyl-3 phényl)-3 uréido]-2 N-phényl-acétamide fondant à 130°C.

L'amino-2 N-[(aza-8 dioxa-1,4 spiro [4,5] décanyl-8)-2 oxo-2 éthyl] N-phényl-acétamide peut être préparé d'une manière analogue à celle décrite dans l'exemple 106, pour la préparation de l'amino-2 N-[(aza-8 spiro [4,5] décanyl-8)-2 oxo-2 éthyl] N-phényl-acétamide, mais à partir de 2,7 g de N-[(aza-8 dioxa-1,4 spiro [4,5] décanyl-8)-2 oxo-2 éthyl] N-phényl phtalimido-2 acétamide et de 0,58 g d'hydrate d'hydrazine. On obtient ainsi, 1,0 g d'amino-2 N-[(aza-8 dioxa-1,4 spiro [4,5] décanyl-8)-2 oxo-2 éthyl] N-phényl-acétamide sous forme d'une huile utilisée telle quelle dans les synthèses ultérieures.

Le N-[(aza-8 dioxa-1,4 spiro [4,5] décanyl-8)-2 oxo-2 éthyl] N-phényl phtalimido-2 acétamide peut être préparé de la manière suivante : à une suspension de 10 g d'acide (N-phényl phtalimido-2 acétamido)-2 acétique dans 100 cm3 de tétrahydrofuranne anhydre, on ajoute 5,3 g de N,N'-diimidazole carbonyle. Le mélange est agité à une température voisine de 25°C pendant 3 heures, puis on ajoute 4,75 g d'aza-8 dioxa-1,4 spiro [4,5] décane. Le mélange est agité à une température voisine de 25°C pendant 15 heures. Le produit insoluble est séparé par filtration et le filtrat est concentré à sec sous pression réduite (2,7 kPa) à 40°C. Le résidu est purifié par chromatographie sur 70 g de silice (0,065-0,200 mm) contenus dans une colonne de 2,5 cm de diamètre [éluant : acétate d'éthyle] en recueillant des fractions de 50 cm3. Les fractions 5 à 9 sont réunies et concentrées à sec sous pression réduite (2,7 kPa) à 40°C. On obtient, après recristallisation dans un mélange acétate d'éthyle-oxyde de diisopropyle (50/50 en volumes), 2,7 g de N-[(aza-8 dioxa-1,4 spiro [4,5] décanyl-8)-2 oxo-2 éthyl] N-phényl phtalimido-2 acétamide fondant à 200°C.

### EXEMPLE 115

On opère d'une manière analogue à celle décrite à l'exemple 74, mais à partir de 2,5 g de {{N-[N-(fluoro-2 phényl) N-méthyl-carbamoylméthyl] N-phényl-carbamoylméthyl}-3 uréido}-3 benzoate d'éthyle et de 4,9 cm3 d'une solution aqueuse de soude 1N. On obtient après recristallisation dans un mélange acétate d'éthyle-diméthylformamide (90/10 en volumes) 0,5 g d'acide {{N-[N-(fluoro-2 phényl) N-méthyl-carbamoylméthyl] N-phényl-carbamoylméthyl}-3 uréido)-3 benzoïque fondant à 250°C.

Le {{N-[N-(fluoro-2 phényl) N-méthyl-carbamoylméthyl] N-phényl-carbamoylméthyl}-3 uréido}-3 benzoate d'éthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 11,6 g de N-(fluoro-2 phényl) {[(imidazolyl-1) carboxamido]-2 N-phényl-acétamido}-2 N-méthyl-acétamide et de 9,2 g d'amino-3 benzoate d'éthyle. Le produit brut obtenu est purifié par chromatographie sur 100 g de silice (0,065-0,200mm) contenus dans une colonne de 5 cm de diamètre [éluant : chlorure de méthylène-acétate d'éthyle (40/60 en volumes)] en recueillant des fractions de 20 cm3. Les fractions 6 à 10 sont réunies et concentrées à sec sous pression réduite (2,7kPa) à 40°C. On obtient ainsi 2,5 g de {{N-[N-(fluoro-2 phényl) N-méthyl-carbamoylméthyl] N-phényl-carbamoylméthyl}-3 uréido}-3 benzoate d'éthyle fondant à environ 105°C.

Le N-(fluoro-2 phényl) {[(imidazolyl-1) carboxamido]-2 N-phényl-acétamido}-2 N-méthyl-acétamide peut être préparé d'une manière analogue à celle décrite dans l'exemple 29, pour la préparation de l'{[(imidazolyl-1) carboxamido]-2 N-phényl-acétamido}-2 N-méthyl N-phényl-acétamide, mais à partir de 9,4 g d'(amino-2 N-phényl-acétamido)-2 N-(fluoro-2 phényl) N-méthyl-acétamide et de 7,3 g de N,N'-diimidazole carbonyle. On obtient ainsi 11,6 g de N-(fluoro-2 phényl) {[(imidazolyl-1) carboxamido]-2 N-phényl-acétamido}-2 N-méthyl-acétamide sous forme d'une huile utilisée telle quelle dans les synthèses ultérieures.

### EXEMPLE 116

On opère d'une manière analogue à celle décrite à l'exemple 72, mais à partir de 3,1 g d'{[(imidazolyl-1) carboxamido]-2 N-phényl-acétamido}-2 N-méthyl N-phényl-acétamide et de 7,0 g d'amino-3 benzylsulfonate de tétra-n-butyl ammonium. Le produit brut obtenu est dissous dans 30 cm3 d'acétone et on ajoute une solution de 2,45 g de nonafluorobutanesulfonate de potassium dans 10 cm3 d'acétone. Le produit insoluble est séparé par filtration, lavé par 2 fois 3 cm3 d'acétone et séché à l'air. On obtient, après recristallisation dans l'éthanol, 0,7 g de {[N-(N-méthyl N-phényl-carbamoylméthyl) N-phényl-carbamoylméthyl]-3 uréido}-3 benzylsulfonate de potassium fondant à 194°C.

L'amino-3 benzylsulfonate de tétra-n-butyl ammonium peut être préparé d'une manière analogue à celle décrite à l'exemple 99, pour la préparation de l'amino-3 mandélate de méthyle-(RS), mais à partir de 11,6 g de nitro-3 benzylsulfonate de tétra-n-butyl ammonium et de 0,3 g de palladium sur noir à 5%. On obtient ainsi, 10,5 g d'amino-3 benzylsulfonate de tétra-n-butyl ammonium sous forme d'une huile utilisée telle quelle dans les synthèses ultérieures.

Le nitro-3 benzylsulfonate de tétra-n-butyl ammonium peut être préparé de la manière suivante : à 800 cm3 d'une solution aqueuse de dihydrogénophosphate de potassium 0,5 M, on ajoute 6,9 g de nitro-3 benzylsulfonate de sodium, puis 9,9 g d'hydrogénosulfonate de tétra-n-butyl ammonium. Le mélange est extrait par 500 cm3 de chlorure de méthylène. La phase organique est séchée sur sulfate de magnésium et concentrée à sec sous pression réduite (2,7kPa) à 40°C. On obtient ainsi, 13 g de nitro-3 benzylsulfonate de tétra-n-butyl ammonium sous forme d'une huile utilisée telle quelle dans les synthèses ultérieures.

Le nitro-3 benzylsulfonate de sodium peut être préparé selon la méthode décrite par PURGOTTI et MONTI, Gaz. Chim. Ital., 30, II, 247.

### EXEMPLE 117

On opère d'une manière analogue à celle décrite à l'exemple 74, mais à partir de 0,7 g de {{[N-(N-méthyl N-phényl-carbamoylméthyl) N-phényl-carbamoylméthyl]-3 uréido}-3 phényl}-2 propio nate de méthyle-(RS) et de 1,3 cm3 d'une solution aqueuse de soude 1N. Le produit brut obtenu est dissous dans 4 cm3 d'une solution aqueuse de soude 1N. La solution est filtrée puis acidifiée à pH1 avec une solution aqueuse d'acide chlorhydrique 1N. Le produit insoluble est séparé par filtration, lavé par 5 fois 2 cm3 d'eau et séché à l'air. On obtient ainsi, 0,5 g d'acide {{[N-(N-méthyl N-phényl-carbamoylméthyl) N-phényl-carbamoylméthyl]-3 uréido}-3 phényl}-2 propionique-(RS) fondant à 140°C.

Le {{[N-(N-méthyl N-phényl-carbamoylméthyl) N-phényl-carbamoylméthyl]-3 uréido}-3 phényl}-2 propionate de méthyle-(RS) peut être préparé d'une manière analogue à celle décrite à l'exemple 72, pour la préparation du {[N-(N-méthyl N-phényl-carbamoylméthyl) N-phényl-carbamoylméthyl]-3 uréido}-3 benzoate d'éthyle, mais à partir de 1,1 g d'{[(imidazolyl-1) carboxamido]-2 N-phényl-acétamido}-2 N-méthyl N-phényl-acétamide et de 1,0 g d'(amino-3 phényl)-2 propionate de méthyle-(RS). Le produit brut est purifié par chromatographie sur 50 g de silice (0,065-0,200mm) contenus dans une colonne de 2,5 cm de diamètre [éluant : chlorure de méthylène-acétate d'éthyle (60/40 en volumes)] en recueillant des fractions de 25 cm3. Les fractions 5 à 13 sont réunies et concentrées à sec sous pression réduite (2,7kPa) à 40°C. On obtient ainsi, 0,7 g de {{[N-(N-méthyl N-phényl-carbamoylméthyl) N-phényl-carbamoylméthyl]-3 uréido}-3 phényl}-2 propionate de méthyle-(RS) fondant à 184°C.

L'(amino-3 phényl)-2 propionate de méthyle-(RS) peut être préparé d'une manière analogue à celle décrite à l'exemple 99, pour la préparation de l'amino-3 mandélate de méthyle-(RS), mais à partir de 4 g de (nitro-3 phényl)-2 propionate de méthyle-(RS) et de 0,3 g de palladium sur noir à 5%. On obtient ainsi, 3,3 g d'(amino-3 phényl)-2 propionate de méthyle-(RS) sous forme d'une huile utilisée telle quelle dans les synthèses ultérieures.

Le (nitro-3 phényl)-2 propionate de méthyle -(RS) peut être préparé de la manière suivante : on fait buller pendant 3 heures de l'acide chlorhydrique dans une solution de 5 g de (nitro-3 phényl)-2 propionitrile-(RS) dans 40 cm3 de méthanol. Le mélange obtenu est agité à reflux pendant 30 minutes, et le produit insoluble est séparé par filtration. Le filtrat est concentré à sec sous pression réduite (2,7kPa) à 40°C. Le produit brut est purifié par chromatographie sur 80 g de silice (0,065-0,200mm) contenus dans une colonne de 3,5 cm de diamètre [éluant : éther de pétrole-acétate d'éthyle (80/20 en volumes)] en recueillant des fractions de 100 cm3. Les fractions 1 et 2 sont réunies et concentrées à sec sous pression réduite (2,7kPa) à 40°C. On obtient ainsi 4,0 g de (nitro-3 phényl)-2 propionate de méthyle-(RS) sous forme d'une huile utilisée telle quelle dans les synthèses ultérieures.

Le (nitro-3 phényl)-2 propionitrile-(RS) peut être préparé selon la méthode décrite par E. Felder et Coll., J. Med. Chem., 13, 559 (1970).

### EXEMPLE 118

On opère d'une manière analogue à celle décrite à l'exem- ple 72, mais à partir de 1,6 g d'{[(imidazolyl-1) carboxamido]-2 N-phénylacétamido}-2 N-méthyl N-phényl-acétamide et de 1,2 g d'amino-5 méthoxy-2 phénylméthanol. Le produit brut obtenu est purifié par chromatographie sur 50 g de silice (0,065-0,20mm) contenus dans une colonne de 3 cm de diamètre [éluant : chlorure de méthylène-éthanol (95/5 en volumes)] en recueillant des fractions de 20 cm3. Les fractions 21 à 29 sont réunies et concentrées à sec sous pression réduite (2,7kPa) à 40°C. On obtient ainsi, après recristallisation dans l'acétonitrile, 0,82 g d'{[(hydroxyméthyl-3 méthoxy-4 phényl)-3 uréido]-2 N-phényl-acétamido}-2 N-méthyl N-phényl-acétamide fondant à 181°C.

L'amino-5 méthoxy-2 phénylméthanol peut être préparé d'une manière analogue à celle décrite à l'exemple 99, pour la préparation de l'amino-3 mandélate de méthyle-(RS), mais à partir de 3 g de méthoxy-2 nitro-5 phénylméthanol et de 0,5 g de palladium sur noir à 5%. On obtient ainsi, 1,2 g d'amino-5 méthoxy-2 phénylméthanol sous forme d'une meringue utilisée telle quelle dans les synthèses ultérieures.

Le méthoxy-2 nitro-5 phénylméthanol peut être préparé de la manière suivante : à une solution de 9,3 g d'hydroxy-2 nitro-5 phénylméthanol dans 110 cm3 de diméthylformamide anhydre, on ajoute en 10 minutes, 2,0 g d'une suspension huileuse (50% en poids) d'hydrure de sodium. Le mélange obtenu est agité à une température voisine de 25°C pendant 30 minutes, puis on ajoute en 10 minutes 7,8 g d'iodure de méthyle. Le mélange est agité pendant 2 heures à une température voisine de 25°C, puis versé dans 500 cm3 d'eau et extrait par 3 fois 150 cm3 d'acétate d'éthyle. Les phases organiques réunies sont séchées sur sulfate de magnésium et concentrées à sec sous pression réduite (2,7kPa) à 35°C. On obtient, après recristallisation dans l'acétate d'éthyle, 8 g de méthoxy-2 nitro-5 phénylméthanol fondant à 124°C.

L'hydroxy-2 nitro-5 phénylméthanol peut être préparé de la manière suivante : à une solution de 10 g d'hydroxy-2 nitro-5 benzaldéhyde dans 300 cm3 d'éthanol on ajoute en 10 minutes 2,0 g de borohydrure de sodium. Le mélange est agité pendant 12 heures à une température voisine de 25°C puis versé dans 200 cm3 d'une solution aqueuse d'acide chlorhydrique 1N. La solution obtenue est concentrée à environ 200 cm3 sous pression réduite (2,7kPa) à 40°C puis extraite par 2 fois 100 cm3 d'acétate d'éthyle. Les phases organiques réunies sont séchées sur sulfate de magnésium et concentrées à sec sous pression réduite (2,7kPa) à 40°C. On obtient ainsi, 8,5 g d'hydroxy-2 nitro-5 phénylméthanol fondant à 126°C.

### EXEMPLE 119

On opère d'une manière analogue à celle décrite à l'exemple 74, mais à partir de 1,3 g de méthoxy-2 {[N-(N-méthyl N-phényl-carbamoylméthyl) N-phényl-carbamoylméthyl]-3 uréido}-5 benzoate de méthyle et de 2,6 cm3 d'une solution aqueuse de soude 1N. La solution est filtrée puis acidifiée à pH1 avec une solution aqueuse d'acide chlorhydrique 1N. Le produit insoluble est séparé par filtration, lavé par 5 fois 2 cm3 d'eau et séché à l'air. On obtient ainsi, 0,53 g d'acide méthoxy-2 {[N-(N-méthyl N-phényl-carbamoylméthyl) N-phényl-carbamoylméthyl]-3 uréido}-5 benzoïque fondant à 165°C.

Le méthoxy-2 {[(N-(N-méthyl N-phényl-carbamoylméthyl) N-phényl-carbamoylméthyl]-3 uréido}-5 benzoate de méthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 2,35 g d'{[(imidazolyl-1) carboxamido]-2 N-phényl-acétamido}-2 N-méthyl N-phényl-acétamide et de 2,2 g d'amino-5 méthoxy-2 benzoate de méthyle. Le produit brut est purifié par chromatographie sur 150 g de silice (0,065-0,200mm) contenus dans une colonne de 4,0 cm de diamètre [éluant : chlorure de méthylène-éthanol (95/5 en volumes)] en recueillant des fractions de 30 cm3. Les fractions 14 à 30 sont réunies et concentrées à sec sous pression réduite (2,7kPa) à 40°C. On obtient, après recristallisation dans l'acétate d'éthyle, 1,3 g de méthoxy-2 {[N-(N-méthyl N-phényl-carbamoylméthyl) N-phényl-carbamoylméthyl]-3 uréido}-5 benzoate de méthyle fondant à 140°.

L'amino-5 méthoxy-2 benzoate de méthyle peut être préparé d'une manière analogue à celle décrite à l'exemple 99 pour la préparation de l'amino-3 mandélate de méthyle-(RS) mais à partir de 20 g de méthoxy-2 nitro-5 benzoate de méthyle et de 0,5 g de palladium sur noir à 5%. On obtient ainsi, 17 g d'amino-5 méthoxy-2 benzoate de méthyle sous forme d'une huile utilisée telle quelle dans les synthèse ultérieures.

Le méthoxy-2 nitro-5 benzoate de méthyle peut être préparé de la façon suivante : à une solution de 7,3 g de méthylate de sodium dans 600 cm3 de méthanol on ajoute 25 g de chloro-2 nitro-5 benzoate de méthyle. Le mélange est agité à reflux pendant 3 heures puis coulé dans 1000 cm3 d'une solution aqueuse d'acide chlorhydrique 0,1 N. Le produit insoluble est séparé par filtration, lavé par 5 fois 60 cm3 d'eau et séché à l'air. On obtient ainsi 19,7 g de méthoxy-2 nitro-5 benzoate de méthyle fondant à 99 °C.

Le chloro-2 nitro-5 benzoate de méthyle peut être préparé de la manière suivante : à une solution de 30,1 g d'acide chloro-2 nitro-5 benzoïque dans 580 cm3 de dichloro-1,2 éthane, on ajoute 80 cm3 de méthanol et 16 cm3 d'acide méthanesulfonique. Le mélange est agité à reflux pendant 12 heures puis à une température voisine de 25°C pendant 12 heures. La solution est lavée par 2 fois 200 cm3 d'eau, 2 fois 150 cm3 d'une solution aqueuse saturée d'hydrogénocarbonate de sodium, 2 fois 150 cm3 d'eau puis séchée sur sulfate de magnésium et concentrée à sec sous pression réduite (2,7kPa) à 35°C. On obtient ainsi 31 g de chloro-2 nitro-5 benzoate de méthyle fondant à 70°C.

### EXEMPLE 120

On opère d'une manière analogue à celle décrite à l'exemple 74, mais à partir de 1,5 g de chloro-2 {[N-(N-méthyl N-phényl-carbamoylméthyl) N-phényl-carbamoylméthyl]-3 uréido}-5 benzoate de méthyle et de 2,9 cm3 d'une solution aqueuse de soude 1N. Le produit brut obtenu est dissous dans 24 cm3 d'une solution aqueuse de soude 0,1 N. La solution est lavée par 20 cm3 d'acétate d'éthyle et acidifiée à pH1 avec une solution aqueuse d'acide chlorhydrique 1N. Le produit insoluble est lavé par 5 fois 2 cm3 d'eau et séché à l'air. On obtient ainsi, 0,8 g d'acide chloro-2 {[N-(N-méthyl N-phényl-carbamoylméthyl) N-phényl-carbamoylméthyl]-3 uréido}-5 benzoïque fondant à 240°C.

Le chloro-2 {[N-(N-méthyl N-phényl-carbamoylméthyl) N-phényl-carbamoylméthyl]-3 uréido}-5 benzoate de méthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 2,1 g d'{[(imidazolyl-1) carboxamido]-2 N-phényl-acétamido}-2 N-méthyl N-phényl-acétamide et de 2,0 g d'amino-5 chloro-2 benzoate de méthyle. Le produit brut obtenu est purifié par chromatographie sur 60 g de silice (0,065-0,200mm) contenus dans une colonne de 3 cm de diamètre [éluant cyclohexane-acétate d'éthyle (30/70 en volumes)] en recueillant des fractions de 30 cm3. Les fractions 12 à 23 sont réunies et concentrées à sec sous pression réduite (2,7kPa) à 40°C. On obtient ainsi 1,5 g de chloro-2 {[N-(N-méthyl N-phényl-carbamoylméthyl) N-phényl-carbamoylméthyl]-3 uréido}-5 benzoate de méthyle fondant à 210°C.

L'amino-5 chloro-2 benzoate de méthyle peut être préparé de la manière suivante : à une suspension de 6 g de chloro-2 nitro-5 benzoate de méthyle dans 800 cm3 d'éthanol, on ajoute 80 cm3 d'eau, 4,17 cm3 d'une solution aqueuse d'acide chlorhydrique 11,9N et 4,2 g de fer en poudre. Le mélange est agité à reflux pendant 4 heures puis le produit insoluble est séparé par filtration et lavé par 100 cm3 de chlorure de méthylène. Le filtrat est concentré à environ 100 cm3, lavé par 3 fois 50 cm3 d'acétate d'éthyle, alcalinisé à pH7 avec une solution aqueuse saturée d'hydrogénocarbonate de sodium et extrait par 3 fois 50 cm3 d'oxyde de diéthyle. Les phases organiques réunies sont séchées sur sulfate de magnésium et concentrées à sec sous pression réduite (2,7kPa) à 30°C. On obtient ainsi 2 g d'amino-5 chloro-2 benzoate de méthyle sous forme d'une huile utilisée telle quelle dans les synthèses ultérieures.

### EXEMPLE 121

On opère d'une manière analogue à celle décrite à l'exemple 74, mais à partir de 1,6 g de méthoxy-2 {[N-(N-méthyl N-phényl-carbamoylméthyl) N-phényl-carbamoylméthyl]-3 uréido}-5 phénylacétate de méthyle et de 3,1 cm3 d'une solution aqueuse de soude 1N. Le produit brut obtenu est dissous dans 8 cm3 d'une solution aqueuse de soude 1N. La solution est lavée par 10 cm3 d'acétate d'éthyle, filtrée et acidifiée à pH1 avec une solution aqueuse d'acide chlorhydrique 1N. Le produit insoluble est lavé par 5 fois 2 cm3 d'eau et séché à l'air. On obtient ainsi, 1,1 g d'acide méthoxy-2 {[N-(N-méthyl N-phényl-carbamoylméthyl) N-phényl-carbamoylméthyl]-3 uréido}-5 phénylacétique fondant à 130°C.

Le méthoxy-2 {[N-(N-méthyl N-phényl-carbamoylméthyl) N-phényl-carbamoylméthyl]-3 uréido}-5 phénylacétate de méthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 2,2 g d'{[(imidazolyl-1) carboxamido]-2 N-phényl-acétamido}-2 N-méthyl N-phényl-acétamide et de 2,6 g d'amino-5 méthoxy-2 phénylacétate de méthyle. Le produit brut obtenu est purifié par chromatographie sur 70 g de silice (0,065-0,200mm) contenus dans une colonne de 3 cm de diamètre [éluant : chlorure de méthylène-acétate d'éthyle (65/35 en volumes)] en recueillant des fractions de 25 cm3. Les fractions 18 à 33 sont réunies et concentrées à sec sous pression réduite (2,7kPa) à 40°C. On obtient ainsi 1,7 g de méthoxy-2 {[N-(N-méthyl N-phényl-carbamoylméthyl) N-phényl-carbamoylméthyl]-3 uréido}-5 phénylacétate de méthyle sous forme d'une meringue utilisée telle quelle dans les synthèses ultérieures.

L'amino-5 méthoxy-2 phénylacétate de méthyle peut être préparé d'une manière analogue à celle décrite à l'exemple 99 pour la préparation de l'amino-3 mandélate de méthyle-(RS), mais à partir de 3,1 g de méthoxy-2 nitro-5 phénylacétate de méthyle et de 0,3 g de palladium sur noir à 5%. On obtient ainsi, 2,2 g d'amino-5 méthoxy-2 phénylacétate de méthyle sous forme d'une huile utilisée telle quelle dans les synthèses ultérieures.

Le méthoxy-2 nitro-5 phénylacétate de méthyle peut être préparé de la manière suivante : on fait buller pendant 3 heures de l'acide chlorhydrique dans une solution de 4 g de méthoxy-2 nitro-5 phénylacétonitrile dans 40 cm3 de méthanol. Le mélange obtenu est agité à reflux pendant 1 heure, puis 12 heures à une température voisine de 25°C. Le produit insoluble est séparé par filtration, lavé par 5 fois 10 cm3 d'eau et séché à l'air. On obtient ainsi, 3,2 g de méthoxy-2 nitro-5 phénylacétate de méthyle fondant à 92°C.

Le méthoxy-2 nitro-5 phénylacétonitrile peut être préparé de la manière suivante: à une solution de 6,2 g de bromure de méthoxy-2 nitro-5 benzyle dans 60 cm3 de méthanol on ajoute 10 cm3 d'une solution de cyanure de potassium 3,5 M. Le mélange est agité à reflux pendant 4 heures puis concentré à sec sous pression réduite. Le résidu obtenu est repris par 100 cm3 d'oxyde de diéthyle et 100 cm3 d'eau. La phase organique est séchée sur sulfate de magnésium puis concentrée à sec sous pression réduite (2,7kPa) à 35°C. Le produit brut obtenu est purifié par chromatographie sur 40 g de silice (0,065-0,200) contenus dans une colonne de 3 cm de diamètre (éluant : chlorure de méthylène) en recueillant des fractions de 20 cm3. Les fractions 3 à 8 sont réunies et concentrées à sec sous pression réduite (2,7kPa) à 40°C. On obtient ainsi 4,2 g de méthoxy-2 nitro-5 phénylacétonitrile fondant à 100°C.

Le bromure de méthoxy-2 nitro-5 benzyle peut être préparé de la manière suivante : à une solution de 5,2 g de méthoxy-2 nitro-5 phénylméthanol dans 150 cm3 d'acétonitrile on ajoute 13,9 g de tétrabromure de carbone puis 8,9 g de triphénylphosphine en 10 mn. Le mélange est agité pendant 1 heure à une température voisine de 20°C puis concentré à sec sous pression réduite (2,7kPa) à 40°C. Le produit brut obtenu est purifié par chromatographie sur 25 g de silice (0,065-0,200) contenus dans une colonne de 4,0 cm de diamètre [éluant : chlorure de méthylène-éther de pétrole (40/60 en volumes)] en recueillant des fractions de 125 cm3. Les fractions 5 à 12 sont réunies et concentrées à sec sous presion réduite (2,7kPa) à 40°C. On obtient ainsi, 5,6 g de bromure de méthoxy-2 nitro-5 benzyle fondant 78°C.

### EXEMPLE 122

On opère d'une manière analogue à celle décrite à l'exemple 81, mais à partir de 1 g d'amino-2 N-[oxo-2 (phényl-2 pipéridino)-2 éthyl] N-phényl-acétamide-(RS) et de 0,38 g d'isocyanate de méthyl-3 phényle. On obtient ainsi, après recristallisation dans un mélange acétate d'éthyle-oxyde de diéthyle (60/40 en volumes) 0,9 g de [(méthyl-3 phényl)-3 uréido]-2 N-[oxo-2 (phényl-2 pipéridino)-2 éthyl] N-phényl-acétamide-(RS) fondant à 125°C.

L'amino-2 N-[oxo-2 (phényl-2 pipéridino)-2 éthyl] N-phényl-acétamide-(RS) peut être préparé d'une manière analogue à celle décrite à l'exemple 2, pour la préparation de l'(amino-2 N-phényl-acétamido)-2 N-méthyl N-phényl-acétamide, mais à partir de 15,7 g de N-[oxo-2 (phényl-2 pipéridino)-2 éthyl] N-phényl phtalimido-2 acétamide-(RS) et de 3,2 g d'hydrate d'hydrazine. On obtient ainsi, 8,6 g d'amino-2 N-[oxo-2 (phényl-2 pipéridino)-2 éthyl] N-phényl-acétamide-(RS) sous forme d'une huile utilisée telle quelle dans les synthèses ultérieures.

Le N-[oxo-2 (phényl-2 pipéridino)-2 éthyl] N-phényl phtalimido-2 acétamide-(RS) peut être préparé d'une manière analogue à celle décrite à l'exemple 2, pour la préparation du N-méthyl N-phényl (N-phényl phtalimido-2 acétamido)-2 acétamide, mais à partir de 13,5 g d'acide (N-phényl phtalimido-2 acétamido )-2 acétique, de 5,1 g de dichlorure d'oxalyle, de 2,6 g de pyridine et de 5,9 g de phényl-2 pipéridine-(RS). On obtient ainsi, 15,8 g de N-[oxo-2 (phényl-2 pipéridino)-2 éthyl] N-phényl phtalimido-2 acétamide-(RS) sous forme d'une meringue utilisée telle quelle dans les synthèses ultérieures.

La phényl-2 pipéridine-(RS) peut être préparée selon la méthode décrite par C.G OVERBERGER et L.P. HERIN, J. Org. Chem., 27, 417 (1962).

### EXEMPLE 123

On opère d'une manière analogue à celle décrite à l'exemple 72, mais à partir de 1,1 g d'[(imidazolyl-1) carboxamido]-2 N-[oxo-2 (phényl-2 pipéridino)-2 éthyl] N-phényl-acétamide-(RS) et de 0,55 g d'hydroxyméthyl-3 aniline. Le produit brut est purifié par chromatographie sur 50 g de silice (0,065-0,200mm) contenus dans une colonne de 2,5 cm de diamètre [éluant : chlorure de méthylène-acétate d'éthyle (40/60 en volumes)] en recueillant des fractions de 40 cm3. les fractions 7 à 11 sont réunies et concentrées à sec sous pression réduite (2,7 kPa) à 40°C. On obtient, après recristallisation dans un mélange acétate d'éthyle-oxyde de diisopropyle (20/80 en volumes), 0,7 g d'[(hydroxyméthyl-3 phényl)-3 uréido]-2 N-[oxo-2 (phényl-2 pipéridino)-2 éthyl] N-phényl-acétamide-(RS) fondant à 104°C.

L'[(imidazolyl-1) carboxamido]-2 N-[oxo-2 (phényl-2 pipéridino)-2 éthyl] N-phényl-acétamide-(RS) peut être préparé d'une manière analogue à celle décrite dans l'exemple 72, pour la préparation de l'{[imidazolyl-1) carboxamido]-2 N-phényl-acétamido)-2 N-méthyl N-phényl-acétamide, mais à partir de 7,5 g d'amino-2 N-[oxo-2 (phényl-2 pipéridino)-2 éthyl] N-phényl-acétamide-(RS) et de 5,2 g de N,N'-diimidazole carbonyle. On obtient ainsi, 7,3 g de N-[oxo-2 (diméthyl-3,3 pipéridino)-2 éthyl] N-phényl phtalimido-2 acétamide-(RS) sous forme d'une meringue utilisée telle quelle dans les synthèses ultérieures.

### EXEMPLE 124

On opère d'une manière analogue à celle décrite à l'exemple 74, mais à partir de 0,6 g de {{N-[oxo-2 (phényl-2 pipéridino)-2 éthyl] N-phényl-carbamoylméthyl}-3 uréido}-3 phénylacétate d'éthyle-(RS) et de 1,1 cm3 d'une solution de soude 1N. Le produit est dissous dans 3 cm3 d'une solution aqueuse de soude 1N. La solution est filtrée puis acidifiée avec 3 cm3 d'une solution aqueuse d'acide chlorhydrique 1N. Le produit insoluble est séparé par filtration, lavé par 5 fois 2 cm3 d'eau et séché à l'air. On obtient ainsi, 0,5 g d'acide {{N-[oxo-2 (phényl-2 pipéridino)-2 éthyl] N-phényl-carbamoylméthyl}-3 uréido}-3 phénylacétique-(RS) fondant à 135°C.

Le {{N-[oxo-2 (phényl-2 pipéridino)-2 éthyl] N-phényl-carbamoylméthyl}-3 uréido}-3 phénylacétate d'éthyle-(RS) peut être préparé d'une manière analogue à celle décrite à l'exemple 72 pour la préparation du {[N-(N-méthyl N-phényl-carbamoylméthyl) N-phényl-carbamoylméthyl]-3 benzoate d'éthyle, mais à partir de 1,3 g d'[(imidazolyl-1) carboxamido]-2 N-[oxo-2 (phényl-2 pipéridino)-2 éthyl] N-phényl-acétamide-(RS) et de 1 g d'amino-3 phénylacétate d'éthyle. Le produit brut obtenu est purifié par chromatographie sur 80 g de silice (0,065-0,200mm) contenus dans une colonne de 3 cm de diamètre [éluant : chlorure de méthylène-acétate d'éthyle (70/30 en volumes)] en recueillant des fractions de 30 cm3. Les fractions 11 à 38 sont réunies et concentrées à sec sous pression réduite (2,7kPa) à 40°C. On obtient ainsi 0,8 g de {{N-[oxo-2 (phényl-2 pipéridino)-2 éthyl] N-phényl-carbamoylméthyl}-3 uréido}-3 phénylacétate d'éthyle-(RS) sous forme d'une meringue utilisée telle quelle dans les synthèses ultérieures.

### EXEMPLE 125

On opère d'une manière analogue à celle décrite à l'exemple 74, mais à partir de 0,9 g de {[N-(oxo-2 thiomorpholino-2 éthyl) N-phényl-carbamoylméthyl]-3 uréido}-3 benzoate d'éthyle et de 1,9 cm3 d'une solution aqueuse de soude 1N. Le produit brut obtenu est dissous dans 5 cm3 d'une solution aqueuse de soude 1N. La solution est filtrée, puis acidifiée à pH1 avec une solution aqueuse d'acide chlorhydrique 1N. Le produit insoluble est séparé par filtration, lavé par S fois 2 cm3 d'eau et séché à l'air. On obtient ainsi, 0,6 g d'acide {[N-(oxo-2 thiomorpholino-2 éthyl) N-phényl-carbamoylméthyl]-3 uréido}-3 benzoïque fondant à 280°C.

Le {[N-(oxo-2 thiomorpholino-2 éthyl) N-phényl-carbamoylméthyl]-3 uréido}-3 benzoate d'éthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 1,5 g d'[(imidazolyl-1) carboxamido]-2 N-(oxo-2 thiomorpholino-2 éthyl) N-phényl-acétamide et de 1,3 g d'amino-3 benzoate d'éthyle. Le produit brut obtenu est purifié par chromatographie sur 70 g de silice (0,065-0,200mm) contenus dans une colonne de 3 cm de diamètre [éluant : chlorure de méthylène-acétate d'éthyle (50/50 en volumes)] en recueillant des fractions de 30 cm3. Les fractions 1 et 2 sont réunies et concentrées à sec sous pression réduite (2,7kPa) à 40°C. On obtient ainsi 2,5 g de {[N-(oxo-2 thiomorpholino-2 éthyl) N-phényl-carbamoylméthyl]-3 uréido}-3 benzoate d'éthyle fondant à 183°C.

L'[(imidazolyl-1) carboxamido]-2 N-(oxo-2 thiomorpholino-2 éthyl) N-phényl-acétamide peut être préparé d'une manière analogue à celle décrite dans l'exemple 29 pour la préparation de l'{[imidazolyl-1) carboxamido]-2 N-phényl-acétamido}-2 N-méthyl N-phényl-acétamide, mais à partir de 1,8 g d'amino-2 N-(oxo-2 thiomorpholino-2 éthyl) N-phényl-acétamide et de 3,7 g de N,N'-diimidazole carbonyle. On obtient ainsi, 1,5 g d'[(imidazolyl-1) carboxamido]-2 N-(oxo-2 thiomorpholino-2 éthyl) N-phényl-acétamide sous forme d'une meringue utilisée telle quelle dans les synthèses ultérieures.

L'amino-2 N-(oxo-2 thiomorpholino-2 éthyl) N-phényl-acétamide peut être préparé d'une manière analogue à celle décrite à l'exemple 2, pour la préparation de l'(amino-2 N-phényl-acétamido)-2 N-méthyl N-phényl-acétamide, mais à partir de 4,8 g de N-(oxo-2 thiomorpholino-2 éthyl) N-phényl phtalimido-2 acétamide et de 1,1 g d'hydrate d'hydrazine. On obtient ainsi, 1,8 g d'amino-2 N-(oxo-2 thiomorpholino-2 éthyl) N-phényl-acétamide sous forme d'une huile utilisée telle quelle dans les synthèses ultérieures.

Le N-(oxo-2 thiomorpholino-2 éthyl) N-phényl phtalimido-2 acétamide peut être préparé d'une manière analogue à celle décrite à l'exemple 2, pour la préparation du N-méthyl N-phényl (N-phényl phtalimido-2 acétamido)-2 acétamide, mais à partir de 4,9 g d'acide (N-phényl phtalimido-2 acétamido )-2 acétique, de 2,0 g de dichlorure d'oxalyle et de 3,0 g de thiomorpholine. On obtient ainsi, 5,9 g de N-(oxo-2 thiomorpholino-2 éthyl) N-phényl phtalimido-2 acétamide sous forme d'une huile utilisée telle quelle dans les synthèses ultérieures.

### EXEMPLE 126

On opère d'une manière analogue à celle décrite à l'exemple 81, mais à partir de 0,7 g d'amino-2 N-[(diméthyl-2,2 thiomorpholino)-2 oxo-2 éthyl] N-phényl-acétamide et de 0,28 g d'isocyanate de méthyl-3 phényle. On obtient, après recristallisation dans un mélange acétate d'éthyle-acétonitrile (80/20 en volumes), 0,6 g de N-[(diméthyl-2,2 thiomorpholino)-2 oxo-2 éthyl] [(méthyl-3 phényl)-3 uréido]-2 N-phényl-acétamide fondant à 186°C.

L'amino-2 N-[(diméthyl-2,2 thiomorpholino)-2 oxo-2 éthyl] N-phényl-acétamide peut être préparé d'une manière analogue à celle décrite à l'exemple 2, pour la préparation de l'(amino-2 N-phényl-acétamido)-2 N-méthyl N-phényl-acétamide, mais à partir de 8,0 g de N-[(diméthyl-2,2 thiomorpholino)-2 oxo-2 éthyl] N-phényl phtalimido-2 acétamide et de 1,77 g d'hydrate d'hydrazine. On obtient ainsi, 4,4 g d'amino-2 N-[(diméthyl-2,2 thiomorpholino)-2 oxo-2 éthyl] N-phényl-acétamide sous forme d'une huile utilisée telle quelle dans les synthèses ultérieures.

Le N-[(diméthyl-2,2 thiomorpholino)-2 oxo-2 éthyl] N-phényl phtalimido-2 acétamide peut être préparé d'une manière analogue à celle décrite à l'exemple 2, pour la préparation du N-méthyl N-phényl (N-phényl phtalimido-2 acétamido)-2 acétamide, mais à partir de 7,0 g d'acide (N-phényl phtalimido-2 acétamido )-2 acétique, de 2,9 g de dichlorure d'oxalyle, de 2,1 g de pyridine et de 3,5 g de diméthyl-2,2 thiomorpholine. On obtient ainsi, 8,1 g de N-[(diméthyl-2,2 thiomorpholino)-2 oxo-2 éthyl] N-phényl phtalimido-2 acétamide sous forme d'une huile utilisée telle quelle dans les synthèses ultérieures.

La diméthyl-2,2 thiomorpholine peut être préparée selon la méthode décrite par J. Mc. Manus et coll., J. Med. Chem., 766 (1965).

### EXEMPLE 127

On opère d'une manière analogue à celle décrite à l'exemple 74, mais à partir de 1,3 g de {{N-[(diméthyl-2,2 thiomorpholino)-2 oxo-2 éthyl] N-phényl-carbamoylméthyl}-3 uréido}-3 benzoate d'éthyle et de 2,5 cm3 d'une solution aqueuse de soude 1N. Le produit brut obtenu est dissous dans 5 cm3 d'une solution aqueuse de soude 1N. La solution est filtrée, puis acidifiée à pH1 avec une solution aqueuse d'acide chlorhydrique 1N. Le produit insoluble est séparé par filtration, lavé par 5 fois 2 cm3 d'eau et séché à l'air. On obtient ainsi, 0,6 g d'acide {{N-[(diméthyl-2,2 thiomorpholino)-2 oxo-2 éthyl] N-phényl-carbamoylméthyl}-3 uréido}-3 benzoïque fondant à 275°C.

Le {{N-[(diméthyl-2,2 thiomorpholino)-2 oxo-2 éthyl] N-phényl-carbamoylméthyl}-3 uréido}-3 benzoate d'éthyle peut être préparé d'une manière analogue à celle décrite à l'exemple 72 pour la préparation du {[N-(N-méthyl N-phényl-carbamoylméthyl) N-phényl-carbamoylméthyl}-3 uréido]-3 benzoate d'éthyle, mais à partir de 2,0 g de N-[(diméthyl-2,2 thiomorpholino)-2 oxo-2 éthyl] [(imidazolyl-1) carboxamido]-2 N-phényl-acétamide et de 1,6 g d'amino-3 benzoate d'éthyle. Le produit brut obtenu est purifié par chromatographie sur 60 g de silice (0,065-0,200mm) contenus dans une colonne de 3 cm de diamètre [éluant : chlorure de méthylène-acétate d'éthyle (60/40 en volumes)]en recueillant des fractions de 25 cm3. Les fractions 9 à 25 sont réunies et concentrées à sec sous pression réduite (2,7kPa) 40°C. On obtient ainsi 1,3 g de {{N-[(diméthyl-2,2 thiomorpholino)-2 oxo-2 éthyl] N-phényl-carbamoylméthyl}-3 uréido} -3 benzoate d'éthyle sous forme d'une meringue utilisée telle quelle dans les synthèses ultérieures.

Le N-[(diméthyl-2,2 thiomorpholino)-2 oxo-2 éthyl] [(imidazolyl-1) carboxamido]-2 N-phényl-acétamide peut être préparé d'une manière analogue à celle décrite à l'exemple 29 pour la préparation de l'{[(imidazolyl-1) carboxamido]-2 N-phényl-acétamido}-2 N-méthyl N-phényl-acétamide, mais à partir de 3,6 g d'amino-2 N-[(diméthyl-2,2 thiomorpholino)-2 oxo-2 éthyl] N-phényl-acétamide et de 2,7 g de N,N'-diimidazole carbonyle. On obtient ainsi, 4,3 g de N-[(diméthyl-2,2 thiomorpholino)-2 oxo-2 éthyl] [(imidazolyl-1) carboxamido]-2 N-phényl-acétamide sous forme d'une meringue utilisée telle quelle dans les synthèses ultérieures.

### EXEMPLE 128

A une solution de 0,65 g de méthylate de sodium dans 15 cm3 de méthanol, on ajoute 0,56 g de chlorhydrate d'hydroxylamine. Le mélange est agité 30 minutes à une température voisine de 25°C, puis on ajoute une solution de 2 g de {{N-[N-(fluoro-2 phényl) N-méthyl-carbamoylméthyl] N-phényl-carbamoylméthyl}-3 uréido}-3 benzoate d'éthyle dans 10 cm3 de méthanol. Le mélange est agité à reflux pendant 2 heures, puis 12 heures à 25°C. Le produit insoluble est séparé par filtration. Le filtrat est acidifiée à pH1 avec une solution d'acide chlorhydrique 0,1 N puis extrait par 2 fois 50 cm3 d'acétate d'éthyle. Les phases organiques réunies sont séchées sur sulfate de magnésium puis concentrées à sec sous pression réduite (2,7kPa) à 40°C. Le produit brut obtenu est purifié par chromatographie sur 40 g de silice (0,065-0,200mm) contenus dans une colonne de 1,5 cm de diamètre [éluant : acétate d'éthyle-méthanol (9/10 en volumes)] en recueillant des fractions de 20 cm3. Les fractions 5 à 10 sont réunies et concentrées à sec sous pression réduite (2,7kPa) à 40°C. On obtient, après recristallisation dans l'acétonitrile, 0,3 g d'acide{{N-[N-(fluoro-2 phényl) N-méthyl-carbamoylméthyl] N-phényl-carbamoylméthyl}-3 uréido}-3 benzènecarbohydroxamique fondant à 220°C.

### EXEMPLE 129

On opère d'une manière analogue à celle décrite à l'exemple 72, mais à partir de 0,65 g d'{[imidazolyl-1) carboxamido]-2 N-phényl-acétamido}-2 N-méthyl N-phényl-acétamide et de 0,5 g d'(amino-3 phényl)-3 propène-2 ol-1-(E). Le produit brut obtenu est purifié par chromatographie sur 40 g de silice (0,065-0,200mm) contenus dans une colonne de 2 cm de diamètre [éluant : chlorure de méthylène-acétate d'éthyle (50/50 en volumes)] en recueillant des fractions de 10 cm3. Les fractions 8 à 15 sont réunies et concentrées à sec sous pression réduite (2,7kPa) à 40°C. On obtient, après recristallisation dans un mélange acétate d'éthyle-oxyde de diisopropyle (15/85 en volumes) 0,45 g d'{{[(hydroxy-3 propène-1 yl)-3 phényl]-3 uréido}-2 N-phényl-acétamido}-2 N-méthyl N-phényl-acétamide-(E) fondant à 120°C.

L'(amino-3 phényl)-3 propène-2 ol-1-(E) peut être préparé de la manière suivante : à une solution de 1,7 g d'amino-3 cinnamate d'éthyle-(E) dans 75 cm3 d'oxyde de diéthyle maintenue à une température voisine de -15°C, on ajoute en 10 minutes 1 g d'hydrure de lithium-aluminium. La suspension obtenue est agitée à une température voisine de -10°C pendant 2 heures puis on ajoute 1,2 cm3 d'eau, 0,85 cm3 d'une solution aqueuse de soude 5N et 3,9 cm3 d'eau. Le produit insoluble est séparé par filtration et le filtrat est concentré à sec sous pression réduite (2,7kPa) à 30°C. Le produit brut obtenu est purifié par chromatographie sur 35 g de silice (0,065-0,200mm) contenus dans une colonne de 2,0 cm de diamètre (éluant : acétate d'éthyle) en recueillant des fractions de 20 cm3. Les fractions 6 à 10 sont réunies et concentrées à sec sous pression réduite (2,7kPa) à 40°C. On obtient, après recristallisation dans l'acétate d'éthyle, 0,5 g d'(amino-3 phényl)-3 propène-2 ol-1-(E) fondant à 85°C.

### EXEMPLE 130

On opère d'une manière analogue à celle décrite à l'exemple 72, mais à partir de 1,7 g d'{[imidazolyl-1) carboxamido]-2 N-phényl-acétamido}-2 N-méthyl N-phényl-acétamide et de 1,5 g d'(amino-3 benzyl)-5 tétrazole. Le produit brut obtenu est purifié par chromatographie sur 50 g de silice (0,065-0,200m) contenus dans une colonne de 3 cm de diamètre [éluant : chlorure de méthylène-éthanol (90/10 en volumes)] en recueillant des fractions de 20 cm3. Les fractions 1 et 2 sont réunies et concentrées à sec sous pression réduite (2,7kPa) à 40°C. On obtient, après recristallisation dans un mélange acétonitrile-oxyde de diisopropyle (50/50 en volumes), 0,4 g de N-méthyl N-phényl {{{[(tétrazolyl-5) méthyl]-3 phényl}-3 uréido}-2 N-phényl-acétamido}-2 acétamide fondant à 206°C.

L'(amino-3 benzyl)-5 tétrazole peut être préparé de la manière suivante : à une solution de 3,9 g de (nitro-3 benzyl)-5 tétrazole dans 80 cm3 d'éthanol, on ajoute 0,3 g de palladium sur noir à 5%. La suspension est agitée pendant 2 heures à une température voisine de 25°C sous atmosphère d'hydrogène (100kPa). Le catalyseur est ensuite séparé par filtration et le filtrat est concentré à sec sous pression réduite (2,7kPa) à 40°C. On obtient ainsi, 3,1 g d'(amino-3 benzyl)-5 tétrazole fondant à 140°C.

Le (nitro-3 benzyl}-5 tétrazole peut être préparé de la manière suivante : à une solution de 1,6 g de nitro-3 phénylacétonitrile dans 25 cm3 de diméthylformamide anhydre on ajoute 1,43 g d'azoture de sodium et 1,17 g de chlorure d'ammonium anhydre. Le mélange est agité à une température voisine de 100°C pendant 22 heures, puis concentré à sec sous pression réduite (1,2 kpa)) à 80°C. Le résidu obtenu est repris par 25 cm3 d'une solution d'acide chlorhydrique 2N et le mélange est extrait par 2 fois 50 cm3 de chlorure de méthylène. Les phases organiques réunies sont séchées sur sulfate de magnésium et concentrées à sec sous pression réduite (2,7kPa) à 35°C. On obtient ainsi, 1,6 g de (nitro-3 benzyl)-5 tétrazole fondant à 140°C.

Le nitro-3 phénylacétonitrile peut être préparé de la manière suivante : à une solution de 20,6 g de chlorure de nitro-3 benzyle dans 120 cm3 de méthanol on ajoute 20 cm3 d'une solution aqueuse de cyanure de potassium 8,5 M. Le mélange est agité à reflux pendant 4 heures puis concentré à sec sous pression réduite (2,7kPa) à 45°C. Le résidu est repris par 200 cm3 d'oxyde de diéthyle et 150cm3 d'eau. La phase organique est séchée sur sulfate de magnésium et concentrée à sec sous pression réduite (2,7kPa) à 35°C. Le produit brut obtenu est purifié par chromatographie sur 50 g de silice (0,065-0,200mm) contenus dans une colonne de 2 cm de diamètre (éluant : chlorure de méthylène) en recueillant des fractions de 30 cm3. Les fractions 4 à 9 sont réunies et concentrées à sec sous pression réduite (2,7kPa) à 40°C. On obtient ainsi, 11 g de nitro-3 phénylacétonitrile fondant à 60°C.

### EXEMPLE 131

On opère d'une manière analogue à celle décrite à l'exemple 74, mais à partir de 0,4 g d'hydroxyimino-2 {{[N-(N-méthyl N-phényl-carbamoylméthyl) N-phényl-carbamoylméthyl]-3 uréido}-3 phényl}-2 acétate de méthyle, Forme A et de 7,7 cm3 d'une solution aqueuse de soude 0,1N. Le produit obtenu est dissous dans 2 cm3 d'une solution aqueuse de soude 1N, filtré puis acidifié à pH1 avec une solution aqueuse d'acide chlorhydrique 4N. Le produit insoluble est séparé par filtration, lavé par 3 fois 1 cm3 d'eau et séché à l'air. On obtient ainsi 0,35 g d'acide hydroxyimino-2 {{[N-(N-méthyl N-phényl-carbamoylméthyl) N-phényl-carbamoylméthyl]-3 uréido}-3 phényl}-2 acétique, Forme A fondant à 158°C.

Les formes A et B de l'hydroxyimino-2 {{[N-(N-méthyl N-phényl-carbamoylméthyl) N-phényl-carbamoylméthyl]-3 uréido}-3 phényl}-2 acétate de méthyle peuvent être préparées de la manière suivante : à une solution de 0,9 g de {N-(N-méthyl N-phényl-carbamoylméthyl) N-phényl-carbamoylméthyl]-3 uréido}-3 phénylglyoxylate de méthyle dans 5 cm3 de méthanol, on ajoute 0,14 g de pyridine puis 0,15 g de chlorhydrate d'hydroxylamine en solution dans 2,5 cm3 d'eau. Le mélange est chauffé à reflux pendant 3 heures et coulé dans 25 cm3 d'eau. Le produit insoluble est séparé par filtration, lavé par 3 fois 2 cm3 d'eau et purifié par chromatographie sur 20 g de silice (0,065-0,200mm) contenus dans une colonne de 2,0 cm de diamètre [éluant : chlorure de méthylène-acétate d'éthyle (50/50 en volumes)] en recueillant des fractions de 10 cm3. Les fractions 5 à 10 sont réunies et concentrées à sec sous pression réduite (2,7kPa) à 40°C. On obtient ainsi, 0,4 g d'hydroxyimino-2 {{[N-(N-méthyl N-phényl-carbamoylméthyl) N-phényl-carbamoylméthyl]-3 uréido}-3 phényl}-2 acétate de méthyle, forme A sous forme d'une meringue utilisée telle quelle dans les synthèses ultérieures. Les fractions 22 à 30 sont réunies et concentrées à sec sous pression réduite (2,7kPa) à 40°C. On obtient ainsi, 0,3 g d'hydroxyimino-2 {{N-(N-méthyl N-phényl-carbamoylméthyl) N-phényl-carbamoylméthyl]-3 uréido}-3 phényl}-2 acétate de méthyle, forme B, sous forme d'une meringue utilisée telle quelle dans les synthèses ultérieures.

### EXEMPLE 132

On opère d'une manière analogue à celle décrite à l'exemple 74, mais à partir de 0,3 g d'hydroxyimino-2 {{(N-(N-méthyl N-phényl-carbamoylméthyl) N-phényl-carbamoylméthyl]-3 uréido}-3 phényl}-2 acétate de méthyle, Forme B et de 5,8 cm3 d'une solution aqueuse de soude 0,1N. Le produit brut obtenu est dissous dans 2 cm3 d'une solution aqueuse de soude 1N, filtré puis acidfié à pH1 avec une solution aqueuse d'acide chlorhydrique 4N. Le produit insoluble est séparé par filtration, lavé par 3 fois 0,5 cm3 d'eau et séché à l'air. On obtient ainsi 0,1 g d'acide hydroxyimino-2 {{[N-(N-méthyl N-phényl-carbamoylméthyl) N-phényl-carbamoylméthyl]-3 uréido}-3 phényl}-2 acétique, Forme B fonfant à 220°C.

### EXEMPLE 133

A une solution de 1,7 g d'{[(amino-3 phényl)-3 uréido]-2 N-phényl-acétamido}-2 N-méthyl N-phényl-acétamide dans 25 cm3 de chlorure de méthylène, on ajoute 0,47 g de triéthylamine et 1,32 g d'anhydride trifluorométhanesufonique. Le mélange est agité pendant 18 heures à une température voisine de 25°C, dilué par 30 cm3 de chlorure de méthylène et lavé par 3 fois 50 cm3 d'eau. La phase organique est séchée sur sulfate de magnésium, concentrée à sec sous pression réduite (2,7kPa) à 35°C et purifié par chromatographie sur 50 g de silice (0,065-0,200) contenus dans une colonne de 2,5 cm de diamètre [éluant : chlorure de méthylène-méthanol (95/5 en volumes)] en recueillant des fractions de 30 cm3. Les fractions 10 à 20 sont réunies et concentrées à sec sous pression réduite (2,7kPa) à 40°C. On obtient après recristallisation dans l'acétate d'éthyle, 0,32 g de N-méthyl N-phényl {[(trifluorométhylsulfamoyl-3 phényl)-3 uréido]-2 N-phényl-acétamido}-2 acétamide fondant à 232°C.

### EXEMPLE 134

On opère d'une manière analogue à celle décrite à l'exemple 74, mais à partir de 1,0 g de {[N-(N-méthyl N-phényl-carbamoylméthyl) N-phényl-carbamoylméthyl]-3 uréido}-3 phénylthioacétate d'éthyle et de 1,9 cm3 d'une solution aqueuse de soude 1N. Le produit brut obtenu est dissous dans 30 cm3 d'une solution aqueuse de soude 1N. La solution est lavée par 30 cm3 d'acétate d'éthyle, filtrée et acidifiée à pH1 avec une solution aqueuse d'acide chlorhydrique 1N. Le produit insoluble est lavé par 5 fois 2 cm3 d'eau et séché à l'air. On obtient ainsi, 0,65 g d'acide {[N-(N-méthyl N-phényl-carbamoylméthyl) N-phényl-carbamoylméthyl]-3 uréido}-3 phénylthioacétique fondant à 185°C.

Le {[N-(N-méthyl N-phényl-carbamoylméthyl) N-phényl-carbamoylméthyl]-3 uréido}-3 phénylthioacétate d'éthyle peut être préparé d'une manière analogue à celle décrite à l'exemple 72 pour la préparation du {[N-(N-méthyl N-phényl-carbamoylméthyl) N-phényl-carbamoylméthyl]-3 uréido}-3 benzoate d'éthyle, mais à partir de 1,44 g d'{[imidazolyl-1) carboxamido]-2 N-phényl-acétamido}-2 N-méthyl N-phényl-acétamide et de 1,6 g d'amino-3 phénylthioacétate d'éthyle. Le produit brut obtenu est purifié par chromatographie sur 50 g de silice (0,065-0,200mm) contenus dans une colonne de 2,5 cm de diamètre [éluant : chlorure de méthylène-éthanol (95-5 en volumes)] en recueillant des fractions de 50 cm3. Les fractions 11 à 17 sont réunies et concentrées à sec sous pression réduite (2,7kPa) à 40°C. On obtient ainsi, 1,0 g de {[N-(N-méthyl N-phényl-carbamoylméthyl) N-phényl-carbamoylméthyl]-3 uréido}-3 phénylthioacétate d'éthyle fondant à 120°C.

L'amino-3 phénylthioacétate d'éthyle peut être préparé de la manière suivante : à une solution de 12,5 g d'amino-3 thiophénol dans 200 cm3 d'éthanol, on ajoute en 5 minutes 16,7 g de bromoacétate d'éthyle. Le mélange est agité à une température voisine de 20°C pendant 3 heures, puis concentré à sec sous pression réduite (2,7kPa) à 40°C. Le produit obtenu est dissous dans un mélange de 100 cm3 d'acétate d'éthyle et de 100 cm3 d'une solution aqueuse de soude 1N. La phase organique est séparée, lavée par 2 fois 50 cm3 d'eau, séchée sur sulfate de magnésium et concentrée à sec sous pression réduite (2,7kPa) à 40°C. Le produit obtenu est purifié par chromatographie sur 250 g de silice (0,065-0,200mm) contenus dans une colonne de 5 cm de diamètre [éluant : acétate d'éthyle-cyclohexane (70/30 en volumes)] en recueillant des fractions de 20 cm3. Les fractions 12 à 19 sont réunies et concentrées à sec sous pression réduite (2,7 kPa) à 40°C. On obtient ainsi 13 g d'amino-3 phénylthioacétate d'éthyle sous forme d'une huile utilisée telle quelle dans les synthèses ultérieures.

La présente invention concerne également les médicaments constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemples des crèmes, lotions, collyres, collutoires, gouttes nasales ou aérosols.

En thérapeutique humaine, les composés selon l'invention sont particulièrement utiles pour le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la maladie de Parkinson, de la diskinésie tardive, du syndrome du colon irritable, de la pancréatite aiguë, des ulcères et des désordres de la motilité intestinale, de certaines tumeurs de l'oesophage inférieur, du colon et de l'intestin, comme potentialisateur de l'activité analgésique des médicaments analgésiques narcotiques et non narcotiques et comme régulateur de l'appétit.

Les doses dépendent de l'effet recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter. Les exemples suivants illustrent des compositions selon l'invention :

### Exemple A

On prépare, selon la technique habituelle, des gélules dosées à 50 mg de produit actif ayant la composition suivante :
- {{[(hydroxyimino-1 éthyl)-3 phényl]-3 uréido}-2 N-phényl-acétamido}-2 N-méthyl N-phényl-acétamide-(E) 50 mg
- cellulose 18 mg
- lactose 55 mg
- silice colloïdale 1 mg
- carboxyméthylamidon sodique 10 mg
- talc 10 mg
- stéarate de magnésium 1 mg

### Exemple B

On prépare, selon la technique habituelle, des comprimés dosés à 50 mg de produit actif ayant la composition suivante :
- {[(hydroxyméthyl-3 phényl)-3 uréido]-2 N-phényl-acétamido}-2 N-méthyl N-phényl-acétamide 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (72-3,5-24,5) q.s.p 1 comprimé pelliculé terminé à 245 mg

### Exemple C

On prépare une solution injectable contenant 10 mg de produit actif ayant la composition suivante :
- acide {[N-(N-méthyl N-phényl-carbamoylméthyl) N-phényl carbamoylméthyl]-3 uréido}-3 benzoïque 50 mg
- acide benzoïque 80 mg
- alcool benzylique 0,06 cm3
- benzoate de sodium 80 mg
- éthanol à 95 % 0,4 cm3
- hydroxyde de sodium 24 mg
- propylène glycol 1,6 cm3
- eau q.s.p. 4 cm3

## Revendications (Revendications pour l'(les) Etat(s) contractant(s) suivant(s): AT, BE, CH, DE, DK, FR, GB, IT, LI, LU, NL, SE)

1. Composés de formule : 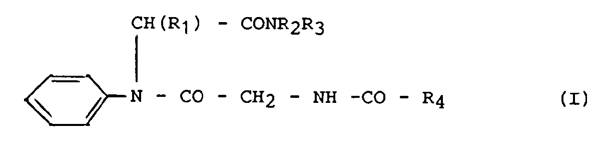 dans laquelle
- R₁ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un atome d'hydrogène ou un radical alkyle (éventuellement substitué par un radical alcoxycarbonyle),
- R₃ représente un radical alkyle, phénylalkyle, indanyle, cycloalkylalkyle, phényle (éventuellement substitué par un ou plusieurs substituants choisis parmi les atomes d'halogène et les radicaux alkyle, alcoxy, alkylthio et dialkylamino) ou quinolyle, ou bien
- R₂ et R₃ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un ou plusieurs radicaux alkyle, alcoxycarbonyle, dialkylcarbamoyle, phényle, alcoxy ou en combinaison avec un atome de carbone de l'hétérocycle un cycle spiro monocyclique à 4 ou 5 chaînons et contenant éventuellement un ou plusieurs hétéroatomes (O,S,N),
- R₄ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, hydroxy, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, hydroxyaminocarbonyle, tétrazolyl-5, tétrazolyl-5 alkyle, alkylsulfinyle, mono ou polyhydroxyalkyle, sulfo, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX et -S-alk-COOX,
- X représente un atome d'hydrogène ou un radical alkyle,
- alk représente un radical alkyle ou alkylène,
- alk' représente un radical hydroxyalkylène ou hydroxyalkyle,
à l'exception des composés pour lesquels R₁ représente un atome d'hydrogène, R₂ et R₃ représentent chacun un radical alkyle ou R₂ et R₃ forment avec l'atome d'azote auquel ils sont rattachés un radical pyrrolidinyl-1 éventuellement substitué par un radical alkyle et R₄ représente un radical naphtyle, indolyle ou phénylamino dont le noyau phényle est éventuellement substitué par un radical alkyle, alcoxy, nitro, hydroxy ou alkylthio ou par un ou deux atomes d'halogène,
étant entendu que les radicaux acyle contiennent 2 à 4 atomes de carbone, les radicaux alkyle, alkylène et alcoxy et les portions alkyle, alkylène et alcoxy contiennent 1 à 4 atomes de carbone en chaîne droite ou ramifiée et les radicaux cycloakyle contiennent 3 à 6 atomes de carbone, ainsi que leurs racémiques et leurs énantiomères lorsqu'ils contiennent au moins un centre asymétrique et leurs sels.

2. Composés de formule (I) selon la revendication 1 pour lesquels R₂ et R₃ représentent avec l'atome d'azote auquel ils sont rattachés un hétérocycle choisi parmi les cycles pipéridino (éventuellement substitué par au moins un radical alkyle, alcoxycarbonyle, phényle ou dialkylcarbamoyle), perhydroazépinyl-1, tétrahydro-1,2,3,6 pyridyl-1, tétrahydro-1,2,3,4 quinolyl-1, pyrrolidinyl-1, dihydro-3,4 2H-benzoxazine-1,4 yl-4, dihydro-3,4 2H-benzothiazine-1,4 yl-4, N-alkyl tétrahydro-1,2,3,4 quinoxalinyl-1, perhydroquinolyl-1, tétrahydro-1,2,3,4 isoquinolyl-2, aza-8 spiro [4,5] décanyl-8, phényl-2 ou -3 pyrrolidinyl-1, aza-8 dioxa-1,4 spiro [4,5] décanyl-8, thiomorpholino (éventuellement substitué par au moins un radical alkyle) ou indolinyl-1.

3. Composés de formule (I) selon la revendication 1 pour lesquels R₁ représente un atome d'hydrogène, R₂ représente un radical alkyle, R₃ représente un radical phényle ou bien R₂ et R₃ forment avec l'atome d'azote auquel ils sont rattachés un radical pipéridino substitué par au moins un radical alkyle ou thiomorpholino substitué par au moins un radical alkyle et R₄ représente un radical phénylamino dont le noyau phényle est substitué par un ou plusieurs substituants choisis parmi les radicaux hydroxy, carboxy, hydroxyiminoalkyle, -alk-COOH, alkyle, alkylthio, monohydroxyalkyle et -C(=NOH)-COOH, ainsi que leurs racémiques et leurs énantiomères lorsqu'ils contiennent au moins un centre asymètrique et leurs sels.

4. Procédé de préparation des composés de formule (I) selon la revendication 1 pour lesquels R₄ est un radical phénylamino dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk caractérisé en ce que l'on fait réagir un dérivé aminé de formule : 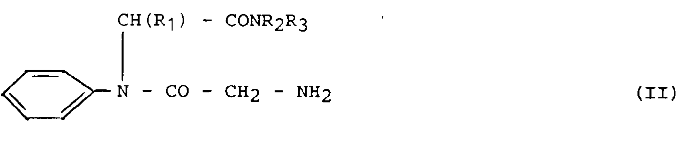 dans laquelle R₁, R₂ et R₃ ont les mêmes significations que dans la revendication 1 sur un isocyanate de formule :
OCN - R₅ (III)
dans laquelle R₅ représente un radical phényle éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk, puis isole le produit et le transforme éventuellement en sel.

5. Procédé de préparation des composés de formule (I) selon la revendication 1 pour lequels R₄ représente un radical phénylamino dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk, caractérisé en ce que l'on fait réagir une amine de formule : 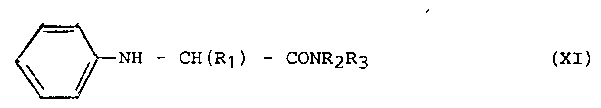 dans laquelle R₁, R₂ et R₃ ont les mêmes significations que dans la revendication 1, sur un acide de formule :
HOOC - CH₂ - NH - CO - R₄ (XV)
dans laquelle R₄ a les mêmes significations que ci-dessus puis isole le produit et le transforme éventuellement en sel.

6. Procédé de préparation des composés de formule (I) selon la revendication 1 pour lesquels R₄ représente un radical phénylamino éventuellement substitué caractérisé en ce que l'on fait réagir un dérivé de formule : 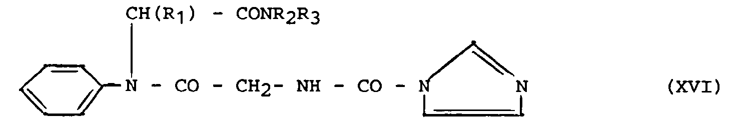 dans laquelle R₁, R₂ et R₃ ont les mêmes significations que dans la revendication 1, sur une amine de formule :
H₂N - R₇ (XVII)
dans laquelle R₇ représente un radical phényle éventuellement substitué par un ou plusieurs substituants choisis parmi les atomes d'halogène et les radicaux alkyle, alcoxy, alkylthio, hydroxy, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, tétrazolyl-5, tétrazolyl-5 alkyle, alkylsulfinyle, hydroxyaminocarbonyle, mono ou polyhydroxyalkyle, sulfo, -alk-O-CO-alk, -alk-O-alk, -alk-COOX, -O-alk-COOX, -alk'-COOX, -CH=CH-COOX, -CO-COOX, -alk-SO₃H, -CH=CH-alk',-C(=NOH)-COOX et -S-alk-COOX, isole le produit et le transforme éventuellement en sel.

7. Procédé de préparation des composés de formule (I) selon la revendication 1 pour lesquels R₄ représente un radical phénylamino dont le noyau phényle est substitué par au moins un radical carboxy, -alk-COOH, -O-alk-COOH, -alk'-COOH, -CH=CH-COOH, -CO-COOH, -C(=NOH)-COOH ou -S-alk-COOH caractérisé en ce que l'on hydrolyse un ester correspondant, isole le produit et le transforme éventuellement en sel.

8. Procédé de préparation des composés de formule (I) selon la revendication 1 pour lesquels R₄ représente un radical phénylamino dont le noyau phényle est substitué par au moins un radical amino à l'exception de ceux pour lesquels R₁ représente un radical phényle substitué par au moins un radical nitro caractérisé en ce que l'on réduit le dérivé nitré correspondant, isole le produit et le transforme éventuellement en sel.

9. Procédé de préparation des composés de formule (I) selon la revendicaton 1 pour lesquels R₄ représente un radical phénylamino dont le noyau phényle est substitué par au moins un radical phénylhydroxyméthyle caractérisé en ce que l'on réduit le dérivé benzoylé correspondant, isole le produit et le transforme éventuellement en sel.

10. Procédé de préparation des composés de formule (I) selon la revendication 1 pour lesquels R₄ représente un radical phénylamino dont le noyau phényle est substitué par au moins un radical acyle caractérisé en ce que l'on oxyde un composé hydroxyalkylé correspondant, isole le produit et le transforme éventuellement en sel.

11. Procédé de préparation des composés de formule (I) selon la revendicaton 1 pour lesquels R₄ représente un radical phénylamino dont le noyau phényle est substitué par au moins un radical hydroxyiminoalkyle, alcoxyiminoalkyle, hydroxyaminocarbonyle ou -C(=NOH)-COOX dans lequel X représente un radical alkyle caractérisé en ce que l'on fait réagir un dérivé correspondant pour lequel R₄ représente un radical phénylamino dont le noyau phényle est substitué par au moins un radical acyle, formyle, alcoxycarbonyle ou -CO-COOX dans lequel X représente un radical alkyle sur un dérivé de formule :
H₂N-OR₈ (XVIII)
dans laquelle R₈ représente un atome d'hydrogène ou un radical alkyle, isole le produit et le transforme éventuellement en sel.

12. Procédé de préparation des composés de formule (I) selon la revendication 1 pour lesquels R₄ représente un radical phénylamino dont le noyau phényle est substitué par au moins un radical alkylsulfinyle caractérisé en ce que l'on oxyde un dérivé alkylthio correspondant, isole le produit et le transforme éventuellement en sel.

13. Procédé de préparation des composés de formule (I) selon la revendication 1 pour lesquels R₄ représente un radical phénylamino dont le noyau phényle est substitué par au moins un radical -alk-O-CO-alk caractérisé en ce que l'on fait réagir un chlorure d'acyle sur un composé hydroxyalkylé correspondant, isole le produit et le transforme éventuellement en sel.

14. Procédé de préparation des composés de formule (I) selon la revendication 1 pour lesquels R₄ représente un radical phénylamino dont le noyau phényle est substitué par au moins un radical trifluorométhylsulfonamido caractérisé en ce que l'on fait réagir un composé correspondant pour lequel R₄ représente un radical phénylamino dont le noyau phényle est substitué par au moins un radical amino sur l'anhydride trifluorométhanesulfonique, isole le produit et le transforme éventuellement en sel.

15. Procédé de préparation des composés de formule (I) selon la revendication 1, pour lesquels R₄ représente un radical phényle éventuellement substitué, naphtyle, indolyle ou quinolyle carctérisé en ce que l'on fait réagir un dérivé de formule : 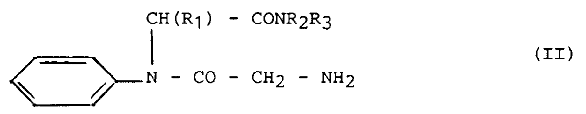 dans laquelle R₁, R₂, R₃, ont les mêmes significations que dans la revendication 1 sur un dérivé de formule :
HOOC R₄ (XIX)
dans laquelle R₄ a les mêmes significations que ci-dessus, isole le produit et le transforme éventuellement en sel.

16. Médicaments caractérisés en ce qu'ils contiennent comme ingrédient actif au moins un comosé de formule (I) selon la revendication 1.

17. Médicaments caractérisés en ce qu'ils contiennent comme ingrédient actif au moins un composé de formule (I) selon la revendications 2 ou 3.

18. Médicaments selon les revendications 16 et 17 pour le traitement et la prévention des désordres liés à la CCK et à la gastrine au niveau du système nerveux et de l'appareil gastrointestinal.

## Revendications (Revendications pour l'(les) Etat(s) contractant(s) suivant(s): ES, GR)

1. Procédé de préparation des composés de formule : 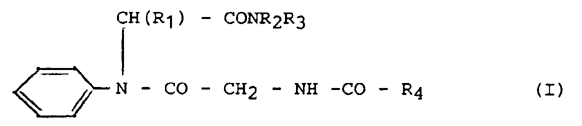 dans laquelle
- R₁ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un atome d'hydrogène ou un radical alkyle (éventuellement substitué par un radical alcoxycarbonyle),
- R₃ représente un radical alkyle, phénylalkyle, indanyle, cycloalkylalkyle, phényle (éventuellement substitué par un ou plusieurs substituants choisis parmi les atomes d'halogène et les radicaux alkyle, alcoxy, alkylthio et dialkylamino) ou quinolyle,
ou bien
- R₂ et R₃ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un ou plusieurs radicaux alkyle, alcoxycarbonyle, dialkylcarbamoyle, phényle, alcoxy ou en combinaison avec un atome de carbone de l'hétérocycle un cycle spiro monocyclique à 4 ou 5 chaînons et contenant éventuellement un ou plusieurs hétéroatomes (O,S,N),
- R₄ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, hydroxy, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, hydroxyaminocarbonyle, tétrazolyl-5, tétrazolyl-5 alkyle, alkylsulfinyle, mono ou polyhydroxyalkyle, sulfo, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX et -S-alk-COOX,
- X représente un atome d'hydrogène ou un radical alkyle,
- alk représente un radical alkyle ou alkylène,
- alk' représente un radical hydroxyalkylène ou hydroxyalkyle,
à l'exception des composés pour lesquels R₁ représente un atome d'hydrogène, R₂ et R₃ représentent chacun un radical alkyle ou R₂ et R₃ forment avec l'atome d'azote auquel ils sont rattachés un radical pyrrolidinyl-1 éventuellement substitué par un radical alkyle et R₄ représente un radical naphtyle, indolyle ou phénylamino dont le noyau phényle est éventuellement substitué par un radical alkyle, alcoxy, nitro, hydroxy ou alkylthio ou par un ou deux atomes d'halogène,
étant entendu que les radicaux acyle contiennent 2 à 4 atomes de carbone, les radicaux alkyle, alkylène et alcoxy et les portions alkyle, alkylène et alcoxy contiennent 1 à 4 atomes de carbone en chaîne droite ou ramifiée et les radicaux cycloakyle contiennent 3 à 6 atomes de carbone, ainsi que leurs racémiques et leurs énantiomères lorsqu'ils contiennent au moins un centre asymétrique et leurs sels, caractérisé en ce que
A- pour la préparation des composés pour lesquels R₄ est un radical phénylamino dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk on fait réagir un dérivé aminé de formule : 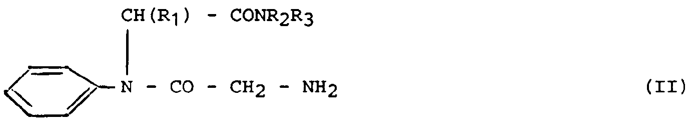 dans laquelle R₁, R₂ et R₃ ont les mêmes significations que dans la formule (I) sur un isocyanate de formule :
OCN **-** R₅ (III)
dans laquelle R₅ représente un radical phényle éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk, puis isole le produit et le transforme éventuellement en sel,
B- pour la préparation des composés pour lequels R₄ représente un radical phénylamino dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle, trifluorométhylsulfonamido et -alk-O-alk, on fait réagir une amine de formule : 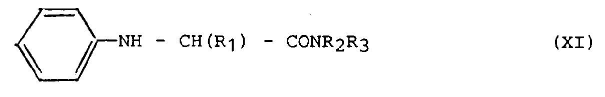 dans laquelle R₁, R₂ et R₃ ont les mêmes significations que dans la formule (I), sur un acide de formule :
HOOC - CH₂ - NH - CO - R₄ (XV)
dans laquelle R₄ a les mêmes significations que ci-dessus puis isole le produit et le transforme éventuellement en sel,
C- pour la préparation des composés pour lesquels R₄ représente un radical phénylamino éventuellement substitué on fait réagir un dérivé de formule : 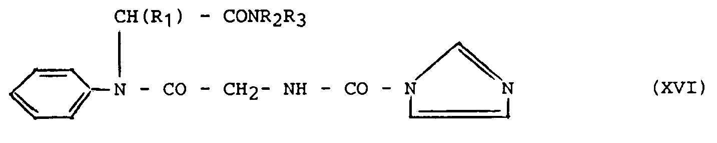 dans laquelle R₁, R₂ et R₃ ont les mêmes significations que dans la formule (I), sur une amine de formule :
H₂N - R₇ (XVII)
dans laquelle R₇ représente un radical phényle éventuellement substitué par un ou plusieurs substituants choisis parmi les atomes d'halogène et les radicaux alkyle, alcoxy, alkylthio, hydroxy, nitro, amino, acyle, cyano, sulfamoyle, trifluorométhylsulfonamido, carbamoyle, benzoyle, carboxy, alcoxycarbonyle, phénylhydroxyméthyle, pipéridino, hydroxyiminoalkyle, alcoxyiminoalkyle, tétrazolyl-5, tétrazolyl-5 alkyle, alkylsulfinyle, hydroxyaminocarbonyle, mono ou polyhydroxyalkyle, sulfo, -alk-O-CO-alk, -alk-O-alk, -alk-COOX, -O-alk-COOX, -alk'-COOX, -CH=CH-COOX, -CO-COOX, -alk-SO₃H, -CH=CH-alk',-C(=NOH)-COOX et -S-alk-COOX, isole le produit et le transforme éventuellement en sel,
D - pour la préparation des composés pour lesquels R₄ représente un radical phénylamino dont le noyau phényle est substitué par au moins un radical carboxy, -alk-COOH, -O-alk-COOH, -alk'-COOH, -CH=CH-COOH, -CO-COOH, -C(=NOH)-COOH ou -S-alk-COOH on hydrolyse un ester correspondant, isole le produit et le transforme éventuellement en sel,
E - pour la préparation des composés pour lesquels R₄ représente un radical phénylamino dont le noyau phényle est substitué par au moins un radical amino à l'exception de ceux pour lesquels R₁ représente un radical phényle substitué par au moins un radical nitro on réduit le dérivé nitré correspondant, isole le produit et le transforme éventuellement en sel,
F - pour la préparation des composés pour lesquels R₄ représente un radical phénylamino dont le noyau phényle est substitué par au moins un radical phénylhydroxyméthyle on réduit le dérivé benzoylé correspondant, isole le produit et le transforme éventuellement en sel,
G - pour la préparation des composés pour lesquels R₄ représente un radical phénylamino dont le noyau phényle est substitué par au moins un radical acyle on oxyde un composé hydroxyalkylé correspondant, isole le produit et le transforme éventuellement en sel,
H - pour la préparation des composés pour lesquels R₄ représente un radical phénylamino dont le noyau phényle est substitué par au moins un radical hydroxyiminoalkyle, alcoxyiminoalkyle, hydroxyaminocarbonyle ou -C(=NOH)-COOX dans lequel X représente un radical alkyle on fait réagir un dérivé correspondant pour lequel R₄ représente un radical phénylamino dont le noyau phényle est substitué par au moins un radical acyle, formyle, alcoxycarbonyle ou -CO-COOX dans lequel X représente un radical alkyle sur un dérivé de formule :
H₂N-OR₈ (XVIII)
dans laquelle R₈ représente un atome d'hydrogène ou un radical alkyle, isole le produit et le transforme éventuellement en sel,
I - pour la préparation des composés pour lesquels R₄ représente un radical phénylamino dont le noyau phényle est substitué par au moins un radical alkylsulfinyle on oxyde un dérivé alkylthio correspondant, isole le produit et le transforme éventuellement en sel,
J - pour la préparation des composés pour lesquels R₄ représente un radical phénylamino dont le noyau phényle est substitué par au moins un radical -alk-O-CO-alk on fait réagir un chlorure d'acyle sur un composé hydroxyalkylé correspondant, isole le produit et le transforme éventuellement en sel,
K - pour la préparation des composés pour lesquels R₄ représente un radical phénylamino dont le noyau phényle est substitué par au moins un radical trifluorométhylsulfonamido on fait réagir un composé correspondant pour lequel R₄ représente un radical phénylamino dont le noyau phényle est substitué par au moins un radical amino sur l'anhydride trifluorométhanesulfonique, isole le produit et le transforme éventuellement en sel,
L - pour la préparation des composés pour lesquels R₄ représente un radical phényle éventuellement substitué, naphtyle, indolyle ou quinolyle on fait réagir un dérivé de formule : 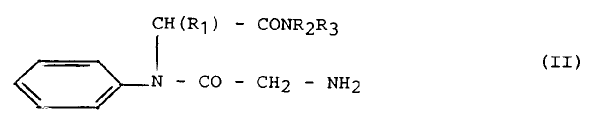 dans laquelle R₁, R₂, R₃, ont les mêmes significations que dans la formule (I) sur un dérivé de formule :
HOOC R₄ (XIX)
dans laquelle R₄ a les mêmes significations que ci-dessus, isole le produit et le transforme éventuellement en sel.

2. Procédé selon la revendication 1 pour la préparation des composés de formule (I) pour lesquels R₂ et R₃ représentent avec l'atome d'azote auquel ils sont rattachés un hétérocycle choisi parmi les cycles pipéridino (éventuellement substitué par au moins un radical alkyle, alcoxycarbonyle, phényle ou dialkylcarbamoyle), perhydroazépinyl-1, tétrahydro-1,2,3,6 pyridyl-1, tétrahydro-1,2,3,4 quinolyl-1, pyrrolidinyl-1, dihydro-3,4 2H-benzoxazine-1,4 yl-4, dihydro-3,4 2H-benzothiazine-1,4 yl-4, N-alkyl tétrahydro-1,2,3,4 quinoxalinyl-1, perhydroquinolyl-1, tétrahydro-1,2,3,4 isoquinolyl-2, aza-8 spiro [4,5] décanyl-8, phényl-2 ou -3 pyrrolidinyl-1, aza-8 dioxa-1,4 spiro [4,5] décanyl-8, thiomorpholino (éventuellement substitué par au moins un radical alkyle) ou indolinyl-1.

3. Procédé selon la revendication 1 pour la préparation des composés de formule (I) pour lesquels R₁ représente un atome d'hydrogène, R₂ représente un radical alkyle, R₃ représente un radical phényle ou bien R₂ et R₃ forment avec l'atome d'azote auquel ils sont rattachés un radical pipéridino substitué par au moins un radical alkyle ou thiomorpholino substitué par au moins un radical alkyle et R₄ représente un radical phénylamino dont le noyau phényle est substitué par un ou plusieurs substituants choisis parmi les radicaux hydroxy, carboxy, hydroxyiminoalkyle, -alk-COOH, alkyle, alkylthio, monohydroxyalkyle et -C(=NOH)-COOH, ainsi que leurs racémiques et leurs énantiomères lorsqu'ils contiennent au moins un centre asymètrique et leurs sels.

## Claims (Claims for the following Contracting State(s): AT, BE, CH, DE, DK, FR, GB, IT, LI, LU, NL, SE)

1. Compounds of formula: 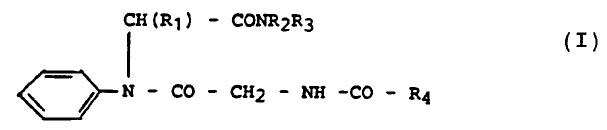 in which
- R₁ represents a hydrogen atom or an alkyl or alkoxycarbonyl or phenyl radical (optionally substituted by one or more substituents chosen from halogen atoms and alkyl, alkoxy, alkylthio, nitro and amino radicals),
- R₂ represents a hydrogen atom or an alkyl radical (optionally substituted by an alkoxycarbonyl radical),
- R₃ represents an alkyl, phenylalkyl, indanyl, cycloalkylalkyl, phenyl (optionally substituted by one or more substituents chosen from halogen atoms and alkyl, alkoxy, alkylthio and dialkylamino radicals) or quinolyl radical,
or else
- R₂ and R₃ form, together with the nitrogen atom to which they are attached, a saturated or unsaturated mono- or polycyclic heterocyclic ring containing 4 to 9 carbon atoms and one or more heteroatoms (O,N,S) and optionally substituted by one or more alkyl, alkoxycarbonyl, dialkylcarbamoyl, phenyl or alkoxy radicals or in combination with a carbon atom of the heterocyclic ring a monocyclic spiro ring containing 4 or 5 chain members and optionally containing one or more heteroatoms (O,S,N),
- R₄ represents a phenyl (optionally substituted by one or more substituents chosen from halogen atoms and alkyl, alkoxy and alkylthio radicals), naphthyl, indolyl, quinolyl or phenylamino radical in which the phenyl nucleus is optionally substituted by one or more substituents chosen from halogen atoms and alkyl, alkoxy, alkylthio, hydroxyl, nitro, amino, acyl, cyano, sulphamoyl, trifluoromethylsulphonamido, carbamoyl, benzoyl, carboxyl, alkoxycarbonyl, phenylhydroxymethyl, piperidino, hydroxyiminoalkyl, alkoxyiminoalkyl, hydroxyaminocarbonyl, 5-tetrazolyl, 5-tetrazolylalkyl, alkylsulphinyl, mono- or polyhydroxyalkyl, sulpho, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX and -S-alk-COOX radicals,
- X represents a hydrogen atom or an alkyl radical,
- alk represents an alkyl or alkylene radical, and
- alk' represents a hydroxyalkylene or hydroxyalkyl radical,
with the exception of the compounds in which R₁ represents a hydrogen atom, R₂ and R₃ each represent an alkyl radical or R₂ and R₃ form, with the nitrogen atom to which they are attached, a 1-pyrrolidinyl radical optionally substituted by an alkyl radical, and R₄ represents a naphthyl, indolyl or phenylamino radical in which the phenyl nucleus is optionally substituted by an alkyl, alkoxy, nitro, hydroxyl, or alkylthio radical or by one or two halogen atoms,
it being understood that the acyl radicals contain 2 to 4 carbon atoms, the alkyl, alkylene and alkoxy radicals and the alkyl, alkylene and alkoxy portions contain 1 to 4 carbon atoms in a straight or branched chain and the cycloalkyl radicals contain 3 to 6 carbon atoms, as well as their racemates and their enantiomers when they contain at least one centre of asymmetry, and their salts.

2. Compounds of formula (I) according to Claim 1, in which R₂ and R₃ represent, with the nitrogen atom to which they are attached, a heterocyclic ring chosen from the piperidino (optionally substituted by at least one alkyl, alkoxycarbonyl, phenyl or dialkylcarbamoyl radical), 1-perhydroazepinyl, 1,2,3,6-tetrahydro-1-pyridyl, 1,2,3,4-tetrahydro-1-quinolyl, 1-pyrrolidinyl, 3,4-dihydro-1,4-2H-benzoxazin-4-yl, 3,4-dihydro-1,4-2H-benzothiazin-4-yl, N-alkyl-1,2,3,4-tetrahydro-1-quinoxalinyl, 1-perhydroquinolyl, 1,2,3,4-tetrahydro-2-isoquinolyl, 8-azaspiro[4,5]decan-8-yl, 2- or 3-phenyl-1-pyrrolidinyl, 8-aza-1,4-dioxaspiro[4,5]decan-8-yl, thiomorpholino (optionally substituted by at least one alkyl radical) or 1-indolinyl rings.

3. Compounds of formula (I) according to Claim 1, in which R₁ represents a hydrogen atom, R₂ represents an alkyl radical, R₃ represents a phenyl radical or else R₂ and R₃ form, with the nitrogen atom to which they are attached, a piperidino radical substituted by at least one alkyl or thiomorpholino radical substituted by at least one alkyl radical, and R₄ represents a phenylamino radical in which the phenyl nucleus is substituted by one or more substituents chosen from hydroxyl, carboxyl, hydroxyiminoalkyl, -alk-COOH, alkyl, alkylthio, monohydroxyalkyl and -C(=NOH)-COOH radicals, and their racemates and their enantiomers when they contain at least one centre of asymmetry, and their salts.

4. Process for the preparation of compounds of formula (I) according to Claim 1, in which R₄ is a phenylamino radical in which the phenyl nucleus is optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido, and -alk-O-alk radicals, characterized in that an amino derivative of formula: 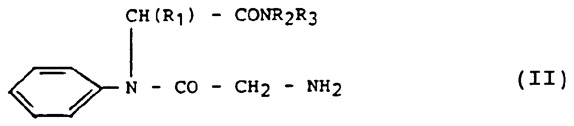 in which R₁, R₂ and R₃ have the same meanings as in Claim 1, is reacted with an isocyanate of formula:
OCN - R₅ (III)
in which R₅ represents a phenyl radical optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido and -alk-O-alk radicals, and the product is then isolated and optionally converted to a salt.

5. Process for the preparation of compounds of formula (I) according to Claim 1, in which R₄ represents a phenylamino radical in which the phenyl nucleus is optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido and -alk-O-alk radicals, characterized in that an amine of formula: 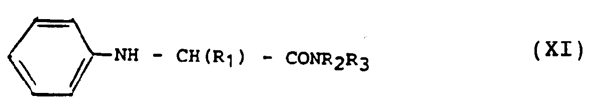 in which R₁, R₂ and R₃ have the same meanings as in Claim 1, is reacted with an acid of formula:
HOOC - CH₂ - NH - CO - R₄ (XV)
in which R₄ has the same meanings as above, and the product is then isolated and optionally converted to a salt.

6. Process for the preparation of compounds of formula (I) according to Claim 1, in which R₄ represents an optionally substituted phenylamino radical, characterized in that a derivative of formula: 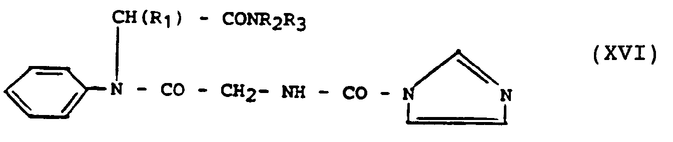 in which R₁, R₂ and R₃ have the same meanings as in Claim 1, is reacted with an amine of formula:
H₂N - R₇ (XVII)
in which R₇ represents a phenyl radical optionally substituted by one or more substituents chosen from halogen atoms and alkyl, alkoxy, alkylthio, hydroxyl, nitro, amino, acyl, cyano, sulphamoyl, trifluoromethylsulphonamido, carbamoyl, benzoyl, carboxyl, alkoxycarbonyl, phenylhydroxymethyl, piperidino, hydroxyiminoalkyl, alkoxyiminoalkyl, 5-tetrazolyl, 5-tetrazolylalkyl, alkylsulphinyl, hydroxyaminocarbonyl, mono- or polyhydroxyalkyl, sulpho, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX and -S-alk-COOX radicals, and the product is isolated and optionally converted to a salt.

7. Process for the preparation of compounds of formula (I) according to Claim 1, in which R₄ represents a phenylamino radical in which the phenyl nucleus is substituted by at least one carboxyl, -alk-COOH, -O-alk-COOH, -alk'-COOH, -CH=CH-COOH, -CO-COOH, -C(=NOH)-COOH or -S-alk-COOH radical, characterized in that a corresponding ester is hydrolysed and the product is isolated and optionally converted to a salt.

8. Process for the preparation of compounds of formula (I) according to Claim 1, in which R₄ represents a phenylamino radical in which the phenyl nucleus is substituted by at least one amino radical, with the exception of those in which R₁ represents a phenyl radical substituted by at least one nitro radical, characterized in that the corresponding nitro derivative is reduced and the product is isolated and optionally converted to a salt.

9. Process for the preparation of compounds of formula (I) according to Claim 1, in which R₄ represents a phenylamino radical in which the phenyl nucleus is substituted by at least one phenylhydroxymethyl radical, characterized in that the corresponding benzoyl derivative is reduced and the product is isolated and optionally converted to a salt.

10. Process for the preparation of compounds of formula (I) according to Claim 1, in which R₄ represents a phenylamino radical in which the phenyl nucleus is substituted by at least one acyl radical, characterized in that a corresponding hydroxyalkyl compound is oxidized and the product is isolated and optionally converted to a salt.

11. Process for the preparation of compounds of formula (I) according to Claim 1, in which R₄ represents a phenylamino radical in which the phenyl nucleus is substituted by at least one hydroxyiminoalkyl, alkoxyiminoalkyl, hydroxyaminocarbonyl or -C(=NOH)-COOX radical in which X represents an alkyl radical, characterized in that a corresponding derivative in which R₄ represents a phenylamino radical in which the phenyl nucleus is substituted by at least one acyl, formyl, alkoxycarbonyl or -CO-COOX radical in which X represents an alkyl radical is reacted with a derivative of formula:
H₂N-OR₈ (XVIII)
in which R₈ represents a hydrogen atom or an alkyl radical, and the product is isolated and optionally converted to a salt.

12. Process for the preparation of compounds of formula (I) according to Claim 1, in which R₄ represents a phenylamino radical in which the phenyl nucleus is substituted by at least one alkylsulphinyl radical, characterized in that a corresponding alkylthio derivative is oxidized and the product is isolated and optionally converted to a salt.

13. Process for the preparation of compounds of formula (I) according to Claim 1, in which R₄ represents a phenylamino radical in which the phenyl nucleus is substituted by at least one -alk-O-CO-alk radical, characterized in that an acyl chloride is reacted with a corresponding hydroxyalkyl compound and the product is isolated and optionally converted to a salt.

14. Process for the preparation of compounds of formula (I) according to Claim 1, in which R₄ represents a phenylamino radical in which the phenyl nucleus is substituted by at least one trifluoromethylsulphonamido radical, characterized in that a corresponding compound in which R₄ represents a phenylamino radical in which the phenyl nucleus is substituted by at least one amino radical is reacted with trifluoromethanesulphonic anhydride and the product is isolated and optionally converted to a salt.

15. Process for the preparation of compounds of formula (I) according to Claim 1, in which R₄ represents an optionally substituted phenyl radical or a naphthyl, indolyl or quinolyl radical, characterized in that a derivative of formula: 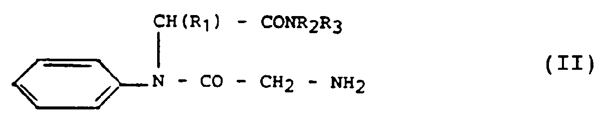 in which R₁, R₂ and R₃ have the same meanings as in Claim 1 is reacted with a derivative of formula:
HOOC R₄ (XIX)
in which R₄ has the same meanings as above and the product is isolated and optionally converted to a salt. 16.

16. Medicaments, characterized in that they contain, as active ingredient, at least one compound of formula (I) according to Claim 1.

17. Medicaments, characterized in that they contain, as active ingredient, at least one compound of formula (I) according to Claims 2 or 3.

18. Medicaments according to Claims 16 and 17 for the treatment and the prevention of disorders associated with CCK and gastrin in the nervous system and the gastrointestinal system.

## Claims (Claims for the following Contracting State(s): ES, GR)

1. Process for the preparation of compounds of formula: 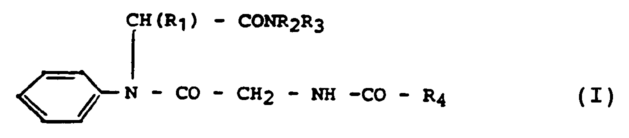 in which
- R₁ represents a hydrogen atom or an alkyl or alkoxycarbonyl or phenyl radical (optionally substituted by one or more substituents chosen from halogen atoms and alkyl, alkoxy, alkylthio, nitro and amino radicals),
- R₂ represents a hydrogen atom or an alkyl radical (optionally substituted by an alkoxycarbonyl radical),
- R₃ represents an alkyl, phenylalkyl, indanyl, cycloalkylalkyl, phenyl (optionally substituted by one or more substituents chosen from halogen atoms and alkyl, alkoxy, alkylthio and dialkylamino radicals) or quinolyl radical,
or else
- R₂ and R₃ form, together with the nitrogen atom to which they are attached, a saturated or unsaturated mono- or polycyclic heterocyclic ring containing 4 to 9 carbon atoms and one or more heteroatoms (O,N,S) and optionally substituted by one or more alkyl, alkoxycarbonyl, dialkylcarbamoyl, phenyl or alkoxy radicals or in combination with a carbon atom of the heterocyclic ring a monocyclic spiro ring containing 4 or 5 chain members and optionally containing one or more heteroatoms (O,S,N),
- R₄ represents a phenyl (optionally substituted by one or more substituents chosen from halogen atoms and alkyl, alkoxy and alkylthio radicals), naphthyl, indolyl, quinolyl or phenylamino radical in which the phenyl nucleus is optionally substituted by one or more substituents chosen from halogen atoms and alkyl, alkoxy, alkylthio, hydroxyl, nitro, amino, acyl, cyano, sulphamoyl, trifluoromethylsulphonamido, carbamoyl, benzoyl, carboxyl, alkoxycarbonyl, phenylhydroxymethyl, piperidino, hydroxyiminoalkyl, alkoxyiminoalkyl, hydroxyaminocarbonyl, 5-tetrazolyl, 5-tetrazolylalkyl, alkylsulphinyl, mono- or polyhydroxyalkyl, sulpho, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX and -S-alk-COOX radicals,
- X represents a hydrogen atom or an alkyl radical,
- alk represents an alkyl or alkylene radical, and
- alk' represents a hydroxyalkylene or hydroxyalkyl radical,
with the exception of the compounds in which R₁ represents a hydrogen atom, R₂ and R₃ each represent an alkyl radical or R₂ and R₃ form, with the nitrogen atom to which they are attached, a 1-pyrrolidinyl radical optionally substituted by an alkyl radical, and R₄ represents a naphthyl, indolyl or phenylamino radical in which the phenyl nucleus is optionally substituted by an alkyl, alkoxy, nitro, hydroxyl or alkylthio radical or by one or two halogen atoms,
it being understood that the acyl radicals contain 2 to 4 carbon atoms, the alkyl, alkylene and alkoxy radicals and the alkyl, alkylene and alkoxy portions contain 1 to 4 carbon atoms in a straight or branched chain and the cycloalkyl radicals contain 3 to 6 carbon atoms, as well as their racemates and their enantiomers when they contain at least one centre of asymmetry, and their salts, characterized in that
A - for the preparation of compounds in which R₄ is a phenylamino radical in which the phenyl nucleus is optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido and -alk-O-alk radicals an amino derivative of formula: 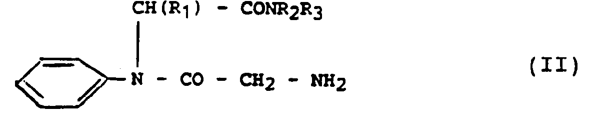 in which R₁, R₂ and R₃ have the same meanings as in formula (I) is reacted with an isocyanate of formula:
OCN - R₅ (III)
in which R₅ represents a phenyl radical optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido and -alk-O-alk radicals, and the product is then isolated and optionally converted to a salt,
B - for the preparation of compounds in which R₄ represents a phenylamino radical in which the phenyl nucleus is optionally substituted by one or more substituents chosen from halogen atoms and alkyl, alkoxy, alkylthio, nitro, acyl, cyano, sulphamoyl, benzoyl, alkoxycarbonyl, 5-tetrazolyl, 5-tetrazolylalkyl, trifluoromethylsulphonamido and -alk-O-alk radicals, an amine of formula: 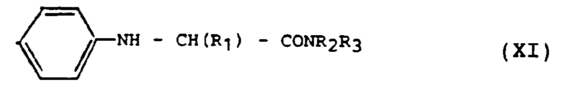 in which R₁, R₂ and R₃ have the same meanings as in formula (I), is reacted with an acid of formula:
HOOC - CH₂ - NH - CO - R₄ (XV)
in which R₄ has the same meanings as above, and the product is then isolated and optionally converted to a salt,
C - for the preparation of compounds in which R₄ represents an optionally substituted phenylamino radical, a derivative of formula: 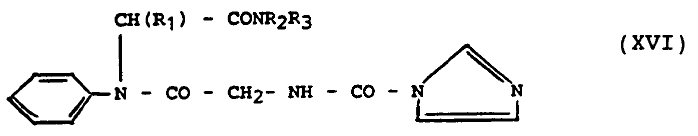 in which R₁, R₂ and R₃ have the same meanings as in formula (I), is reacted with an amine of formula:
H₂N - R₇ (XVII)
in which R₇ represents a phenyl radical optionally substituted by one or more substituents chosen from halogen atoms and alkyl, alkoxy, alkylthio, hydroxyl, nitro, amino, acyl, cyano, sulphamoyl, trifluoromethylsulphonamido, carbamoyl, benzoyl, carboxyl, alkoxycarbonyl, phenylhydroxymethyl, piperidino, hydroxyiminoalkyl, alkoxyiminoalkyl, 5-tetrazolyl, 5-tetrazolylalkyl, alkylsulphinyl, hydroxyaminocarbonyl, mono- or polyhydroxyalkyl, sulpho, -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX and -S-alk-COOX radicals and the product is isolated and optionally converted to a salt,
D - for the preparation of compounds in which R₄ represents a phenylamino radical in which the phenyl nucleus is substituted by at least one carboxyl, -alk-COOH, -O-alk-COOH, -alk'-COOH, -CH=CH-COOH, -CO-COOH, -C(=NOH)-COOH or -S-alk-COOH radical, a corresponding ester is hydrolysed and the product is isolated and optionally converted to a salt,
E - for the preparation of compounds in which R₄ represents a phenylamino radical in which the phenyl nucleus is substituted by at least one amino radical, with the exception of those in which R₁ represents a phenyl radical substituted by at least one nitro radical, the corresponding nitro derivative is reduced and the product is isolated and optionally converted to a salt,
F - for the preparation of compounds in which R₄ represents a phenylamino radical in which the phenyl nucleus is substituted by at least one phenylhydroxymethyl radical, the corresponding benzoyl derivative is reduced and the product is isolated and optionally converted to a salt,
G - for the preparation of compounds in which R₄ represents a phenylamino radical in which the phenyl nucleus is substituted by at least one acyl radical, a corresponding hydroxyalkyl compound is oxidized and the product is isolated and optionally converted to a salt,
H - for the preparation of compounds in which R₄ represents a phenylamino radical in which the phenyl nucleus is substituted by at least one hydroxyiminoalkyl, alkoxyiminoalkyl, hydroxyaminocarbonyl or -C(=NOH)-COOX radical, in which X represents an alkyl radical, a corresponding derivative in which R₄ represents a phenylamino radical in which the phenyl nucleus is substituted by at least one acyl, formyl, alkoxycarbonyl or -CO-COOX radical in which X represents an alkyl radical, is reacted with a derivative of formula:
H₂N-OR₈ (XVIII)
in which R₈ represents a hydrogen atom or an alkyl radical and the product is isolated and optionally converted to a salt,
I - for the preparation of compounds in which R₄ represents a phenylamino radical in which the phenyl nucleus is substituted by at least one alkylsulphinyl radical, a corresponding alkylthio derivative is oxidized and the product is isolated and optionally converted to a salt,
J - for the preparation of compounds in which R₄ represents a phenylamino radical in which the phenyl nucleus is substituted by at least one -alk-O-CO-alk radical, an acyl chloride is reacted with a corresponding hydroxyalkyl compound and the product is isolated and optionally converted to a salt,
K - for the preparation of compounds in which R₄ represents a phenylamino radical in which the phenyl nucleus is substituted by at least one trifluoromethylsulphonamido radical, a corresponding compound in which R₄ represents a phenylamino radical in which the phenyl nucleus is substituted by at least one amino radical is reacted with trifluoromethanesulphonic anhydride and the product is isolated and optionally converted to a salt,
L - for the preparation of compounds in which R₄ represents an optionally substituted phenyl radical or a naphthyl, indolyl or quinolyl radical, a derivative of formula: 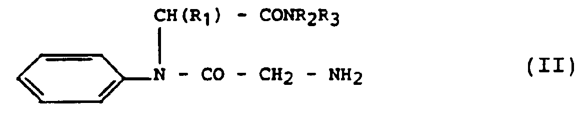 in which R₁, R₂ and R₃ have the same meanings as in formula (I) is reacted with a derivative of formula:
HOOC R₄ (XIX)
in which R₄ has the same meanings as above and the product is isolated and optionally converted to a salt.

2. Process according to Claim 1 for the preparation of compounds of formula (I), in which R₂ and R₃ represent, with the nitrogen atom to which they are attached, a heterocyclic ring chosen from the piperidino (optionally substituted by at least one alkyl, alkoxycarbonyl, phenyl or dialkylcarbamoyl radical), 1-perhydroazepinyl, 1,2,3,6-tetrahydro-1-pyridyl, 1,2,3,4-tetrahydro-1-quinolyl, 1-pyrrolidinyl, 3,4-dihydro-1,4-2H-benzoxazin-4-yl, 3,4-dihydro-1,4-2H-benzothiazin-4-yl, N-alkyl-1,2,3,4-tetrahydro-1-quinoxalinyl, 1-perhydroquinolyl, 1,2,3,4-tetrahydro-2-isoquinolyl, 8-azaspiro[4,5]decan-8-yl, 2- or 3-phenyl-1-pyrrolidinyl, 8-aza-1,4-dioxaspiro[4,5]decan-8-yl, thiomorpholino (optionally substituted by at least one alkyl radical) or 1-indolinyl rings.

3. Process according to Claim 1 for the preparation of compounds of formula (I), in which R₁ represents a hydrogen atom, R₂ represents an alkyl radical, R₃ represents a phenyl radical or else R₂ and R₃ form, with the nitrogen atom to which they are attached, a piperidino radical substituted by at least one alkyl or thiomorpholino radical substituted by at least one alkyl radical, and R₄ represents a phenylamino radical in which the phenyl nucleus is substituted by one or more substituents chosen from hydroxyl, carboxyl, hydroxyiminoalkyl, -alk-COOH, alkyl, alkylthio, monohydroxyalkyl and -C(=NOH)-COOH radicals, and their racemates and their enantiomers when they contain at least one centre of asymmetry, and their salts.

## Patentansprüche (Patentansprüche für folgende(n) Vertragsstaat(en): AT, BE, CH, DE, DK, FR, GB, IT, LI, LU, NL, SE)

1. Verbindungen der Formel 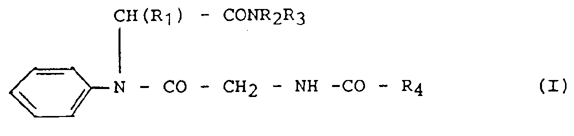 worin
R₁ ein Wasserstoffatom oder einen Alkyl-, Alkoxycarbonyl- oder Phenylrest (gegebenenfalls substituiert durch einen oder mehrere Substituenten, ausgewählt unter den Halogenatomen und den Alkyl-, Alkoxy-, Alkylthio-, Nitro- und Aminoresten) bedeutet,
R₂ für ein Wasserstoffatom oder einen Alkylrest (gegebenenfalls substituiert durch einen Alkoxycarbonylrest) steht,
R₃ einen Alkyl-, Phenylalkyl-, Indanyl-, Cycloalkalkyl-, Phenyl- (gegebenenfalls substituiert durch einen oder mehrere Substituenten, ausgewählt unter den Halogenatomen und den Alkyl-, Alkoxy-, Alkylthio- und Dialkylaminoresten) oder Chinolylrest bedeutet oder
R₂ und R₃ gemeinsam mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N, S), der gegebenenfalls substituiert ist durch einen oder mehrere Alkyl-, Alkoxycarbonyl-, Dialkylcarbamoyl-, Phenyl-, Alkoxyreste, oder in Kombination mit einem Kohlenstoffatom des Heterocyclus einen monocyclischen Spiroring mit 4 oder 5 Gliedern, der gegebenenfalls ein oder mehrere Heteroatome (O, S, N) aufweist, bilden,
R₄ einen Phenyl- (gegebenenfalls substituiert durch einen oder mehrere Substituenten, ausgewählt unter den Halogenatomen und den Alkyl-, Alkoxy- und Alkylthioresten), Naphthyl-, Indolyl-, Chinolyl- oder Phenylaminorest, dessen Phenylkern gegebenenfalls substituiert ist durch einen oder mehrere Substituenten, ausgewählt unter den Halogenatomen und den Alkyl-, Alkoxy-, Alkylthio-, Hydroxy-, Nitro-, Amino-, Acyl-, Cyano-, Sulfamoyl-, Trifluormethylsulfonamido-, Carbamoyl-, Benzoyl-, Carboxy-, Alkoxycarbonyl-, Phenylhydroxymethyl-, Piperidino-, Hydroxyiminoalkyl-, Alkoxyiminoalkyl-, Hydroxyaminocarbonyl-, Tetrazol-5-yl-, Tetrazol-5-yl-alkyl-, Alkylsulfinyl-, Mono- oder Polyhydroxyalkyl-, Sulforesten, den Resten -alk-O-CO-alk, -alk-O-alk, -alk-COOX, -O-alk-COOX, -alk'-COOX, -CH=CH-COOX, -CO-COOX, alk-SO₃H, -CH=CH-alk', -C(=NOH)-COOX und -S-alk-COOX, bedeutet,
X für ein Wasserstoffatom oder einen Alkylrest steht,
alk einen Alkyl- oder Alkylenrest bedeutet,
alk' für einen Hydroxyalkylen- oder Hydroxyalkylrest steht,
mit Ausnahme der Verbindungen, worin R₁ für ein Wasserstoffatom steht, R₂ und R₃ jeweils einen Alkylrest bedeuten oder R₂ und R₃ mit dem Stickstoffatom, an das sie gebunden sind, einen Pyrrolidin-1-yl-Ring, der gegebenenfalls durch einen Alkylrest substituiert ist, bilden und R₄ einen Naphthyl-, Indolyl- oder Phenylaminorest, dessen Phenylkern gegebenenfalls durch einen Alkyl-, Alkoxy-, Nitro-, Hydroxy- oder Alkylthiorest oder durch ein oder zwei Halogenatome substituiert ist, wiedergibt,
mit der Maßgabe, daß die Acylreste 2 bis 4 Kohlenstoffatome aufweisen, die Alkyl-, Alkylen- und Alkoxyreste und die Alkyl-, Alkylen- und Alkoxyteile 1 bis 4 Kohlenstoffatome in gerader oder verzweigter Kette enthalten und die Cycloalkylreste 3 bis 6 Kohlenstoffatome aufweisen, sowie deren racemische Formen und deren Enantiomere, wenn sie zumindest ein Asymmetriezentrum besitzen, und deren Salze.

2. Verbindungen der Formel (I) gemäß Anspruch 1, worin R₂ und R₃ mit dem Stickstoffatom, an das sie gebunden sind, einen Heterocyclus bilden, ausgewählt unter den Piperidino-(gegebenenfalls substituiert durch zumindest einen Alkyl-, Alkoxycarbonyl-, Phenyl- oder Dialkylcarbamoylrest), Perhydroazepin-1-yl-, 1,2,3,6-Tetrahydro-pyrid-1-yl-, 1,2,3,4-Tetrahydrochinol-1-yl-, Pyrrolidin-1-yl-, 3,4-Dihydro-2H-1,4-benzoxazin-4-yl-, 3,4-Dihydro-2H-1,4-benzothiazin-4-yl-, N-Alkyl-1,2,3,4-tetrahydrochinoxalin-1-yl-, Perhydrochinol-1-yl-, 1,2,3,4-Tetrahydro-isochinol-2-yl-, 8-Aza-spiro[4.5]-decan-8-yl-, 2- oder 3-Phenylpyrrolidin-1-yl-, 8-Aza-dioxaspiro[4.5]decan-8-yl-, Thiomorpholino- (gegebenenfalls sub-stituiert durch zumindest einen Alkylrest) oder Indolin-1-yl-Ringen.

3. Verbindungen der Formel (I) gemäß Anspruch 1, worin R₁ für ein Wasserstoffatom steht, R₂ einen Alkylrest bedeutet, R₃ einen Phenylrest bedeutet oder R₂ und R₃ mit dem Stickstoffatom, an das sie gebunden sind, einen durch zumindest einen Alkylrest substituierten Piperidinorest oder einen durch zumindest einen Alkylrest substituierten Thiomorpholinorest bilden und R₄ für einen Phenylaminorest steht, dessen Phenylkern durch einen oder mehrere Substituenten, ausgewählt unter den Hydroxy-, Carboxy-, Hydroxyiminoalkylresten, dem Rest -alk-COOH, den Alkyl-, Alkylthio-, Monohydroxyalkylresten und dem Rest -C(=NOH)-COOH, substituiert ist, sowie deren racemische Formen und deren Enantiomere, wenn sie zumindest ein Asymmetriezentrum aufweisen, und deren Salze.

4. Verfahren zur Herstellung der Verbindungen der Formel (I) gemäß Anspruch 1, worin R₄ für einen Phenylaminorest steht, dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamidoresten und dem Rest -alk-O-alk, substituiert ist, dadurch gekennzeichnet, daß man ein Aminoderivat der Formel 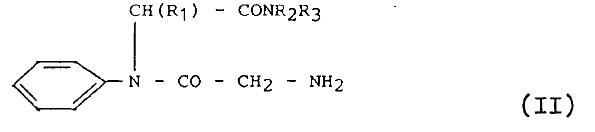 worin R₁, R₂ und R₃ die in Anspruch 1 angegebene Bedeutung besitzen, mit einem Isocyanat der Formel
OCN - R₅ (III)
worin R₅ einen Phenylrest bedeutet, der gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamidoresten und dem Rest -alk-O-alk, substituiert ist, umsetzt, danach das Produkt isoliert und es gegebenenfalls in ein Salz überführt.

5. Verfahren zur Herstellung der Verbindungen der Formel (I) gemäß Anspruch 1, worin R₄ für einen Phenylaminorest steht, dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamidoresten und dem Rest -alk-O-alk substituiert ist, dadurch gekennzeichnet, daß man ein Amin der Formel 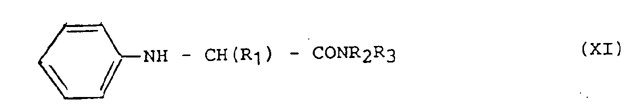 worin R₁, R₂ und R₃ die in Anspruch 1 angegebenen Bedeutungen besitzen, mit einer Säure der Formel
HOOC - CH₂ - NH - CO - R₄ (XV)
worin R₄ die vorstehend angegebenen Bedeutungen besitzt, umsetzt und danach das Produkt isoliert und es gegebenenfalls in ein Salz überführt.

6. Verfahren zur Herstellung der Verbindungen der Formel (I) gemäß Anspruch 1, worin R₄ für einen gegebenenfalls substituierten Phenylaminorest steht, dadurch gekennzeichnet, daß man ein Derivat der Formel 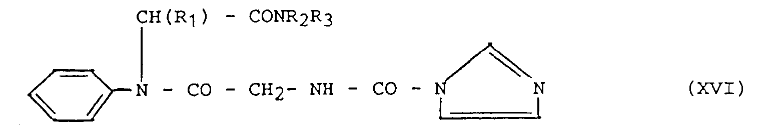 worin R₁, R₂ und R₃ die in Anspruch 1 angegebenen Bedeutungen besitzen, mit einem Amin der Formel
H₂N - R₇ (XVII)
worin R₇ einen Phenylrest bedeutet, der gegebenenfalls durch einen oder mehrere Substituenten, ausgewählt unter den Halogenatomen und den Alkyl-, Alkoxy-, Alkylthio-, Hydroxy-, Nitro-, Amino-, Acyl-, Cyano-, Sulfamoyl-, Trifluormethylsulfonamido-, Carbamoyl-, Benzoyl-, Carboxy-, Alkoxycarbonyl-, Phenylhydroxymethyl-, Piperidino-, Hydroxyiminoalkyl-, Alkoxyiminoalkyl-, Tetrazol-5-yl-, Tetrazol-5-yl-alkyl-, Alkylsulfinyl-, Hydroxyaminocarbonyl-, Mono- oder Polyhydroxyalkyl-, Sulforesten, den Resten -alk-O-CO-alk, -alk-O-alk, -alk-COOX, -O-alk-COOX, -alk'-COOX, -CH=CH-COOX, -CO-COOX, -alk-SO₃H, -CH=CH-alk', -C(=NOH)-COOX und -S-alk-COOX, substituiert ist, umsetzt, das Produkt isoliert und es gegebenenfalls in ein Salz überführt.

7. Verfahren zur Herstellung der Verbindungen der Formel (I) gemäß Anspruch 1, worin R₄ für einen Phenylaminorest steht, dessen Phenylkern durch zumindest einen Carboxyrest, einen Rest -alk-COOH, -O-alk-COOH, -alk'-COOH, -CH= CH-COOH, -CO-COOH, -C(=NOH)-COOH oder -S-alk-COOH substituiert ist, dadurch gekennzeichnet, daß man einen entsprechenden Ester hydrolysiert, das Produkt isoliert und es gegebenenfalls in ein Salz überführt.

8. Verfahren zur Herstellung der Verbindungen der Formel (I) gemäß Anspruch 1, worin R₄ für einen Phenylaminorest steht, dessen Phenylkern durch zumindest einen Aminorest substituiert ist, mit Ausnahme derjenigen, worin R₁ einen Phenylrest bedeutet, der durch zumindest eine Nitrogruppe substituiert ist, dadurch gekennzeichnet, daß man das entsprechende Nitroderivat reduziert, das Produkt isoliert und es gegebenenfalls in ein Salz überführt.

9. Verfahren zur Herstellung der Verbindungen der Formel (I) gemäß Anspruch 1, worin R₄ einen Phenylaminorest bedeutet, dessen Phenylkern durch zumindest einen Phenylhydroxymethylrest substituiert ist, dadurch gekennzeichnet, daß man das entsprechende Benzoylderivat reduziert, das Produkt isoliert und es gegebenenfalls in ein Salz überführt.

10. Verfahren zur Herstellung der Verbindungen der Formel (I) gemäß Anspruch 1, worin R₄ einen Phenylaminorest bedeutet, dessen Phenylkern durch zumindest einen Acylrest substituiert ist, dadurch gekennzeichnet, daß man eine entsprechende Hydroxyalkylverbindung oxidiert, das Produkt isoliert und es gegebenenfalls in ein Salz überführt.

11. Verfahren zur Herstellung der Verbindungen der Formel (I) gemäß Anspruch 1, worin R₄ einen Phenylaminorest bedeutet, dessen Phenylkern durch zumindest einen Hydroxyiminoalkyl-, Alkoxyiminoalkyl-, Hydroxyaminocarbonylrest oder einen Rest -C(=NOH)-COOX, worin X für einen Alkylrest steht, substituiert ist, dadurch gekennzeichnet, daß man ein entsprechendes Derivat, worin R₄ eine Phenylaminogruppe bedeutet, deren Phenylkern durch zumindest einen Acyl-, Formyl-, Alkoxycarbonyl-Rest oder einen Rest -CO-COOX, worin X für einen Alkylrest steht, substituiert ist, mit einem Derivat der Formel
H₂N - OR₈ (XVIII)
worin R₈ ein Wasserstoffatom oder einen Alkylrest bedeutet, umsetzt, das Produkt isoliert und es gegebenenfalls in ein Salz überführt.

12. Verfahren zur Herstellung der Verbindungen der Formel (I) gemäß Anspruch 1, worin R₄ einen Phenylaminorest bedeutet, dessen Phenylkern durch zumindest einen Alkylsulfinylrest substituiert ist, dadurch gekennzeichnet, daß man ein entsprechendes Alkylthioderivat oxidiert, das Produkt isoliert und es gegebenenfalls in ein Salz überführt.

13. Verfahren zur Herstellung der Verbindungen der Formel (I) gemäß Anspruch 1, worin R₄ einen Phenylaminorest bedeutet, dessen Phenylkern durch zumindest einen Rest -alk-O-CO-alk substituiert ist, dadurch gekennzeichnet, daß man ein Acylchlorid mit einer entsprechenden Hydroxyalkylverbindung umsetzt, das Produkt isoliert und es gegebenenfalls in ein Salz überführt.

14. Verfahren zur Herstellung der Verbindungen der Formel (I) gemäß Anspruch 1, worin R₄ einen Phenylaminorest bedeutet, dessen Phenylkern durch zumindest einen Trifluormethylsulfonamidolrest substituiert ist, dadurch gekennzeichnet, daß man eine entsprechende Verbindung, worin R₄ einen Phenylaminorest bedeutet, dessen Phenylkern durch zumindest einen Aminorest substituiert ist, mit Trifluormethansulfon- säureanhydrid umsetzt, das Produkt isoliert und es gegebenenfalls in ein Salz überführt.

15. Verfahren zur Herstellung der Verbindungen der Formel (I) gemäß Anspruch 1, worin R₄ für einen Phenylrest, der gegebenenfalls substituiert ist, einen Naphthyl-, Indolyl-oder Chinolylrest steht, dadurch gekennzeichnet, daß man ein Derivat der Formel 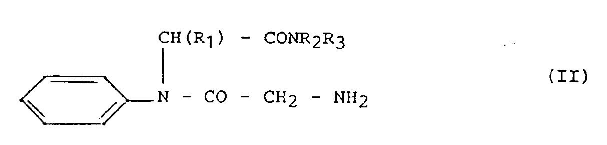 worin R₁, R₂ und R₃ die in Anspruch 1 angegebenen Bedeutungen besitzen, mit einem Derivat der Formel
HOOC R₄ (XIX)
worin R₄ die vorstehend angegebenen Bedeutungen besitzt, umsetzt, das Produkt isoliert und es gegebenenfalls in ein Salz überführt.

16. Arzneimittel, dadurch gekennzeichnet, daß sie als Wirkstoff zumindest eine Verbindung der Formel (I) gemäß Anspruch 1 enthalten.

17. Arzneimittel, dadurch gekennzeichnet, daß sie als Wirkstoff zumindest eine Verbindung der Formel (I) gemäß den Ansprüchen 2 oder 3 enthalten.

18. Arzneimittel gemäß den Ansprüchen 16 und 17 für die Behandlung und Prävention von Störungen, die an das CCK und an das Gastrin im Bereich des Nervensystems und des Gastrointestinaltrakts geknüpft sind.

## Patentansprüche (Patentansprüche für folgende(n) Vertragsstaat(en): ES, GR)

1. Verfahren zur Herstellung der Verbindungen der Formel 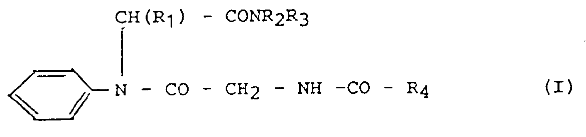 worin
R₁ ein Wasserstoffatom oder einen Alkyl-, Alkoxycarbonyl- oder Phenylrest (gegebenenfalls substituiert durch einen oder mehrere Substituenten, ausgewählt unter den Halogenatomen und den Alkyl-, Alkoxy-, Alkylthio-, Nitro-und Aminoresten) bedeutet,
R₂ für ein Wasserstoffatom oder einen Alkylrest (gegebenenfalls substituiert durch einen Alkoxycarbonylrest) steht,
R₃ einen Alkyl-, Phenylalkyl-, Indanyl-, Cycloalk-alkyl-, Phenyl- (gegebenenfalls substituiert durch einen oder mehrere Substituenten, ausgewählt unter den Halogenatomen und den Alkyl-, Alkoxy-, Alkylthio- und Dialkylaminoresten) oder Chinolylrest bedeutet oder
R₂ und R₃ gemeinsam mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N, S), der gegebenenfalls substituiert ist durch einen oder mehrere Alkyl-, Alkoxycarbonyl-, Dialkylcarbamoyl-, Phenyl-, Alkoxyreste,oder in Kombination mit einem Kohlenstoffatom des Heterocyclus einen monocyclischen Spiroring mit 4 oder 5 Gliedern, der gegebenenfalls ein oder mehrere Heteroatome (O, S, N) aufweist, bilden,
R₄ einen Phenyl- (gegebenenfalls substituiert durch einen oder mehrere Substituenten, ausgewählt unter den Halogenatomen und den Alkyl-, Alkoxy- und Alkylthioresten), Naphthyl-, Indolyl-, Chinolyl- oder Phenylaminorest, dessen Phenylkern gegebenenfalls substituiert ist durch einen oder mehrere Substituenten, ausgewählt unter den Halogenatomen und den Alkyl-, Alkoxy-, Alkylthio-, Hydroxy-, Nitro-, Amino-, Acyl-, Cyano-, Sulfamoyl-, Trifluormethylsulfonamido-, Carbamoyl-, Benzoyl-, Carboxy-, Alkoxycarbonyl-, Phenylhydroxymethyl-, Piperidino-, Hydroxyiminoalkyl-, Alkoxyiminoalkyl-, Hydroxyaminocarbonyl-, Tetrazol-5-yl-, Tetrazol-5-yl-alkyl-, Alkylsulfinyl-, Mono- oder Polyhydroxyalkyl-, Sulforesten, den Resten -alk-O-CO-alk, -alk-O-alk, -alk-COOX, -O-alk-COOX, -alk'-COOX, -CH=CH-COOX, -CO-COOX, alk-SO₃H, -CH=CH-alk', -C(=NOH)-COOX und -S-alk-COOX, bedeutet,
X für ein Wasserstoffatom oder einen Alkylrest steht,
alk einen Alkyl- oder Alkylenrest bedeutet,
alk' für einen Hydroxyalkylen- oder Hydroxyalkylrest steht,
mit Ausnahme der Verbindungen, worin R₁ für ein Wasserstoffatom steht, R₂ und R₃ jeweils einen Alkylrest bedeuten oder R₂ und R₃ mit dem Stickstoffatom, an das sie gebunden sind, einen Pyrrolidin-1-yl-Ring, der gegebenenfalls durch einen Alkylrest substituiert ist, bilden und R₄ einen Naphthyl-, Indolyl- oder Phenylaminorest, dessen Phenylkern gegebenenfalls durch einen Alkyl-, Alkoxy-, Nitro-, Hydroxy- oder Alkylthiorest oder durch ein oder zwei Halogenatome substituiert ist, wiedergibt,
mit der Maßgabe, daß die Acylreste 2 bis 4 Kohlenstoffatome aufweisen, die Alkyl-, Alkylen- und Alkoxyreste und die Alkyl-, Alkylen- und Alkoxyteile 1 bis 4 Kohlenstoffatome in gerader oder verzweigter Kette enthalten und die Cycloalkylreste 3 bis 6 Kohlenstoffatome aufweisen, sowie von deren racemischen Formen und deren Enantiomeren, wenn sie zumindest ein Asymmetriezentrum aufweisen, und von deren Salzen, dadurch gekennzeichnet, daß man
A - zur Herstellung der Verbindungen, worin R₄ für einen Phenylaminorest steht, dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamido-Resten und dem Rest -alk-O-alk, substituiert ist, ein Aminoderivat der Formel 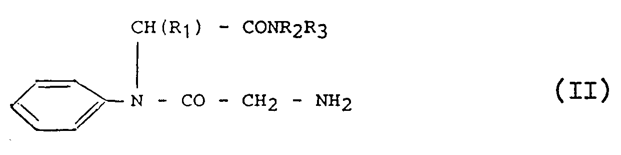 worin R₁, R₂ und R₃ die für Formel (I) angegebenen Bedeutungen besitzen, mit einem Isocyanat der Formel
OCN - R₅ (III)
worin R₅ einen Phenylrest bedeutet, der gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamidoresten und dem Rest -alk-O-alk, substituiert ist, umsetzt und anschließend das Produkt isoliert und es gegebenenfalls in ein Salz überführt,
B - zur Herstellung der Verbindungen, worin R₄ einen Phenylaminorest bedeutet, dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl-, Trifluormethylsulfonamidoresten und dem Rest -alk-O-alk, substituiert ist, ein Amin der Formel 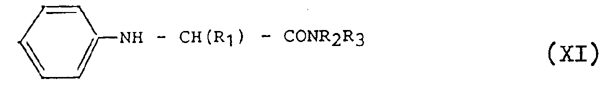 worin R₁, R₂ und R₃ die für Formel (I) angegebenen Bedeutungen besitzen, mit einer Säure der Formel
HOOC - CH₂ - NH - CO - R₄ (XV)
worin R₄ die vorstehend angegebenen Bedeutungen besitzt, umsetzt, danach das Produkt isoliert und es gegebenenfalls in ein Salz überführt,
C - zur Herstellung der Verbindungen, worin R₄ einen gegebenenfalls substituierten Phenylaminorest bedeutet, ein Derivat der Formel 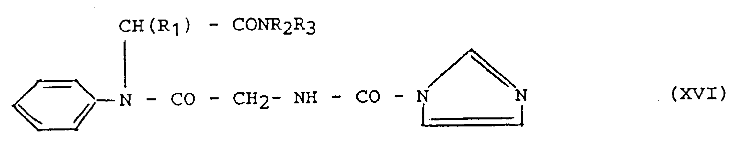 worin R₁, R₂ und R₃ die für Formel (I) angegebenen Bedeutungen besitzen, mit einem Amin der Formel
H₂N - R₇ (XVII)
umsetzt, worin R₇ einen Phenylrest bedeutet, der gegebenenfalls durch einen oder mehrere Substituenten, ausgewählt unter den Halogenatomen und den Alkyl-, Alkoxy-, Alkylthio-, Hydroxy-, Nitro-, Amino-, Acyl-, Cyano-, Sulfamoyl-, Trifluormethylsulfonamido-, Carbamoyl-, Benzoyl-, Carboxy-, Alkoxycarbonyl-, Phenylhydroxymethyl-, Piperidino-, Hydroxyiminoalkyl-, Alkoxyiminoalkyl-, Tetrazol-5-yl-, Tetrazol5-yl-alkyl-, Alkylsulfinyl-, Hydroxyaminocarbonyl-, Mono- oder Polyhydroxyalkyl-, Sulforesten, den Resten -alk-O-CO-alk, -alk-O-alk, -alk-COOX, -O-alk-COOX, -alk'-COOX, -CH= CH-COOX, -CO-COOX, -alk-SO₃H, -CH=CH-alk', -C(=NOH)-COOX und -S-alk-COOX, substituiert ist, das Produkt isoliert und es gegebenenfalls in ein Salz überführt,
D - zur Herstellung der Verbindungen, worin R₄ einen Phenylaminorest bedeutet, dessen Phenylkern durch zumindest einen Carboxyrest, einen Rest -alk-COOH, -O-alkCOOH, -alk'-COOH, -CH=CH-COOH, -CO-COOH, -C(=NOH)-COOH oder -S-alk-COOH substituiert ist, einen entsprechenden Ester hydrolysiert, das Produkt isoliert und es gegebenenfalls in ein Salz überführt,
E - zur Herstellung der Verbindungen, worin R₄ einen Phenylaminorest bedeutet, dessen Phenylkern durch zumindest eine Aminogruppe substituiert ist, mit Ausnahme derjenigen, worin R₁ einen Phenylrest bedeutet, der durch zumindest einen Nitrorest substituiert ist, man das entsprechende Nitroderivat reduziert, das Produkt isoliert und es gegebenenfalls in ein Salz überführt,
F - zur Herstellung der Verbindungen, worin R₄ einen Phenylaminorest bedeutet, dessen Phenylkern durch zumindest einen Phenylhydroxymethylrest substituiert ist, das entsprechende Benzoylderivat reduziert, das Produkt isoliert und es gegebenenfalls in ein Salz überführt,
G - zur Herstellung der Verbindungen, worin R₄ einen Phenylaminorest bedeutet, dessen Phenylkern durch zumindest einen Acylrest substituiert ist, eine entsprechende Hydroxyalkylverbindung oxidiert, das Produkt isoliert und es gegebenenfalls in ein Salz überführt,
H - zur Herstellung der Verbindungen, worin R₄ einen Phenylaminorest bedeutet, dessen Phenylkern durch zumindest einen Hydroxyiminoalkyl-, Alkoxyiminoalkyl-, Hydroxyaminocarbonylrest oder durch einen Rest -C(=NOH)-COOX, worin X für einen Alkylrest steht, substituiert ist, ein entsprechendes Derivat, worin R₄ für einen Phenylaminorest steht, dessen Phenylkern durch zumindest einen Acyl-, Formyl-, Alkoxycarbonylrest oder einen Rest -CO-COOX substituiert ist, worin X für einen Alkylrest steht, mit einem Derivat der Formel
H₂N-OR₈ (XVIII)
worin R₈ für ein Wasserstoffatom oder einen Alkylrest steht, umsetzt, das Produkt isoliert und es gegebenenfalls in ein Salz überführt,
I - zur Herstellung der Verbindungen, worin R₄ für einen Phenylaminorest steht, dessen Phenylkern durch zumindest einen Alkylsulfinylrest substituiert ist, ein entsprechendes Alkylthioderivat oxidiert, das Produkt isoliert und es gegebenenfalls in ein Salz überführt,
J - zur Herstellung der Verbindungen, worin R₄ einen Phenylaminorest bedeutet, dessen Phenylkern durch zumindest einen Rest -alk-O-CO-alk substituiert ist, ein Acylchlorid mit einer entsprechenden Hydroxyalkylverbindung umsetzt, das Produkt isoliert und es gegebenenfalls in ein Salz überführt,
K - zur Herstellung der Verbindungen, worin R₄ einen Phenylaminorest bedeutet, dessen Phenylkern durch zumindest einen Trifluormethylsulfonamidorest substituiert ist, eine entsprechende Verbindung, worin R₄ einen Phenylaminorest bedeutet, dessen Phenylkern durch zumindest einen Aminorest substituiert ist, mit Trifluormethansulfonsäureanhydrid umsetzt, das Produkt isoliert und es gegebenenfalls in ein Salz überführt,
L - zur Herstellung der Verbindungen, worin R₄ einen gegebenenfalls substituierten Phenylrest, einen Naphthyl-, Indolyl- oder Chinolylrest bedeutet, ein Derivat der Formel 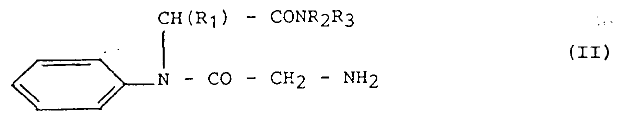 worin R₁, R₂ und R₃ die für Formel (I) angegebenen Bedeutungen besitzen, mit einem Derivat der Formel
HOOC R₄ (XIX)
worin R₄die vorstehend angegebenen Bedeutungen besitzt, umsetzt, das Produkt isoliert und es gegebenenfalls in ein Salz überführt.

2. Verfahren gemäß Anspruch 1 zur Herstellung der Verbindungen der Formel (I), worin die Reste R₂ und R₃ mit dem Stickstoffatom, an das sie gebunden sind, einen Heterocyclus bilden, ausgewählt unter den Piperidino- (gegebenenfalls substituiert durch zumindest einen Alkyl-, Alkoxycarbonyl-, Phenyl- oder Dialkylcarbamoylrest), Perhydroazepin-1-yl-, 1,2,3,6-Tetrahydro-pyrid-1-yl-, 1,2,3,4-Tetrahydrochinol-1-yl-, Pyrrolidin-1-yl-, 3,4-Dihydro-2H-1,4-benzoxazin-4-yl-, 3,4-Dihydro-2H-1,4-benzothiazin-4-yl-, N-Alkyl-1 2,3,4-tetrahydrochinoxalin-1-yl-, Perhydrochinol-1-yl-, 1,2,3,4-Tetrahydro-isochinol-2-yl-, 8-Aza-spiro[4.5]decan-8-yl-, 2- oder 3-Phenylpyrrolidin-1-yl-, 8-Aza-1,4-dioxa-spiro[4.5]decan-8-yl-, Thiomorpholino- (gegebenenfalls substituiert durch zumindest einen Alkylrest) oder Indolin-1-yl-Ringen.

3. Verfahren gemäß Anspruch 1 zur Herstellung der Verbindungen der Formel (I), worin R₁ für ein Wasserstoffatom steht, R₂ einen Alkylrest bedeutet, R₃ einen Phenylrest bedeutet oder R₂ und R₃ mit dem Stickstoffatom, an das sie gebunden sind, einen durch zumindest einen Alkylrest substituierten Piperidinorest oder einen durch zumindest einen Alkylrest substituierten Thiomorpholinorest bilden und R₄ für einen Phenylaminorest steht, dessen Phenylkern durch einen oder mehrere Substituenten, ausgewählt unter den Hydroxy-, Carboxy-, Hydroxyiminoalkylresten, dem Rest -alk-COOH, den Alkyl-, Alkylthio-, Monohydroxyalkylresten und dem Rest -C(=NOH)-COOH, substituiert ist, sowie von deren racemischen Formen und deren Enantiomeren, wenn sie zumindest ein Asymmetriezentrum aufweisen, und von deren Salzen.
